(19) Europäisches Patentamt
European Patent Office
Office européen des brevets

(11) EP 2 003 199 B1

(12) **EUROPEAN PATENT SPECIFICATION**

(45) Date of publication and mention of the grant of the patent:
**25.04.2012 Bulletin 2012/17**

(21) Application number: **07739741.2**

(22) Date of filing: **27.03.2007**

(51) Int Cl.:
*C12N 15/09* (2006.01) *C12M 1/40* (2006.01)
*C12N 1/15* (2006.01) *C12N 1/19* (2006.01)
*C12N 1/21* (2006.01) *C12N 9/04* (2006.01)
*C12Q 1/32* (2006.01) *G01N 33/66* (2006.01)

(86) International application number:
**PCT/JP2007/056303**

(87) International publication number:
**WO 2007/116710 (18.10.2007 Gazette 2007/42)**

(54) **GLUCOSE DEHYDROGENASE**

GLUCOSEDEHYDROGENASE

GLUCOSE DÉSHYDROGÉNASE

(84) Designated Contracting States:
**AT BE BG CH CY CZ DE DK EE ES FI FR GR HU IE IS IT LI LT LU LV MC MT NL PL PT RO SE SI SK TR**

(30) Priority: **31.03.2006 US 788252 P**
**28.11.2006 JP 2006319645**
**28.11.2006 JP 2006319644**
**28.11.2006 JP 2006319643**
**28.12.2006 JP 2006354168**

(43) Date of publication of application:
**17.12.2008 Bulletin 2008/51**

(73) Proprietor: **Toyo Boseki Kabushiki Kaisha**
**Osaka-shi, Osaka 530-8230 (JP)**

(72) Inventors:
- **KITABAYASHI, Masao**
**Fukui 9140047 (JP)**
- **TSUJI, Yuji**
**Fukui 9140047 (JP)**
- **AIBA, Hiroshi**
**Fukui 9140047 (JP)**
- **KAWAMINAMI, Hiroshi**
**Fukui 9140047 (JP)**
- **KISHIMOTO, Takahide**
**Fukui 9140047 (JP)**
- **NISHIYA, Yoshiaki**
**Fukui 9140047 (JP)**

(74) Representative: **Arends, William Gerrit et al**
**Marks & Clerk LLP**
**90 Long Acre**
**London**
**WC2E 9RA (GB)**

(56) References cited:
**WO-A-03/012071** **WO-A-2006/101239**
**WO-A1-2004/058958**

- **BAK T.G. ET AL.: 'Studies on the glucose dehydrogenase of Aspergillus oryzae. I. Induction of its synthesis by rho-benzoquinone and hydroquinone' BIOCHIM BIOPHYS ACTA vol. 139, no. 2, 11 July 1967, pages 265 - 276, XP003018175**
- **BAK T.G. ET AL.: 'Studies on glucose dehydrogenase of Aspergillus oryzae. II. Purification and physical and chemical properties' BIOCHIM BIOPHYS ACTA vol. 139, no. 2, 11 July 1967, pages 277 - 293, XP009067679**
- **BAK T.G. ET AL.: 'Studies on glucose dehydrogenase of Aspergillus oryzae. 3. General enzymatic properties' BIOCHIM BIOPHYS ACTA vol. 146, no. 2, 1967, pages 317 - 327, XP002373580**
- **BAK T.G. ET AL.: 'Studies on glucose dehydrogenase of Aspergillus oryzae. IV. Histidyl residue as an active site' BIOCIM BIOPHYS ACTA vol. 146, no. 2, 1967, pages 328 - 335, XP003018176**

EP 2 003 199 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to a gene encoding glucose dehydrogenase derived from *Aspergillus oryzae* and a method for producing the glucose dehydrogenase by gene recombination.

BACKGROUND ART

Self-monitoring of blood glucose is important for a patient with diabetes to grasp the usual blood glucose level of the patient and apply it to treatment. An enzyme taking glucose as a substrate is utilized as a sensor used in the self-monitoring of blood glucose. An example of such an enzyme includes, for example, glucose oxidase (EC. 1.1.3.4). Glucose oxidase is advantageous in that it has a high specificity for glucose and excellent thermal stability, and thus has been used as the enzyme for blood glucose sensors from a long time ago. Its first publication goes back to 40 years ago. In a blood glucose sensor using glucose oxidase, measurement is performed by transferring electrons produced in a process of oxidizing glucose, to convert into D-glucono-δ-lactone, to an electrode via a mediator. However, glucose oxidase easily transfers protons produced in the reaction to oxygen, and thus dissolved oxygen affects the measured value, which has been problematic.

In order to avoid such a problem, for example, NAD(P)-dependent glucose dehydrogenase (EC. 1.1.1.47) or pyrrolo-quinoline quinone (hereinafter sometimes abbreviated as PQQ) - dependent glucose dehydrogenase (EC. 1.1.5.2; former EC. 1.1.99.17) is used as the enzyme for the blood glucose sensor. They dominate in that they are not affected by dissolved oxygen, but the former NAD(P)-dependent glucose dehydrogenase (hereinafter sometimes abbreviated as NADGDH) has poor stability and requires the addition of a coenzyme. Meanwhile, the latter PQQ-dependent glucose dehydrogenase (hereinafter sometimes abbreviated as PQQGDH) is inferior in substrate specificity, reacts with other sugars such as maltose and lactose, and thus correctness of the measured value is impaired.

In Non-patent documents 1 to 4, glucose dehydrogenase derived from *Aspergillus oryzae* has been reported, but no glucose dehydrogenase gene has been reported. In Non-patent documents 1 to 4, it has not been described to produce the glucose dehydrogenase derived from *Aspergillus oryzae* by gene recombination.

In Patent document 1, flavin-bound glucose dehydrogenase derived from genus *Aspergillus* has been disclosed. This enzyme dominates in that this is excellent in substrate specificity and is not affected by the dissolved oxygen. For the thermal stability, it has been described that a residual activity ratio after treatment at 50°C for 15 minutes is about 89% and this enzyme is excellent in thermal stability (hereinafter also described as heat resistance). In Patent document 2, a gene sequence and an amino acid sequence of that enzyme have been reported. However, considering the case of requiring treatment with heat in a step of producing sensor chips, its stability is not always sufficient. However, it is very difficult even using recombinant DNA technology to produce flavin-binding type glucose dehydrogenase (also referred to as FAD-dependent glucose dehydrogenase). In fact, the yield of FAD-dependent glucose dehydrogenase in recombinant *Escherichia coli* K-12 strain disclosed in Patent document 2 was 0.09 U/mL, which was an extremely low level. The *Escherichia coli* K-12 strain is most commonly used in recombinant protein production, and is the host most frequently used industrially in terms of easy recombinant engineering, easy culture and safety. Therefore, a method for efficiently producing recombinant FAD-dependent glucose dehydrogenase using the *Escherichia coli* K-12 strain as the host has been desired.

Recently, the complete genome sequence of *Aspergillus* oryzae has been determined. However, there is no available information as to what part of the sequence encodes glucose dehydrogenase.

Patent Document 1: WO 2004/058958
Patent Document 2: WO 2006/101239
Nonpatent-Document 1: Biochim Biophys Acta.1967 Jul 11;139(2):265-76
Nonpatent Document 2: Biochim Biophys Acta.1967 Jul 11;139(2):277-93
Nonpatent Document 3: Biochim Biophys Acta.146(2):317-27
Nonpatent Document 4: Biochim Biophys Acta.146(2):328-35

DISCLOSURE OF THE INVENTION

PROBLEM TO BE SOLVED BY THE INVENTION

A first problem with regard to enzymes for use in blood glucose sensor is to overcome shortcomings which publicly known enzymes for a blood glucose sensor as described above have and provide an enzyme for the blood glucose sensor which is practically more advantageous.

A second problem is the provision a method for efficiently producing a practically more advantageous enzyme for the blood glucose sensor. More specifically, the problem is to establish a method for stably producing glucose dehydrogenase derived from *Aspergillus oryzae* on a large scale by specifying, acquiring and utilizing a gene encoding the glucose dehydrogenase.

A third problem is the provision of an enzyme for a blood glucose sensor that is practically more advantageous. More specifically, the object is to acquire and utilize glucose dehydrogenase (GDH) derived from filamentous fungi, which is excellent in not only substrate specificity but also production cost on a large scale.

A fourth problem is to overcome the shortcomings of the thermal stability of publicly known enzymes as described above and provide a composition capable of being used as a practically more advantageous reagent for measuring a blood glucose level.

The present invention means to solve one or more of the above problems.

MEANS FOR SOLVING THE PROBLEM

As a result of extensive study on solving one or more of the above problems, the present inventors have acquired glucose dehydrogenase from filamentous fungus belonging to the genus *Penicillium,* and found that said glucose dehydrogenase has more excellent properties than the publicly known enzymes for measuring the blood glucose level.

In order to solve one or more of the above problems, the present inventors presumed and acquired a glucose dehydrogenase gene derived from *Aspergillus* oryzae by utilizing the database of the National Center for Biotechnology Information (NCBI) and found that glucose dehydrogenase derived from *Aspergillus oryzae* could be acquired from *Escherichia coli* using the gene. They also deduced an amino acid sequence from genomic information, and found that an amino acid sequence thought to be a signal peptide was present in an N-terminal region of the amino acid sequence.

According to the present invention, by utilizing the glucose dehydrogenase gene isolated from *Aspergillus oryzae*, it becomes possible to efficiently produce glucose dehydrogenase and acquire more practical glucose dehydrogenase.

Since the signal peptide acts as a migration signal to a periplasma space, a potential restriction on an amount of a protein to be expressed was a concern because of the capacity of the space. Thus, the function of the signal peptide was deleted. Consequently, the productivity of GDH was increased by about 10 times, and it became possible to reduce the production cost to 1/10.

As described above, for the thermal stability of FADGDH described in Patent document 1, it has been reported that the residual activity ratio after treatment at 50°C for 15 minutes is about 89%.

However, this is a result of an enzyme preparation absolutely obtained by culturing a wild type strain and purifying from the culture. In our study, in the recombinant enzyme from *Escherichia coli*, it was found that a polysaccharide was not added to the enzyme surface and the thermal stability was remarkably reduced.

The thermal stability of FADGDH obtained by us from *Aspergillus* oryzae strain was compared with that of recombinant FADGDH (AOGDH) obtained by expressing an FADGDH gene from *Aspergillus oryzae* in *Escherichia coli.* Consequently, the former kept about 77% of its activity whereas the latter rFADGDH had only about 13% of its activity after being treated at 50°C for 15 minutes.

We also compared the thermal stability of FADGDH obtained from *Aspergillus* terreus strain (NBRC33026) with that of recombinant FADGDH (ATGDH) obtained by expressing an FADGDH gene from *Aspergillus* terreus in *Escherichia coli.* Consequently, the former kept about 90% of its activity whereas the latter rFADGDH had only about 2% of its activity after being treated at 50°C for 15 minutes.

In the process for producing chips for the blood glucose sensor, a heating and drying treatment is sometimes given. If the recombinant enzyme is used, a large thermal deactivation potentially occurs. Thus, it has been necessary to enhance the thermal stability.

A tactic for enhancing the stability of PQQGDH has been reported in Patent document 3, in which a study using PQQGDH modified at a gene level was reported. However, for a procedure to increase the stability without modifying the enzyme, no possibility thereof has been described.

Patent document 3: WO02/072839

A level capable of being heated and dried refers to a state where the residual activity is 20% or more, preferably a state where the residual activity is 40% or more, and more preferably a state where the residual activity is 60% or more, after treatment at 50°C for 15 minutes.

Described herein are an embodiment of the invention and three concepts.

An Embodiment of the invention

Item 1B

A gene composed of the following DNA (a), (b), (c) or (d) :

(a) DNA composed of a base sequence described in SEQ ID NO:11;
(b) DNA composed of a base sequence described in SEQ ID NO: 14, comprising the base sequence described in SEQ ID NO:11 and an intron;
(c) DNA which hybridizes with DNA composed of a base sequence complementary to DNA (a) under stringent conditions and encodes a protein having glucose dehydrogenase activity;
(d) DNA which hybridizes with DNA composed of a base sequence complementary to DNA (b) under stringent conditions and comprises a region encoding a protein having glucose dehydrogenase activity.

Item 2B

A gene encoding the following protein (a) or (b):

(a) a protein composed of an amino acid sequence described in SEQ ID NO:10; or
(b) a protein composed of an amino acid sequence having one or more amino acid deletions, substitutions, additions or insertions in the amino acid sequence described in SEQ ID NO:10, and having glucose dehydrogenase activity.

Item 3B

A recombinant vector comprising the gene according to Item 1B or 2B.

Item 4B

A transformant transformed with the recombinant vector according to Item 3B.

Item 5B

The transformant according to Item 4B, wherein a host is *Escherichia coli.*

Item 6B

A method for producing a protein having glucose dehydrogenase activity, characterized in that the transformant according to Item 4B or 5B is cultured in a nutrient medium and the protein having glucose dehydrogenase activity is collected.

First Concept

Item 1A

Glucose dehydrogenase characterized by being derived from a eukaryotic organism and keeping 90% or more activity after treatment at 55°C for 15 minutes in a liquid form compared with the activity before the treatment with heat.

Item 2A

Glucose dehydrogenase characterized by being derived from a eukaryotic organism and keeping activity after treatment at 60°C for 15 minutes in a liquid form.

Item 3A

The glucose dehydrogenase according to Item 2A characterized by being derived from a eukaryotic organism and keeping 40% or more activity after treatment at 60°C for 15 minutes in a liquid form compared with activity before the treatment with heat.

Item 4A

The glucose dehydrogenase according to any one of Items 1A to 3A, wherein the eukaryotic organism is a filamentous fungus.

Item 5A

The glucose dehydrogenase according to Item 4A, wherein the filamentous fungus is a filamentous fungus belonging to genus *Penicillium.*

Item 6A

The glucose dehydrogenase according to Item 5A, wherein the filamentous fungus belonging to genus *Penicillium* is *Penicillium lilacinoechinulatum* or *Penicillium italicum.*

Item 7A

The glucose dehydrogenase according to any one of Items 1A to 6A characterized in that an action upon maltose is less than 1% of an action upon glucose.

Item 8A

The glucose dehydrogenase according to Item 7A characterized in that an action upon galactose is less than 2% of an action upon glucose.

Item 9A

Glucose dehydrogenase derived from a eukaryotic organism and having the following physicochemical properties (a) to (f):

(a) optimum reaction temperature: 50°C;
(b) optimum reaction pH: about 6.5;
(c) temperature stability: a residual activity ratio of GDH after treatment at 55°C for 15 minutes is 90% or more, and a residual activity ratio of GDH after treatment at 60°C for 15 minutes is 40% or more;
(d) pH stability: 5.0 to 8.0 (the residual activity ratio of GDH after treatment at 25°C for 16 hours is 90% or more) ;
(e) substrate specificity: when an action upon glucose is 100%, an action upon xylose is about 10%, an action upon 2-deoxy-D-glucose is about 14%, and a reactivity to maltose, fructose, arabinose, sucrose, galactose, mannose, melezitose, sorbose, ribose, maltotriose, maltotetraose and trehalose is less than 2%; and
(f) effects of chemicals: strongly inhibited by copper, silver and cadmium and inhibited by monoiodoacetic acid, N-ethyl maleimide, hydroxylamine and sodium azide.

Item 10A

Glucose dehydrogenase derived from a eukaryotic organism and having the following physicochemical properties (a) to (f) :

(a) optimum reaction temperature: 60°C;
(b) optimum reaction pH: about 6.5;
(c) temperature stability: a residual activity ratio of GDH after treatment at 55°C for 15 minutes is 95% or more and a residual activity ratio of GDH after treatment at 60°C for 15 minutes is 70% or more;
(d) pH stability: 5.0 to 8.5 (the residual activity ratio of GDH after treatment at 25°C for 16 hours is 80% or more);
(e) substrate specificity: when an action upon glucose is 100, an action upon xylose is about 10%, an action upon 2-deoxy-D-glucose is about 17%, and a reactivity to maltose, fructose, arabinose, sucrose, galactose, mannose, melezitose, sorbose, ribose, maltotriose, maltotetraose and trehalose is less than 2%; and
(f) effects of chemicals: strongly inhibited by copper, silver and cadmium and inhibited by iron, zinc, monoiodoacetic acid, N-ethyl maleimide and hydroxylamine.

Item 11A

A protein corresponding to any of the following (a) to (e) :

(a) a protein composed of an amino acid sequence represented by SEQ ID NO:2, and having glucose dehydrogenase activity;
(b) a protein having consecutive multiple amino acid residue deletions at the N terminal side in a range in which glucose dehydrogenase activity is not lost in the amino acid sequence represented by SEQ ID NO:2;
(c) a protein having 15 or more and 22 or less consecutive amino acid residue deletions at the N terminal side in the amino acid sequence represented by SEQ ID NO:2;
(d) a protein composed of an amino acid sequence having one or more amino acid residue deletions, substitutions, insertions or additions in the amino acid sequence represented by any of (a) to (c) and having glucose dehydrogenase activity; or
(e) a protein having 80% or more homology to the amino acid sequence represented by SEQ ID NO:2 and having glucose dehydrogenase activity.

Item 12A

A protein corresponding to any of the following (a) to (e) :

(a) a protein composed of an amino acid sequence represented by SEQ ID NO:4, and having glucose dehydrogenase activity;
(b) a protein having several consecutive amino acid residue deletions at the N terminal side in a range in which glucose dehydrogenase activity is not lost in the amino acid sequence represented by SEQ ID NO:4;
(c) a protein having 15 or more and 19 or less consecutive amino acid residue deletions at the N terminal side in the amino acid sequence represented by SEQ ID NO:4;
(d) a protein composed of an amino acid sequence having one or more amino acid residue deletions, substitutions, insertions or additions in the amino acid sequence represented by any of the above (a) to (c) and having glucose dehydrogenase activity; or
(e) a protein having 80% or more homology to the amino acid sequence represented by SEQ ID NO:4 and having glucose dehydrogenase activity.

Item 13A

A method for producing glucose dehydrogenase by culturing a eukaryotic organism, extracting and purifying the glucose dehydrogenase according to any of Items 1A to 12A.

Item 14A

A nucleic acid having a base sequence encoding the protein according to any of Items 1A to 12A.

Item 15A

A recombinant plasmid comprising a nucleic acid having a base sequence encoding the protein according to any of Items 1A to 12A under a functional promoter in a host organism.

Item 16A

A recombinant microorganism obtained by transforming a host microorganism with a nucleic acid molecule having a sequence comprising the nucleic acid according to Item 14A.

Item 17A

The recombinant microorganism according to Item 16A, wherein the nucleic acid molecule is the recombinant plasmid according to Item 15A.

Item 18A

The recombinant microorganism according to Item 16A or 17A, wherein the host microorganism is a eukaryotic microorganism.

Item 19A

The recombinant microorganism according to Item 16A or 17A, wherein the host microorganism is a prokaryotic microorganism.

Item 20A

The recombinant microorganism according to Item 19A, wherein the prokaryotic microorganism is a gram-negative bacterium.

Item 21A

The recombinant microorganism according to Item 20A, wherein the gram-negative bacterium is *Escherichia coli.*

Item 22A

A method for producing glucose dehydrogenase by culturing the microorganism according to any of Items 16A to 21A, extracting and purifying the glucose dehydrogenase.

Item 23A

A method for measuring a glucose concentration using the glucose dehydrogenase according to any of Items 1A to 12A.

Item 24A

A glucose assay kit comprising the glucose dehydrogenase according to any of Items 1A to 12A.

Item 25A

A glucose sensor comprising the glucose dehydrogenase according to any of Items 1A to 12A.

Second Concept

Item 1C

A method for producing recombinant glucose dehydrogenase derived from filamentous fungus characterized in that an amount of GDH expression is enhanced by introducing a mutation in a signal peptide sequence present in the N terminal region of GDH, compared with an amount before introduction of the mutation.

Item 2C

The method for producing recombinant GDH derived from filamentous fungus according to Item 1C characterized in that an amount of GDH expression is enhanced by deleting or substituting a part of an amino acid sequence of the signal peptide present in the N-terminal region of GDH, compared with an amount before the introduction of the mutation.

Item 3C

The method for producing GDH according to Item 1C characterized in that, an expression amount is enhanced by expressing the GDH in that a part or all of an amino acid sequence, MLFSLAFLSALSLATASPAGRA, present in the N terminal region of the amino acid sequence described in SEQ ID NO: 9 or 10 is deleted, as compared with a case where the deleted sequence is present.

Item 4C

The method for producing GDH according to Item 1C characterized in that an amount of GDH is enhanced by substituting or inserting 1 to 22 amino acid residues in the amino acid sequence, MLFSLAFLSALSLATASPAGRA, present in the N terminal region of the amino acid sequence described in SEQ ID NO: 9 or 10, compared with a case where the original amino acid sequence is present.

Item 5C

The method for producing GDH according to Item 1C characterized in that an GDH expression is enhanced by expressing filamentous fungus-derived GDH in that a part or all of an amino acid sequence, MLGKLSFLSALSLAVAATLSNSTSA, present in the N terminal region is deleted, or 1 to 25 amino acid residues are substituted and/or inserted, compared with a case where the original amino acid sequence is present.

Item 6C

A DNA sequence which encodes a GDH gene substituting and/or deleting a part or all of a DNA sequence encoding a signal peptide present in the N terminal region of glucose dehydrogenase (GDH) derived from filamentous fungus, and is used for the method of any of Items IC to 5C.

Item 7C

A recombinant vector comprising the DNA sequence of Item 6C.

Item 8C

A transformant obtained by introducing the recombinant vector of Item 7C into a host.

Item 9C

A GDH protein produced using the transformant according to Item 8C.

Item 10C

A composition comprising the GDH protein according to Item 9C.

Item 11C

A method for measuring a glucose concentration using the composition according to Item 10C.

Item 12C

A glucose sensor comprising the composition according to Item 11C.

Third Concept

Item 1D

A method for enhancing the stability of glucose dehydrogenase (GDH), the method comprising the step of, in a composition containing soluble GDH requiring a coenzyme, causing one or more compounds selected from amino acids and sugars which do not act as substrates of the enzyme to coexist with the enzyme, wherein the stability is enhanced compared with a case where the compound is not made coexist.

Item 2D

The method for enhancing the stability according to Item 1D, wherein a final concentration of each compound coexisting in a solution is 0.01% by weight or more and a total concentration of respective compounds is 30% by weight or less.

Item 3D

The method for enhancing the thermal stability according to Item 1D or 2D characterized in that the compounds to be added are one or more selected from the group consisting of trehalose, mannose, melezitose, sodium gluconate, sodium glucuronate, galactose, methyl-α-D-glucoside, cyclodextrin, α-D-melibiose, sucrose, cellobiose, glycine, alanine, serine, BSA, sodium chloride, sodium sulfate, trisodium citrate, ammonium sulfate, succinic acid, malonic acid, glutaric acid, arabinose, sorbitan, 2-deoxy-D-glucose, xylose, fructose, sodium aspartate, glutamic acid, phenylalanine, proline, lysine hydrochloride, sarcosine and taurine.

Item 4D

The method for enhancing the thermal stability according to any of Items 1D to 3D characterized in that GDH requiring a flavin compound as a coenzyme is derived from filamentous fungus.

Item 5D

A composition comprising soluble GDH whose thermal stability has been enhanced by the method according to any of Items 1D to 4D.

Item 6D

The GDH-containing composition according to Item 5D, which contains a flavin compound-bound recombinant GDH, and even when treated at 50°C for 15 minutes, has 20% or more of the GDH activity of the composition stored at 4°C.

Item 7D

The GDH-containing composition according to Item 5D, which contains a flavin compound-bound GDH, and even when treated at 50°C for 30 minutes, has 10% or more of the GDH activity of the composition stored at 4°C.

Item 8D

A method for measuring a glucose concentration using the composition according to any of Items 5D to 7D.

Item 9D

A glucose sensor comprising the composition according to any of Items 5D to 7D.

Item 10D

A method for producing a composition, the method comprising the step of, in a composition containing soluble coenzyme-bound GDH, causing one or more compounds selected from amino acids and sugars which are not substrates of glucose dehydrogenase (GDH) to coexist with GDH, wherein the thermal stability of GDH is enhanced compared with a case where the compound is not made coexist.

THE EFFECT OF THE DISCLOSURE

According to the present disclosure, it is possible to provide glucose dehydrogenase which is excellent in heat resistance and substrate specificity and is not affected by dissolved oxygen.

According to the present disclosure, it has become possible to efficiently produce glucose dehydrogenase. Further, it has become easy to perform molecular biological improvement in order to obtain more practical glucose dehydrogenase.

According to the present disclosure, it has become possible to efficiently produce glucose dehydrogenase and obtain more practical glucose dehydrogenase.

According to the present disclosure, it has become possible to efficiently produce recombinant glucose dehydrogenase and obtain more practical glucose dehydrogenase in terms of industrial usage by deducing the amino acid sequence from the glucose dehydrogenase gene isolated from the microorganism belonging to genus *Aspergillus* or *Penicillium*, predicting the signal peptide region, deleting the DNA sequence encoding a part or all of the signal peptide, or substituting or inserting amino acid residues in the amino acid sequence encoding the signal peptide.

The enhancement of the stability of the GDH composition according to the present disclosure can reduce thermal deactivation of the enzyme when a glucose measurement reagent, a glucose assay kit and a glucose sensor are produced, thereby reducing the amount of the enzyme used and enhancing measurement accuracy. It can also provide a reagent for measuring a blood glucose level using the GDH composition excellent in storage stability.

BRIEF DESCRIPTION OF THE DRAWINGS

FIG. 1 is a graph showing the temperature dependency of reaction rates of GDH as described herein. A relative activity (%) is shown when the maximum activity is made 100.

FIG. 2 is a graph showing the pH dependency of reaction rates of GDH as described herein. The relative activity (%) is shown when the maximum activity is made 100.

FIG. 3 is a graph showing the temperature stability of GDH as described herein. An activity value after treatment with heat at each temperature is represented by the relative activity (%) when the activity before treatment with heat is made 100.

FIG. 4 is a graph showing the pH stability of GDH derived from accession number NBRC6092. Acetate buffer, PIPES buffer and Tris hydrochloride buffer were used for pH 3.3 to 6, pH 6 to 7 and pH 7 to 8.5, respectively.

FIG. 5 is a graph showing the pH stability of GDH derived from accession number NBRC32032. Acetate buffer, PIPES buffer and Tris hydrochloride buffer were used for pH 3.3 to 6, pH 6 to 7 and pH 7 to 8.5, respectively.

FIG. 6 is a graph showing the relationship between a glucose concentrations and response voltage values in a glucose electrode made using GDH as described herein. The response voltage value 1 V output by a detector corresponds to 10 μA of current in the glucose electrode.

FIG. 7 is a graph showing the relationship between a glucose concentrations and response voltage values in a glucose electrode made using GDH (recombinant protein) as described herein. The response voltage value 1 V output by the detector corresponds to 10 μA of current in the glucose electrode.

FIG. 8 shows signal profiles predicting a cleavage site in a signal peptide in FAD-GDH derived from *Aspergillus oryzae*.

FIG. 9 is a graph showing the relationship of a culture phase of GDH (having the signal peptide) derived from wild type A. oryzae with turbidity (OD), pH and GDH activity.

FIG. 10 is a graph showing the relationship of the culture phase of GDH-S2 derived from mutant *A. oryzae* with the turbidity (OD), pH and GDH activity.

FIG. 11 is a graph showing the relationship of the culture phase of GDH-S3 derived from mutant *A. oryzae* with turbidity (OD), pH and GDH activity.

FIG. 12 is a graph showing the relationship of the culture phase of GDH (the predicted signal peptide was removed) derived from wild type *A. terreus* at 28°C at Kd P of 0.5 with turbidity (OD), pH and GDH activity.

FIG. 13 is a graph showing the relationship of the culture phase of GDH (the predicted signal peptide was removed) derived from wild type *A. terreus* at 28°C at Kd P of 0.75 with turbidity (OD), pH and GDH activity.

FIG. 14 is a graph showing the relationship of the culture phase of GDH (the predicted signal peptide was removed) derived from wild type *A. terreus* at 28°C at Kd P of 1.0 with turbidity (OD), pH and GDH activity.

FIG. 15 is a graph showing the relationship of the culture phase of GDH (the predicted signal peptide was removed) derived from wild type *A. terreus* at 28°C at Kd P of 1.5 with turbidity (OD), pH and GDH activity.

FIG. 16 is a graph showing the relationship of the culture phase of GDH (the predicted signal peptide was removed) derived from wild type *A. terreus* at 20°C at Kd P of 0.5 with turbidity (OD), pH and GDH activity.

FIG. 17 is a graph showing the relationship of the culture phase of GDH (the predicted signal peptide was removed) derived from wild type *A. terreus* at 23°C at Kd P of 0.5 with turbidity (OD), pH and GDH activity.

FIG. 18 is a graph showing the relationship of the culture phase of GDH (the predicted signal peptide was removed) derived from wild type *A. terreus* at 26°C at Kd P of 0.5 with turbidity (OD), pH and GDH activity.

FIG. 19 is a graph showing the relationship of the culture phase of GDH (the predicted signal peptide was removed) derived from wild type *A. terreus* at 29°C at Kd P of 0.5 with turbidity (OD), pH and GDH activity.

BEST MODE FOR CARRYING OUT THE INVENTION

There is disclosed herein one main embodiment and three main concepts from the first concept to the third concept.

The embodiment and the first concept to the third concept are hereinafter described, but the description of each embodiment or concept is applicable to each other as necessary.

The Embodiment

In order to address the above problems, the present inventors found a gene DNA presumed to encode glucose dehydrogenase by utilizing the NCBI database.

DNA (gene) composed of the base sequence described in SEQ ID NO:7 is a genomic gene sequence comprising DNA (gene) encoding glucose dehydrogenase derived from *Aspergillus oryzae* RIB40 strain, predicted from the NCBI database, where no intron has been eliminated.

DNA composed of the base sequence described in SEQ ID NO:8 is obtained by removing the intron from the sequence of SEQ ID NO:7.

The gene encoding a protein composed of the amino acid sequence described in SEQ ID NO:9 indicates the entire sequence of a glucose dehydrogenase gene predicted from the NCBI database.

The present inventors predicted from Non-patent Documents 1 to 4 and the NCBI database that DNA (gene) encoding the protein having glucose dehydrogenase activity derived from *Aspergillus oryzae* could be easily identified.

And further, the inventors thought that it was also easy to produce a recombinant vector containing the gene, a transformant obtained by the transformation, and to purify a protein encoded by the gene expressed by the transformant was purified.

Although the amino acid sequence and the base sequence encoding flavin-bound glucose dehydrogenase derived from *Aspergillus oryzae* were not specified in the NCBI database, the present inventors specifically tried to culture *Aspergillus* oryzae, purify glucose dehydrogenase (GDH) from its culture supernatant using various chromatography methods, make a probe by analyzing its terminal amino acid sequence and isolate the gene encoding the glucose dehydrogenase, with reference to the methods described in Non-patent Documents 1 to 4 and publicly known technologies.

Likewise, the present inventors tried to isolate the gene encoding glucose dehydrogenase by independently obtaining a microorganism belonging to genus *Aspergillus terreus*.

Although the present inventors studied various methods, it was found that it was difficult to obtain a GDH preparation with high purity whose band could be clearly identified on SDS-PAGE from the culture supernatant of the *Aspergillus oryzae* TI strain by ordinary purification methods using salting out and chromatography methods. It was speculated that sugar chains supposed to be bound to the enzyme protein were one of the causes making the purification and identification difficult. Therefore, they determined that they had no choice but to give up the cloning utilizing the partial amino acid sequence, which is one of the standard methods for acquiring the gene.

Thus, with much trial and error, it was extremely difficult to acquire the gene, but as a result of extensive study, the present inventors isolated the gene encoding the flavin-bound glucose dehydrogenase derived from *Aspergillus oryzae* and completed the present invention.

The details will be described later in Experiments 1B to 3B.

One embodiment of the present invention is a method for producing a protein having glucose dehydrogenase activity characterized by culturing a transformant transformed with a recombinant vector comprising a gene composed of any DNA of the following (a), (b), (c), (d) or a gene encoding a protein of the following (e) or (f) in a nutrient medium, and collecting the protein having glucose dehydrogenase activity.

(a) DNA (gene) composed of a base sequence described in SEQ ID NO:11 indicates the entire sequence of DNA (gene) encoding the protein having glucose dehydrogenase activity derived from *Aspergillus oryzae* TI strain to be described later, identified by the present inventors;

(c) DNA (gene) which hybridizes with DNA composed of the base sequence complementary to DNA composed of the base sequence described in SEQ ID NO:11 under stringent conditions and encodes the protein having glucose dehydrogenase activity is also included in the present invention;

(b) DNA (gene) composed of the base sequence described in SEQ ID NO: 14 is a genomic gene sequence comprising DNA (gene) encoding the protein having glucose dehydrogenase activity derived from *Aspergillus* oryzae TI strain to be described later, where no intron has been eliminated;

(d) DNA which hybridizes DNA composed of a base sequence complementary to DNA composed of the base sequence described in SEQ ID NO:14 under stringent conditions and comprises a region encoding glucose dehydrogenase activity is also included in the present invention;

(e) The gene encoding the protein composed of the amino acid sequence described in SEQ ID NO:10 indicates the entire sequence of DNA (gene) encoding the protein having glucose dehydrogenase activity derived from *Aspergillus oryzae* TI strain to be described later.; or

(f) DNA (gene) encoding a protein composed of amino acid sequence having one or a few amino acid deletions, substitutions or additions (insertions) in the amino acid sequence described in SEQ ID NO:10 and having glucose dehydrogenase activity is also included in the present invention.

The DNA (gene) of the present invention can include those in which codon usage has been changed to enhance the expression of the GDH.
For example, the above GDH gene derived from *Aspergillus oryzae* is inserted into an expression vector (many vectors such as plasmids are known in the art), and an appropriate host (many hosts such as *Escherichia coli* are known in the art) is transformed with the expression vector. A water soluble fraction containing GDH can be yielded by culturing the resulting transformant, collecting microbial cells from the medium by centrifugation, disrupting the microbial cells by a mechanical method or an enzymatic method, e.g., using lysozyme and if necessary adding a chelating agent such as EDTA and a surfactant for solubilization. Alternatively, by the use of an appropriate host-vector system, it is possible to secret the expressed GDH directly in the medium.
A GDH-containing solution obtained as above could be precipitated by concentration under reduced pressure, membrane concentration, salting out using ammonium sulfate or sodium sulfate, or fractional precipitation using a hydrophilic organic solvent such as methanol, ethanol, acetone, etc. The treatment with heat and isoelectric focusing are also effective purification procedures. The purified GDH can also be yielded by performing gel filtration using an absorbing agent or a gel filtration agent, absorption chromatography, ion exchange chromatography and affinity chromatography. It is preferable that the purified enzyme preparation is purified to the extent that the enzyme is detected as a single band on electrophoresis (SDS-PAGE).
These procedures can be carried out in accordance with the following references.

(a) Tanpakushitsu Jikken Protocol Vol. 1, Functional Analysis Vol. 2, Structural Analysis (Shujunsha) edited by Yoshifumi Nishimura and Shigeo Ohno.
(b) Revised Tanpakushitsu Jikken Note, Extraction and Separation/Purification (Yodosha) edited by Masato Okada and Kaori Miyazaki.
(c) Tanpakushitsu Jikken no Susumekata (Yodosha) edited by Masato Okada and Kaori Miyazaki.

Alternatively, the above procedures can also be carried forward by the methods exemplified below.
The produced DNA having genetic information for the protein is transferred into a host microorganism by ligating to a vector.
As the vector, those constructed for gene recombination from a phage or a plasmid capable of independently replicating in the host microorganism are suitable. As the phage, for example, Lambda gt10, Lambda gt11, etc., are exemplified when *Escherichia coli* is used as the host microorganism. As the plasmid, for example, pBR322, pUC19, pKK223-3 and pBluescript are exemplified when *Escherichia coli* is used as the host microorganism. Among them, those such as pBluescript carrying a promoter capable of being recognized in *Escherichia coli* upstream of a cloning site are preferable.
The appropriate host microorganism is not particularly limited as long as the recombinant vector is stable therein and can independently replicate and a trait of the foreign gene can be expressed. For *Escherichia coli, Escherichia coli* W3110, *Escherichia coli* C600, *Escherichia coli* HB101, *Escherichia coli* JW109, *Escherichia coli* DH5$\alpha$, etc., can be used.
As the method for transferring the recombinant vector into the host microorganism, for example, when the host microorganism belongs to genus *Escherichia*, a method of transferring recombinant DNA in the presence of Ca ions can be employed, and further, an electroporation method may be used. Furthermore, commercially available competent cells (e.g., Competent High DH5$\alpha$ supplied by Toyobo Co., Ltd.) may be used. When a yeast is used as the host, a lithium method or an electroporation method is used. When filamentous fungus is used, a protoplast method is used.
In the present invention, the method for yielding the gene encoding GDH includes the following methods. The predicted GDH gene can be found by using the information for a genomic sequence of *Aspergillus oryzae.* Then, mRNA is prepared from the microbial cells of *Aspergillus* oryzae and cDNA is synthesized. The GLD gene is amplified by PCR using the cDNA obtained in this way as a template and the recombinant vector is constructed by binding and closing this gene and the vector at blunt ends or sticky ends of both DNA with DNA ligase. The recombinant vector is transferred into a host microorganism in which the vector can replicate, and subsequently, the recombinant microorganism containing the gene encoding GDH is obtained by utilizing a marker of the vector.
GDH in a large amount can be stably produced by culturing the microorganism which is the transformant yielded as above in the nutrient medium. The transformant could be selected by searching for a microorganism that expressed the marker of the vector and the GDH activity simultaneously. For example, a microorganism that grows in a selection medium based on a drug resistant marker and generates GDH may be selected.
The base sequence of the GDH gene was decoded by a dideoxy method described in Science 214:1205, 1981. The amino acid sequence of GDH was deduced from the base sequence determined as above.
As in the above, the once selected GDH gene in the recombinant vector can be easily transferred into another recombinant vector which can replicate in another microorganism by collecting the DNA which is the GDH gene from the recombinant vector carrying the GDH gene by using restriction enzymes and the PCR method, and binding the DNA to another vector fragment. For the transformation of another microorganism with these vectors, a competent cell method by treating with calcium, an electroporation method and a protoplast method can be used.

The GDH gene of the present invention may be one having the DNA sequence so that one or a few amino acid residues is deleted or substituted in the amino acid sequence after translation of the gene or so that other amino acid residues are added or substituted, as long as the protein encoded by the GDH gene has glucose dehydrogenase activity.

As the method for modifying the gene encoding wild type GDH, the typically performed technique to modify the genetic information is used. That is, DNA having the genetic information of the modified protein is made by converting the specific base in DNA having the genetic information of the protein or inserting or deleting the specific base. The specific methods for converting the base in the DNA include the use of commercially available kits (Transformer Mutagenesis Kit supplied by Clonetech; EXQIII/Mung Bean Deletion Kit supplied by Stratagene; QuickChange Site Directed Mutagenesis Kit supplied by Stratagene), or utilization of a polymerase chain reaction (PCR) method.

For the culture of the host microorganism which is the transformant, the culture conditions can be selected in consideration of the nutritional physiological nature of the host. Liquid culture is used in many cases, and it is industrially advantageous to perform aerobic culture with stirring. However, considering the productivity, it is more advantageous in some cases that the filamentous fungus is used as the host and a solid culture is employed.

As nutrient sources for the medium, those typically used for the culture of a microorganism can be widely used. Carbon sources may be carbon compounds capable of being assimilated. For example, glucose, sucrose, lactose, maltose, lactose, molasses and pyruvic acid are used. Nitrogen sources may be usable nitrogen compounds. For example, peptone, meat extracts, yeast extracts, casein hydrolyzed products, and bean cakes extracted with alkali are used. In addition, phosphate salts, carbonate salts, sulfate salts, salts of magnesium, calcium, potassium, iron, manganese and zinc, particular amino acids and particular vitamins are used if necessary.

The culture temperature can be appropriately changed in the range in which the microorganism grows and produces GDH, and is preferably about 20 to 37°C. The culture time period is somewhat different depending on the conditions, the culture may be terminated at an appropriate time period by judging the time right to reach the maximum yield of GDH, and the culture time period is typically about 6 to 48 hours. The pH value of the medium can be appropriately changed in the range in which the microorganism grows and produces the GDH, and is preferably in the range of about pH 6.0 to 9.0.

The culture medium containing the microbial cells that produce GDH can be directly collected and utilized. However, in general, according to standard methods, when the GDH is present in the culture medium, a GDH-containing solution is separated from the microorganism microbial cells by filtration or centrifugation, and subsequently utilized. When GDH is present in the microbial cells, the microbial cells are collected from the culture by filtration or centrifugation, then disrupted by a mechanical method or an enzymatic method using lysozyme, and if necessary, a chelating agent such as EDTA and a surfactant are added to solubilize, and GDH is separated/collected as an aqueous solution.

The GDH-containing solution obtained above could be precipitated by concentration under reduced pressure, membrane concentration, salting out treatment using ammonium sulfate or sodium sulfate or fractional precipitation using a hydrophilic organic solvent such as methanol, ethanol or acetone. Treatment with heat and isoelectric focusing treatment are also effective purification procedures. The purified GDH can also be yielded by subsequently performing gel filtration using an absorbing agent or a gel filtration agent, absorption chromatography, ion exchange chromatography and affinity chromatography.

For example, it is possible to obtain a purified enzyme preparation by separating and purifying by gel filtration using Sephadex gel (supplied by GE Health Care Bioscience), or column chromatography using DEAE Sepharose CL-6B (supplied by GE Health Care Bioscience) or Octyl Sepharose CL-6B (supplied by GE Health Care Bioscience). It is preferable that the purified enzyme preparation is purified to the extent that the enzyme is detected as a single band on electrophoresis (SDS-PAGE).

The First Concept

The first conceptis glucose dehydrogenase derived from a eukaryotic organism and catalyzes the following reaction.

D-Glucose + Electron transport substance (oxidation type) $\rightarrow$ D-glucono-$\delta$-lactone + Electron transport substance (reduction type)

The glucose dehydrogenase of the first concept is characterized by high heat resistance, and is distinguished in this point from publicly known GDH derived from eukaryotic organisms. In glucose dehydrogenase derived from *Aspergillus terreus* described as excellent in stability among publicly known GDH derived from eukaryotic organisms, a residual activity (activity residual ratio when the activity before treatment with heat is 100%) after treatment with heat at 55°C for 15 minutes is 60% or less. On the contrary, in GDH of the first concept, the residual activity after being treated at 55°C for 15 minutes is 90% or more. In GDH of the first concept, preferably GDH activity is kept after treatment at 60°C for 15 minutes and more preferably the residual activity after treatment at 60°C for 15 minutes is 40% or more.

When it is determined whether it has the aforementioned heat resistance or not, GDH, in the first concept, is dissolved in 20 mM K-phosphate buffer (pH 6.5) so as to have an activity of 1 U/mL, and heated for 15 minutes. The activity was measured by the method shown in Test Experiment described later.

GDH of the first conceptmay be derived from a eukaryotic organism and all organisms having a cell nucleus covered with a nuclear membrane are included in the category of eukaryotic organisms. More preferably, the eukaryotic organisms are filamentous fungi. Among them, preferable filamentous fungi include microorganisms belonging to genera Penicillium and *Aspergillus,* and furthermore, it is more preferable for it to be derived from filamentous fungi belonging to genus Penicillium. Moreover, among genus Penicillium, it is more preferable that GDH of the first conceptis derived from *Penicillium lilacinoechinulatum* or *Penicillium italicum*. These fungal strains are easily available by requesting an assignment from a culture collection. More preferable fungal strains are *Penicillium lilacinoechinulatum* and *Penicillium italicum*, registered under accession numbers NBRC6231 and NBRC32032, respectively, at the Biological Resource Center, National Institute of Technology and Evaluation.

The first conceptis also characterized by high substrate specificity, the action upon maltose is less than 1% of the action upon glucose, and the action upon galactose is less than 2% of the action upon glucose. The action referred to herein indicates GDH activity at a substrate concentration of 4 mM, as measured according to Test Experiment described later, and is the substrate concentration in a reaction solution composition used, the final concentration being adjusted to 4 mM.

From another viewpoint, the first conceptis glucose dehydrogenase derived from a eukaryotic organism and having the following properties.

(a) Apparent molecular weight by gel filtration: about 270 kDa;
(b) optimum reaction temperature: 50°C;
(c) optimum reaction pH: about 6.5;
(d) temperature stability: residual activity ratio of GDH after treatment at 55°C for 15 minutes is 90% or more, and the residual activity ratio of GDH after treatment at 60°C for 15 minutes is 40% or more;
(e) pH stability: 5.0 to 8.0 (the residual activity ratio of GDH after treatment at 25°C for 16 hours is 90% or more);
(f) substrate specificity: when the action upon glucose is 100, the action upon xylose is about 10%, the action upon 2-deoxy-D-glucose is about 14%, and the reactivity to maltose, fructose, arabinose, sucrose, galactose, mannose, melezitose, sorbose, ribose, maltotriose, maltotetraose and trehalose is less than 2%; and
(g) effects of chemicals: strongly inhibited by copper, silver and cadmium and inhibited by monoiodoacetic acid, N-ethyl maleimide, hydroxylamine and sodium azide.

The eukaryotic organism from which the GDH is derived is not particularly limited as long as it produces GDH having the above properties, and is preferably filamentous fungus, more preferably filamentous fungus belonging to genus *Penicillium,* and still more preferably *Penicillium lilacinoechinulatum.* More preferably, the eukaryotic organism is the fungal strain registered under accession number NBRC6231 at Biological Resource Center, National Institute of Technology and Evaluation.

Furthermore, the first conceptis glucose dehydrogenase derived from a eukaryotic organism and having the following properties.

(a) Apparent molecular weight by gel filtration: 79 to 93 kDa;
(b) optimum reaction temperature: 60°C;
(c) optimum reaction pH: about 6.5;
(d) temperature stability: the residual activity ratio of GDH after treatment at 55°C for 15 minutes is 95% or more, and the residual activity ratio of GDH after treatment at 60°C for 15 minutes is 70% or more;
(e) pH stability: 5.0 to 8.5 (the residual activity ratio of GDH after treatment at 25°C for 16 hours is 80% or more);
(f) substrate specificity: when the action upon glucose is 100, the action upon xylose is about 10%, the action upon 2-deoxy-D-glucose is about 17%, and the reactivity to maltose, fructose, arabinose, sucrose, galactose, mannose, melezitose, sorbose, ribose, maltotriose, maltotetraose and trehalose is less than 2%; and
(g) effects of chemicals: strongly inhibited by copper, silver and cadmium and inhibited by iron, zinc, monoiodoacetic acid, N-ethyl maleimide and hydroxylamine.

The eukaryotic organism from which GDH is derived is not particularly limited as long as it produces GDH having the above properties, and is preferably filamentous fungus, more preferably filamentous fungus belonging to genus *Penicillium*, and still more preferably *Penicillium italicum*. More preferably, the eukaryotic organism is the fungal strain registered under accession number NBRC32032 at Biological Resource Center, National Institute of Technology and Evaluation.

When it is determined whether having the aforementioned pH stability or not, GDH, in the first concept, is dissolved in 20 mM acetate buffer (pH 4.5 to 6.0), PIPES buffer (pH 6.0 to 7.5) or Tris hydrochloride buffer (pH 7.0 to 8.5) so as to have the activity of 1 U/mL, and heated for 15 minutes. The activity was measured by the method shown in Test Experiment described later.

The conditions for the aforementioned gel filtration are as follows. A column TSK-GEL G3000SW (7.5 mm x 300 mm) supplied by Tosoh Corporation and a buffer 50 mM Tris-HCl 150 mM NaCl (pH 7.5) are used. An amount of a sample to be charged is 25 μL, which is then fractionated at a flow rate of 0.5 mL/minutes. Based on a calibration curve previously prepared using standard protein solutions, the molecular weight is calculated from a peak position of the sample. A peak position may be specified by monitoring based on ultraviolet absorbance measurement, or collecting fractions from the column and determining the peak of GDH activity in the fractions.

In the measurement of the effects of the chemicals described above, each chemical is dissolved at a final concentration of 2 mM in a reaction reagent shown in Test Experiment to be described later, and the activity is measured using a 0.1 to 5 U/mL GDH solution according to the method in Test Experiment and compared with the activity measured for a reaction reagent to which the chemical has not been added. "Being inhibited" in the first conceptrefers to observing 10% or more activity lowering in the case where the chemical has been added compared with the case where the chemical has not been added in the present method. "Undergoing strong inhibition" refers to observing 50% or more activity lowering in the case where the chemical has been added compared with the case where the chemical has not been added in the present method.

From another viewpoint, the first conceptis a protein composed of the amino acid sequence represented by SEQ ID NO:2 and having glucose dehydrogenase activity. A protein having one or more amino acid substitutions, deletions, insertions or additions in the amino acid sequence represented by SEQ ID NO:2 and having glucose dehydrogenase activity is also included in the first concept. The protein having 80% or more homology, more preferably 85% or more homology, and still more preferably 90% or more homology to the amino acid sequence represented by SEQ ID NO:2 is in the scope of the first concept. Furthermore, amino acid residues in these sequences may undergo various modifications, e.g., addition of a sugar chain or methylation in the process of expression.

Furthermore, the first conceptis a protein composed of the amino acid sequence represented by SEQ ID NO: 4 and having glucose dehydrogenase activity. A protein having one or more amino acid substitutions, deletions, insertions or additions in the amino acid sequence represented by SEQ ID NO:4 and having glucose dehydrogenase activity is also included in the first concept. The protein having 80% or more homology, more preferably 85% or more homology, and still more preferably 90% or more homology to the amino acid sequence represented by SEQ ID NO:4 is also in the scope of the first concept. Furthermore, amino acid residues in these sequences may undergo various modifications, e.g., addition of a sugar chain or methylation in the process of expression.

The GDH of the first conceptis also a protein which has deleted multiple consecutive amino acid residues at the N-terminal side within a range in which glucose dehydrogenase activity is not lost in the amino acid sequence represented by SEQ ID NOS:2 and 4. The number of amino acid residues to be deleted is not particularly limited as long as GDH activity is not lost. An exemplified target is, for example, a protein in which the N terminal sequence corresponding to the signal sequence or the N terminal sequence predicted to correspond to the signal sequence has been deleted. In the production of GDH not by gene recombination or the recombinant GDH production using the host having the same secretory expression mechanism as in the organism from which the GDH was derived, it is thought that the resulting GDH is mature GDH in which the secretory signal has been eliminated, and that in its amino acid sequence, the portion corresponding to the signal sequence at the N terminal side has been deleted in the amino acid sequence represented by SEQ ID NOS:2 and 4. Also in the recombinant GDH production using a host such as a prokaryotic organism having a different nature from the organism from which the GDH was derived, it is possible to express the gene in which the portion encoding the amino acid sequence corresponding to the signal sequence or predicted to correspond to the signal sequence has been eliminated. At that time, when a terminal amino acid residue after deleting the sequence to be deleted is one other than methionine, it is preferable to add an initial codon (ATG). Alternatively, it is also possible to make GDH with a deleted N-terminus by connecting a tag gene through an adaptor to the N terminal sequence to be deleted, and digesting the adaptor site with site directed peptidase after expression. As a method for predicting such an N terminal region, various tools for predicting the signal peptide can be used, and an example of such tools is SignalP var. 3.0. It is possible to access the server (http://www.cbs.dtu.dk/services/SignalP/) of this program and make predictions on line. The signal sequence portion predicted by this method corresponds to either the initial codon to Ala at position 15 or the initial codon to Ser at position 22 in the SEQ ID NO:2, and either the initial codon to Ala at position 15 or the initial codon to Ala at position 19 in SEQ ID NO:4. In particular, when the recombinant GDH is expressed using a prokaryotic organism as the host, it is advantageous that by deleting the above N terminal region, GDH activity in the culture is enhanced compared with the case of not deleting it.

The "homology" described in the first conceptmeans the percentage (%) of identical amino acid residues in all overlapped amino acid residues in an optimal alignment when two amino acid sequences are aligned using a mathematical algorithm publicly known in the art (preferably, the algorithm can consider introducing a gap to one or both of the sequences for the optimal alignment). The homology described in the first conceptwas calculated using the BLAST program incorporating the algorithm described in Non-patent Document 5. Non-patent Document 5: Karlin et al., Proc. Natl. Acad. Sci. USA (1993) Vol.90 p5873-5877. GDH composed of the sequence represented by SEQ ID NO:2 and GDH composed of the sequence represented by SEQ ID NO:4 are identical in the characteristic of high heat stability. The amino acid sequences of these two GDH are 80% homologous, which is extremely high. According to search results in NCBI-BLAST, no amino acid sequence having more than 55% homology to these GDH sequences has been know until now. Therefore, one of indicators which characterize GDH of the first conceptcan include the high homology to the amino acid sequence represented by SEQ ID NO:2 or 4. It can be presumed that a protein having 80% or more, more preferably 85% or more, and still more preferably 90% or more homology is industrially advantageous.

The first conceptalso provides a method for producing glucose dehydrogenase by culturing a eukaryotic microorganism, and extracting and purifying GDH having the above properties. Such a eukaryotic microorganism includes filamentous fungi, yeast and eukaryotic algae, and is not particularly limited as long as it has the above properties in this range, but is preferably filamentous fungus, more preferably filamentous fungus belonging to genus *Penicillium*, and still more preferably *Penicillium lilacinoechinulatum* or *Penicillium italicum*. Fungal strains corresponding to these species belonging to the genus can be used for the first concept, but more preferably it is better to use *Penicillium lilacinoechinulatum* NBRC6231 or *Penicillium italicum* NBRC32032.

A medium for culturing the microorganism is not particularly limited as long as the microorganism can grow and produce GDH shown in the first concept, but more preferably one containing carbon sources, inorganic nitrogen sources and/or organic nitrogen sources required for the growth of the microorganism, and more preferably is a liquid medium suitable for aeration and stirring. In the case of a liquid medium, as the carbon sources, for example, glucose, dextran, glycerol, soluble starch and sucrose are exemplified, and as the nitrogen sources, for example, ammonium salts, nitrates, amino acids, corn steep liquor, peptone, casein, meat extracts, defatted soy beans and potato extracts are exemplified. As desired, other nutrients (e.g., inorganic salts such as calcium chloride, sodium dihydrogen phosphate and magnesium chloride, and vitamins) may be contained.

The culture is performed according to a method known in the art. For example, spores or growing microbial cells of the microorganism are inoculated in the liquid medium containing the above nutrients, and the microbial cells are grown by being left to stand or being aerated and stirred, and preferably the microorganism may be cultured by being aerated and stirred. The pH value in the culture medium is preferably 5 to 9, and more preferably 6 to 8. The temperature is typically 14 to 42°C, and preferably 20 to 40°C. The culture is continued typically for 14 to 144 hours, but preferably may be terminated when the amount of expressed GDH is maximized in various culture conditions. As a tactic for finding such a time point, the change in GDH activity is monitored by sampling the culture medium and measuring GDH activity, and the time point when the increase in GDH activity with time has stopped is regarded as the peak of activity, and the culture may be terminated.

As a method for extracting GDH from the above culture medium, when GDH is accumulated in the microbial cells, only the microbial cells are collected by centrifugation or filtration, and resuspended in a solvent, preferably water or buffer. GDH in the microbial cells can be extracted in the solvent by disrupting the resuspended microbial cells by a publicly known method. As a method for disruption, a lytic enzyme can be used, or a method for physically disrupting may be used. The lytic enzyme is not particularly limited as long as it has the capacity to digest a fungal cell wall, and an example of an applicable enzyme includes "lyticase" supplied by Sigma, etc. The method for disrupting physically includes ultrasonic disruption, glass bead disruption and French press, etc. After the disruption, the debris can be removed by centrifugation or filtration to yield a GDH crude extraction solution. When GDH secreted out of the microbial cells is collected, a culture supernatant obtained by centrifuging or filtrating to remove the microbial cells may be taken as a crude GDH extract for use in the following steps.

As the culture method of the first concept, a solid culture can also be employed. Preferably, the eukaryotic microorganism having a GDH producing capacity of the first conceptis grown on a bran such as wheat under appropriately controlled temperature and humidity. At that time, the culture may be performed by leaving to stand, or may be mixed by stirring. GDH is extracted by adding a solvent, preferably water or a buffer, to the culture to dissolve GDH and removing solid matter such as microbial cells and bran.

It is also possible to produce GDH of the first concept by cloning (or chemically synthesizing) the nucleic acid (hereinafter described as a GDH gene) encoding the GDH protein, and isolating/purifying GDH from the culture of a transformed microorganism containing an expression vector bearing the nucleic acid or inserting the nucleic acid in genomic DNA by recombination.

Cloning of an enzyme gene can be performed according to a publicly known method. A desired enzyme is completely or partially purified from tissue or cells producing the enzyme, and an amino acid sequence at the N terminus is determined by Edman analysis and mass spectrometry. The amino acid sequence is likewise determined for peptides obtained by partially digesting the enzyme using site specific endopeptidase. Using an oligonucleotide having a base sequence corresponding to the amino acid sequence determined in this way as a probe, the gene is cloned from a cDNA library or genomic library by colony (or plaque) hybridization. As the probe, a GDH gene partial sequence amplified by PCR with genomic DNA or cDNA from the organism producing the enzyme as a template using the above oligonucleotide as a primer may be used. Alternatively, the base sequence of the full length gene may be determined by determining flanking sequences of the above partial sequence by the inverse PCR method or RACE method, and the portion corresponding to the GDH gene may be amplified by PCR. Alternatively, the gene may be cloned by screening the genomic DNA or cDNA with an antibody using the antibody against the enzyme or a partially digested product thereof.

The nucleic acid encoding the GDH of the first concept is the nucleic acid encoding a polypeptide composed of the amino acid sequence represented by SEQ ID NO:2 or 4, or a peptide which is substantially identical thereto. Examples of such a nucleic acid sequence include the sequences represented by SEQ ID NOS:1 and 3, and further the sequence encoding the portion corresponding to the signal sequence may be deleted in the polypeptide. Alternatively, the nucleic acid sequence may be substituted with other codons encoding identical or similar amino acids throughout the sequence, or when introduced into the eukaryotic host such as filamentous fungi, insect cells or animal cells, a sequence corresponding to an intron may be inserted. Also, the nucleic acid encoding the GDH of the first conceptcan include a nucleic acid comprising the base sequence which hybridizes a base sequence complementary to the base sequence represented by SEQ ID NO:1 or 3 under stringent conditions and encoding a protein having substantially the same nature as in the protein having the amino acid sequence represented by SEQ ID NO:2 or 4. Those skilled in the art can easily select stringent conditions by changing the temperature in the hybridization reaction and the washing and salt concentrations in the hybridization reaction solution and the washing solution. Specifically, the conditions where the hybridization is performed in 6 x SSC (0.9 M NaCl, 0.09 M trisodium citrate) or 6 x SSPE (3M NaCl, 0.2 M $NaH_2PO_4$, 20 mM EDTA 2Na, pH 7.4) at 42°C and further the washing is performed with 0.5 x SSC at 42°C are included as one example of the stringent conditions of the first concept, but the stringent conditions are not limited thereto. Preferably, the conditions where the hybridization is performed in 50% formamide, 6 x SSC (0.9 M NaCl, 0.09 M trisodium citrate) or 6 x SSPE (3 M NaCl, 0.2 M $NaH_2PO_4$, 20 mM EDTA 2Na, pH 7.4) at 42°C and further the washing is performed with 0.1 x SSC at 42°C are included. The nucleic acid may be DNA or RNA, or DNA/RNA chimera, but preferably is DNA. When the nucleic acid is RNA, the exemplified base sequences are read by changing "t" to "u".

The first conceptalso provides a nucleic acid molecule comprising a nucleic acid encoding the GDH of the first concept. As such a nucleic acid molecule, a recombinant vector obtained by inserting the GDH gene into a plasmid vector or a viral vector can be exemplified. The recombinant plasmid of the first conceptpreferably can keep its replication or autonomically replicate in the host cells, but in the case of intending to introduce the desired nucleic acid into genomic DNA, the recombinant plasmid is not always necessary for replication in the host cells. A vector suitable for a recombinant expression system of the host of the prokaryotic and/or eukaryotic cells can be used. It is preferable that the vector preferably has a restriction enzyme site at a site to which a target nucleic acid is inserted to, and more preferably at a controllable site downstream of a functional promoter in the host cell. It is possible to ligate the above GDH gene using an appropriate restriction enzyme and ligase, or if necessary a linker or an adaptor DNA. Alternatively, if the GDH gene is a gene fragment amplified using a DNA polymerase such as Taq polymerase which adds one base to an amplified end, it is also possible to connect the GDH gene to the vector by TA cloning.

For the purpose of enhancing the solubility of a target protein expressed in the culture or making it easy to purify the GDH protein from the culture medium, a reporter gene and a tag sequence may be connected to the GDH gene in the nucleic acid molecule. Examples of such a sequence include a glutathione-S-transferase gene, a maltose-binding protein gene and 6 x His tag. A spacer sequence may be further inserted between these genes or the tag sequence and the GDH gene.

The vectors used for the first conceptinclude, but are not limited to, for example, pBR322, pUC18, pBluescript and SK(-) for *Escherichia coli,* pSH19 and pSH15 for yeast, and pUB110 and pTP5 for *Bacillus subtilis*, Examples of the functional promoter in the host cell include, but are not limited to, trp promoter and lac promoter for *Escherichia coli,* GAP promoter and AGH promoter for yeast, and SPO1 promoter and penP promoter for *Bacillus subtilis.* It is also preferable that the plasmid contains a selection marker gene for selecting transformants, and examples of such a marker gene include genes resistant to antibiotics typified by ampicillin, kanamycin, tetracycline, chloramphenicol and hygromycin, or genes which compensate via auxotrophic mutation in the host,

As the host into which the produced nucleic acid molecule is introduced, insect cells, animal cells and higher plant cells can be used in addition to cells from microorganisms such as bacteria, Actinomycetes, yeast and filamentous fungi, but preferably the host is a microorganism. The method for transformation can be performed according to a method publicly known in the art, is not particularly limited, and can be performed by electroporation. In addition, competent cells obtained by treatment with a cell wall lytic enzyme or a chemical can be used. When the nucleic acid molecule is introduced using them, the nucleic acid molecule is mixed with the competent cells, and the mixture is incubated, or a heat shock is further given to the mixture.

The transformed microorganism is cultured in accordance with the above method for culturing the microorganism. In order to selectively grow only the transformed microorganism with a target nucleic acid molecule introduced therein, preferably, the medium in which the antibiotic corresponding to the marker gene has been added or the minimum medium in which a substance corresponding to the compensated auxotrophy has been removed is used to refine the culture. When GDH is produced by the culture, in order to intensify the expression of the GDH, it is possible to add an expression-inducing substance corresponding to each promoter, or provide a suitable temperature condition if the promoter is induced by the temperature condition. The GDH can be extracted from the culture medium after the culture according to the above method for extracting the GDH from the culture of a eukaryotic microorganism.

The GDH can be purified by appropriately combining various separation technologies typically used depending on the fraction in which GDH activity is detected. The GDH can be purified from the above GDH extraction solution by appropriately selecting a method from publicly known separation methods such as salting out, solvent precipitation, dialysis, ultrafiltration, gel filtration, unmodified PAGE, SDS-PAGE, ion exchange chromatography, hydroxyapatite chromatography, affinity chromatography, reverse phase high performance liquid chromatography and isoelectric focusing. In particular, when the tag is added to the GDH, the column having the affinity to the tag can be used. For example, when the 6 x His tag is added, it is possible to easily enhance the purity of the GDH by the use of a nickel column.

It is also possible to add various stabilizing agents into the extracted GDH solution or the purified GDH solution. Examples of such a substance can include, for example, sugars and sugar alcohols typified by mannitol, trehalose, sucrose, sorbitol, erythritol, glycerol, etc., amino acids typified by glutamic acid, arginine, etc., and proteins and peptides typified by bovine serum albumin, ovalbumin and various chaperones. These substances may be used alone or two or more may be appropriately selected and used simultaneously.

The GDH of the first conceptcan be provided in a liquid form, but can be powderized by lyophilization, vacuum drying or spray drying. At that time, the GDH can be dissolved in a buffer, or the like, and it is preferable to further add sugars/sugar alcohols, amino acids, proteins and peptides as excipients or the stabilizing agents. The GDH can be further granulated after being powderized.

The composition of the buffer used for the extraction, purification and powderization of the GDH described above is not particularly limited, but could be those having a buffer capacity in the range from pH 5 to 8, and for example, buffers such as Tris hydrochloride and potassium phosphate, and Good's buffers such as BES, Bicine, Bis-Tris, CHES, EPPS, HEPES, HEPPSO, MES, MOPS, MOPSO, PIPES, POPSO, TAPS, TAPSO, TES and Tricine are included.

The Second Concept

The second concept the method for producing GDH characterized in that a mutation is introduced in a signal peptide sequence present in the N terminal region of GDH, thereby increasing the amount of expressed GDH compared with the amount before introduction of the mutation in the method for producing the recombinant GDH derived from filamentous fungus.

As a result of an extensive study on acquiring a GDH protein in high yield, , the present inventors first found the gene DNA predicted to encode glucose dehydrogenase as an original gene DNA for modification by utilizing the genomic information of *Aspergillus oryzae.*

The present inventors found the gene DNA predicted to encode FAD-dependent glucose dehydrogenase by utilizing the NCBI database.

The present inventors predicted from Non-patent Documents 1 to 4 and the NCBI database that the DNA (gene) encoding the protein having glucose dehydrogenase activity derived from *Aspergillus oryzae* could be easily identified. And further, the inventors thought that it was also easy to produce a recombinant vector containing the gene and a transformant transformed therewith, and to purify a protein encoded by the gene expressed by the transformant. Specifically, the present inventors attempted to culture *Aspergillus oryzae,* purify glucose dehydrogenase (GDH) from its culture supernatant using various chromatography methods, make a probe by analyzing its terminal amino acid sequence, and isolate the gene encoding the glucose dehydrogenase, with reference to the methods described in Non-patent Documents 1 to 4 and publicly known technologies.

Although the present inventors studied various methods, it was found that it was difficult to obtain a GDH preparation with high purity whose band could be clearly identified on SDS-PAGE from the culture supernatant of *Aspergillus oryzae* TI strain by ordinary purification methods using salting out and chromatography methods. It was speculated that sugar chains supposed to be bound to the enzyme protein were one of the causes to making the purification and identification difficult. Therefore, the inventors had to determine to give up the cloning utilizing the partial amino acid sequence, which is one of the standard methods for acquiring the gene. Thus, with much trial and error, it was extremely difficult to acquire the gene, but as a result of an extensive study, the present inventors isolated the gene encoding the GDH derived from *Aspergillus oryzae.* The details will be described later in the Experiments.

The present inventors further conducted extensive studies on acquiring GDH genes derived from other species, and isolated the gene encoding GDH derived from *Penicillium lilacinoechinulatum* and the gene encoding GDH derived from *Aspergillus terreus*. The details will be described later in the Experiments.

The gene DNA of the second concept can include those in which codon usage has been changed to enhance the expression of the GDH.

A signal peptide is present at the N terminus of a mature protein in the form of an extended peptide having 15 to 30 residues. The peptide sequence has a hydrophobic amino acid region, adheres to the cytoplasmic reticulum cistern of a nascent polypeptide, directs a protein to pass through the cistern, and is cleaved from the protein after the protein passes through the cistern.

The recombinant GDH derived from filamentous fungi of the second concept is not limited, and examples include those that use genus *Aspergillus* and genus *Penicillium* as GDH gene sources.

These recombinant GDH can be obtained as a water soluble fraction containing the GDH by yielding the gene encoding the GDH by PCR, inserting this gene into an expression vector, culturing a transformant obtained by transforming an appropriate host, collecting microbial cells from the culture medium by centrifugation, subsequently disrupting the microbial cells by a mechanical method or an enzymatic method using lysozyme, and if necessary, adding a chelating agent such as EDTA or a surfactant for solubilization. Alternatively, by the use of an appropriate host-vector system, it is possible to secret the expressed GDH directly in the medium.

In the second concept, it is possible to lower the function of the signal peptide by deleting or substituting a part of the amino acid sequence of the signal peptide present at the N terminal region of GDH.

For example, in GDH having the amino acid sequence described in SEQ ID NO: 10, it is possible to lower the function of the signal peptide by deleting a part or all of the amino acid sequence MLFSLAFLSALSLATASPAGRA present at its N terminus. Alternatively, it is also possible to lower the function of the signal peptide by substituting and/or inserting 1 to 22 amino acid residues.

A specific position to be substituted, for example, is a cleaved site in the signal peptide. Preferably, it is possible to lower the function by substituting alanine corresponding to the C terminus in the signal peptide with another amino acid.

Whether the expression amount of the objective enzyme has been enhanced or not compared with the state where the signal peptide sequence is present can be identified by comparing total activity values per 1 mL of the medium before and after introducing the mutation into the sequence. The modification of the signal peptide sequence can be confirmed by N terminal amino acid sequencing using Edman degradation.

It was found that the amount of expressed GDH protein was increased by deleting the function of the signal peptide present in its N terminal region.

As a tool that predicts the signal peptide, PSORT and Signal P software have been frequently used. They are available from the web sites of psort.nibb.ac.jp/ and www.cbs.dtu.dk/services/SignalP-2.0/, respectively.

Using Signal P software, the cleavage site of the signal peptide was predicted in the amino acid sequence (SEQ ID NO:9) deduced from the GDH gene derived from *Aspergillus oryzae*. Consequently, it was predicted that the signal peptide was potentially cleaved between alanine at position 16 and serine at position 17 or between alanine at position 22 and lysine at position 23 (FIG. 8).

For example, the above GDH gene derived from *Aspergillus oryzae* is inserted into an expression vector (many vectors such as plasmids are known in the art), and an appropriate host (many hosts such as *Escherichia coli* are known in the art) is transformed with the expression vector. A water soluble fraction containing GDH can be yielded by culturing the resulting transformant, collecting microbial cells from the medium by centrifugation, disrupting the microbial cells by a mechanical method or an enzymatic method, e.g., using lysozyme, and if necessary, adding a chelating agent such as EDTA or a surfactant for solubilization. Alternatively, by the use of an appropriate host-vector system, it is possible to secret the expressed GDH directly in the medium.

A GDH-containing solution obtained as above may be precipitated by concentration under reduced pressure, membrane concentration, salting out treatment using ammonium sulfate or sodium sulfate, or fractional precipitation using a hydrophilic organic solvent such as methanol, ethanol or acetone. Treatment with heat and isoelectric focusing treatment are also effective purification procedures. The purified GDH can also be yielded by performing gel filtration using an absorbing agent or a gel filtration agent, absorption chromatography, ion exchange chromatography and affinity chromatography. It is preferable that the purified enzyme preparation is purified to the extent that the enzyme is detected as a single band on electrophoresis (SDS-PAGE).

These procedures can be carried out in accordance with the following references.

(a) Tanpakushitsu Jikken Protocol Vol. 1, Functional Analysis Vol. 2, Structural Analysis (Shujunsha) edited by Yoshifumi Nishimura and Shigeo Ohno.

(b) Revised Tanpakushitsu Jikken Note, Extraction and Separation/Purification (Yodosha) edited by Masato Okada and Kaori Miyazaki.

(c) Tanpakushitsu Jikken no Susumekata (Yodosha) edited by Masato Okada and Kaori Miyazaki.

Alternatively, the above procedure can be carried forward by the methods exemplified below.

The second concept further includes a vector comprising the gene encoding GDH and a transformant transformed with the vector.

The produced DNA having the genetic information of the protein is transferred into the host microorganism by ligating to the vector, which becomes the transformant that produces the modified protein.

When a plasmid is used as the vector, pBluescript, pUC18 or the like can be used when *Escherichia coli* is used as the host microorganism. As a host microorganism, for example, *Escherichia coli* W3110, *Escherichia coli* C600, *Escherichia coli* JM109, *Escherichia coli* DH5α, or the like, can be utilized. As the method for transferring the recombinant vector into the host microorganism, it is possible to employ a method of transferring the recombinant DNA in the presence of calcium ions when the host microorganism is a microorganism belonging to genus *Escherichia,* and further, an electroporation method may be used. Furthermore, commercially available competent cells (e.g., Competent High DH5α supplied by Toyobo Co., Ltd.) may also be used.

Such a gene may be extracted from the fungal strain, or can also be synthesized chemically. Furthermore, it is also possible to yield a DNA fragment containing the GDH gene by the use of PCR.

In the second concept, the method for yielding the gene encoding GDH derived from filamentous fungus includes the following methods. First, with reference to the sequence information of the GDH gene derived from *Aspergillus oryzae*, a predicted objective gene from filamentous fungus can be acquired. mRNA is prepared from the filamentous fungus, and cDNA is synthesized. The GDH gene is amplified by PCR with the cDNA yielded in this way as a template, and the recombinant vector is constructed by binding and closing this gene and the vector with DNA ligase at blunt ends or sticky ends of both DNA. The recombinant vector is transferred into a host microorganism in which the vector can replicate, and subsequently, the microorganism carrying the recombinant vector containing the gene encoding GDH is yielded by utilizing a marker of the vector.

The base sequence of the GDH gene was decoded by a dideoxy method described in Science 214:1205, 1981. The amino acid sequence of GDH was deduced from the base sequence determined as the above.

As in the above, the once selected GDH gene in the recombinant vector can be easily transferred into another recombinant vector, which can replicate therein by collecting the DNA, which is the GDH gene from the recombinant vector carrying the GDH gene, by restriction enzymes and PCR method and binding the DNA to another vector fragment. For the transformation of another microorganism with these vectors, a competent cell method by treating with calcium, an electroporation method, a protoplast method, or the like, can be used.

The GDH gene of the second concept may be those having a DNA sequence so that a part of amino acid residues is deleted or substituted in the amino acid sequence after translation of the gene, or so that other amino acid residues are added or substituted, as long as the protein encoded by the GDH gene has glucose dehydrogenase activity.

As the method for modifying the gene encoding wild type GDH, the typically performed technique to modify the genetic information is used. That is, DNA having the genetic information of the modified protein is made by converting the specific base in DNA having the genetic information of the protein or inserting or deleting the specific base. The specific methods for converting the base in the DNA include the use of commercially available kits (Transformer Mutagenesis Kit supplied by Clonetech; EXQIII/Mung Bean Deletion Kit supplied by Stratagene; QuickChange Site Directed Mutagenesis Kit supplied by Stratagene), or utilization of a polymerase chain reaction (PCR) method.

For the culture of the host microorganism that is the transformant, culture conditions can be selected in consideration of the nutritional physiological natures of the host. In many cases, the transformants is cultured in liquid culture. Industrially, it is advantageous to employ aerobic culture with stirring.

As nutrient sources of the medium, those typically used for the culture of a microorganism can be widely used. Carbon sources may be carbon compounds capable of being assimilated. For example, glucose, sucrose, lactose, maltose, lactose, molasses and pyruvic acid are used. Nitrogen sources may be usable nitrogen compounds. For example, peptone, meat extracts, yeast extracts, casein hydrolyzed products, and bean cakes extracted with alkali are used. In addition, phosphate salts, carbonate salts, sulfate salts, salts of magnesium, calcium, potassium, iron, manganese and zinc, particular amino acids and particular vitamins are used if necessary.

The culture temperature can be appropriately changed in the range in which the microorganism grows and produces GDH, and is preferably about 20 to 37°C. The culture time period is somewhat different depending on the conditions, the culture may be terminated at an appropriate time period by judging the time to be right to reach the maximum yield of GDH, and the culture time period is typically about 6 to 48 hours. In the culture of the recombinant GDH derived from filamentous fungus, it is particularly important to control the pH of the medium. It is desirable to control at pH 7.1 to 7.3 or lower, and it is particularly preferable to culture while controlling in the range of pH 6.0 to 7.3. By culturing while controlling pH in this way, it becomes possible to prepare the GDH protein derived from filamentous fungus in a large amount.

However, GDH derived from the filamentous fungus keeps high stability in an acidic region of pH 7.0 or lower, but becomes unstable at pH 7.1 to 7.3 or higher, and its activity lowers rapidly.

Therefore, in the second concept, it is preferable to control the pH at 7.3 or lower in the culture in the production of the recombinant glucose dehydrogenase (GDH) derived from the filamentous fungus.

In the production by culturing the recombinant GDH derived from the filamentous fungus, when the pH value was increased by 0.2, the activity was decreased by 10% or more compared with the original activity, and the activity was decreased by about 30% at the maximum depending on GDH. Thus, in the present patent, the efficacy of pH control at a level lower than the pH (specifically pH of 7.3 or less, preferably 7.1 or less) in the culture medium before lowering the activity in a phenomenon where the activity was decreased by 10% or more when the pH value was increased by 0.2 was presumed, and actually examined and confirmed.

In the culture of the recombinant organism, control of the pH value is generally performed because the death of microbial cells and degradation of the objective protein are concerned, and it is common to usually keep the pH vale in the neutral region. There is no other example that describes the necessity to keep the pH value neutral or lower than the case of glucose dehydrogenase derived from the filamentous fungus.

The culture medium containing the microbial cells which produce GDH can be directly collected and utilized. However, in general, according to standard methods, when the GDH is present in the culture medium, a GDH-containing solution is separated from the microorganism microbial cells by filtration or centrifugation, and subsequently utilized. When GDH is present in the microbial cells, the microbial cells are collected from the culture by filtration or centrifugation, then disrupted by a mechanical method or an enzymatic method using lysozyme and if necessary a chelating agent such as EDTA and a surfactant are added for solubilization, and GDH is separated/collected as an aqueous solution.

The GDH-containing solution obtained as above may be precipitated by concentration under reduced pressure, membrane concentration, salting out treatment using ammonium sulfate or sodium sulfate or fractional precipitation using a hydrophilic organic solvent such as methanol, ethanol or acetone. Treatment with heat and isoelectric focusing treatment are also effective purification procedures. The purified GDH can also be yielded by subsequently performing gel filtration using an absorbing agent or a gel filtration agent, absorption chromatography, ion exchange chromatography and affinity chromatography.

For example, it is possible to obtain a purified enzyme preparation by separating and purifying by gel filtration using Sephadex gel (supplied by Pharmacia Biotech), or column chromatography using DEAE Sepharose CL-6B (supplied by Pharmacia Biotech) or Octyl Sepharose CL-6B (supplied by Pharmacia Biotech). It is preferable that the purified enzyme preparation is purified to the extent that the enzyme is detected as a single band on electrophoresis (SDS-PAGE).

The Third Concept

GDH is an enzyme which catalyzes the following reaction.

D-Glucose + Electron transport substance (oxidation type) → D-glucono-δ-lactone + Electron transport substance (reduction type)

GDH is the enzyme which catalyzes the reaction in which D-glucose is oxidized to generate D-glucono-1,5-lactone, and the origin and structure thereof are not particularly limited.

GDH applicable to the method of the third concept is not particularly limited as long as it is soluble glucose dehydrogenase (GDH).

As a coenzyme, for example, a flavin compound can be taken.

GDH (FAD-bound GDH) applicable to the method of the present invention and taking FAD as the coenzyme is not particularly limited, and includes, for example, those derived from microorganisms which are filamentous fungi belonging to genus *Penicillium* or *Aspergillus* belonging to the category of eukaryotic organisms. These fungal strains are easily available by requesting assignment from a culture collection for each respective fungus.

For example, *Penicillium lilacinoechinulatum* belonging to genus *Penicillium* has been registered under accession number NBRC6231 at Biological Resource Center, National Institute of Technology and Evaluation. *Aspergillus terreus* belonging to genus *Aspergillus* has been registered under accession number NBRC33026 at Biological Resource Center, National Institute of Technology and Evaluation.

For *Aspergillus oryzae,* the GDH gene can be acquired according to the procedure outlined in Experiment 1B.

The applicable GDH of the third concept may be those having a DNA sequence so that a part of the amino acid residues is deleted or substituted in the amino acid sequence or so that other amino acid residues are added or substituted, as long as the GDH has glucose dehydrogenase activity.

Such a modification can be easily carried out by those skilled in the art using publicly known technologies in the art. For example, various methods for substituting or inserting a base sequence of a gene encoding a protein in order to introduce a site directed mutation into the protein have been described in Sambrook et al., Molecular Cloning; A Laboratory Manual 2nd edition (1989) Cold Spring Harbor Laboratory Press, New York.

For example, a water soluble fraction containing GDH can be obtained by culturing a natural microorganism producing the above GDH or culturing a transformant obtained by inserting a gene encoding the natural GDH directly or after being mutated into an expression vector (many vectors are known in the art, e.g., plasmids) and transforming a host (many hosts are known in the art, e.g., *Escherichia coli*) with the expression vector, collecting microbial cells from the medium by centrifugation, disrupting the microbial cells by a mechanical method or an enzymatic method using lysozyme and if necessary adding a chelating agent such as EDTA or a surfactant for solubilization. Alternatively, by the use of an appropriate host-vector system, it is possible to secret the expressed GDH directly in the medium.

A GDH-containing solution obtained as above may be precipitated by concentration under reduced pressure, membrane concentration, salting out treatment using ammonium sulfate or sodium sulfate, or fractional precipitation using a hydrophilic organic solvent such as methanol, ethanol or acetone. Treatment with heat and isoelectric focusing treatment are also effective purification procedures. Purified GDH can also be yielded by performing gel filtration using an absorbing agent or a gel filtration agent, absorption chromatography, ion exchange chromatography and affinity chromatography. It is preferable that the purified enzyme preparation is purified to the extent that the enzyme is detected as a single band on electrophoresis (SDS-PAGE).

Before or after the above step, in order to increase the percentage of a holo type GDH relative to the total GDH enzyme protein, the treatment with heat preferably at 25 to 50°C, and more preferably 30 to 45°C may be performed.

The concentration of GDH in the third concept is not particularly restricted. An appropriate range varies depending on the properties of the enzyme used; however, the concentration may be that at which those skilled in the art can actually determine as being capable of measuring glucose with sufficient reliability using the enzyme.

For example, the concentration of FAD-GDH in the third concept is not particularly restricted, but in the case of the solution, a lower limit is preferably 0.01 U/mL, more preferably 0.1 U/mL and still more preferably 0.2 U/mL. An upper limit is preferably 5000 U/mL, more preferably 500 U/mL and still more preferably 50 U/mL. The similar concentration is desirable in a powder preparation or a lyophilized product. For the purpose of preparing a powder preparation, it is possible to make the concentration 5000 U/mL or more.

The medium for culturing the microorganism is not particularly limited as long as the microorganism can grow and produce GDH shown in the third concept, but more suitable are those containing carbon sources, inorganic nitrogen sources and/or organic nitrogen sources required for the growth of the microorganism, and more preferably the medium is a liquid medium suitable for aeration and stirring. In the case of a liquid medium, as carbon sources, for example, glucose, dextran, soluble starch and sucrose are exemplified, and nitrogen sources, for example, ammonium salts, nitrates, amino acids, corn steep liquor, peptone, casein, meat extracts, defatted soy beans and potato extracts are exemplified. As desired, other nutrients (e.g., inorganic salts such as calcium chloride, sodium dihydrogen phosphate and magnesium chloride, and vitamins) may be contained.

The culture is performed according to a method known in the art. For example, spores or growing microbial cells of the microorganism are inoculated in a liquid medium containing the above nutrients, and the microbial cells are grown by being left to stand or aeration and stirring, and preferably the microorganism may be cultured by aeration and stirring. The pH value of the culture medium is preferably 5 to 9, and more preferably 6 to 8. The temperature is typically 14 to 42°C, and preferably 20 to 40°C. The culture is continued typically for 14 to 144 hours, but may be terminated when the amount of expressed GDH is maximized in various culture conditions. As a tactic for finding such a time point, the change in GDH activity is monitored by sampling the culture medium and measuring the GDH activity, and the time point when the increase in GDH activity over time is stopped is regarded as a peak of the activity, and the culture may be terminated.

As a method for extracting GDH from the above culture medium, when GDH accumulated in microbial cells is collected, only the microbial cells are collected by centrifugation or filtration, and resuspended in a solvent, preferably water or a buffer. GDH in the microbial cells can be extracted in the solvent by disrupting the resuspended microbial cells by a publicly known method. As the method for disruption, a lytic enzyme can be used, or a method for physically disrupting may be used. The lytic enzyme is not particularly limited, and an example of an applicable enzyme includes "lyticase" supplied by Sigma. The method for physical disruption includes ultrasonic disruption, glass bead disruption, homogenizing disruption, etc. After the disruption, debris can be removed by centrifugation or filtration to yield a GDH crude extraction solution.

The culture method of the third concept can also employ a solid culture. Preferably, a eukaryotic microorganism having a GDH producing capacity of the third concept is grown on a bran such as wheat under the appropriately controlled temperature and humidity. At that time, the culture may be performed by being left to stand, or may be mixed by stirring. GDH is extracted by adding the solvent, preferably water or a buffer to the culture to dissolve GDH and removing solid matters such as microbial cells and bran by centrifugation and filtration.

The GDH can be purified by appropriately combining various separation technologies typically used depending on the fraction in which GDH activity is detected. The GDH can be purified from the above GDH extraction solution by suitably selecting the method from publicly known separation methods such as salting out, solvent precipitation, dialysis, ultrafiltration, gel filtration, unmodified PAGE, SDS-PAGE, ion exchange chromatography, hydroxyapatite chromatography, affinity chromatography, reverse phase high performance liquid chromatography, isoelectric focusing electrophoresis, etc.

One mode of the method for enhancing the heat stability of GDH of the third concept comprises, in a composition containing a soluble glucose dehydrogenase (GDH) capable of binding to a coenzyme, a step of making (1) the coenzyme coexist with (2) one or more compounds selected from amino acids and sugars that are not a substrate for the coenzyme.

Preferable compounds to be added include one or more selected from the group consisting of trehalose, mannose, melezitose, sodium gluconate, sodium glucuronate, galactose, methyl-α-D-glucoside, cyclodextrin, α-D-melibiose, sucrose, cellobiose, glycine, alanine, serine and BSA.

The concentration of each compound made to coexist is not particularly limited. In the case of the solution, the lower limit is 0.001% by weight, preferably 0.01%, more preferably 0.1%. In terms of the risk of contamination with foreign substances, the upper limit is preferably 30% by weight, more preferably 20% and still more preferably 10%. The concentration of the compound described in the Experiments is represented by % by weight relative to the solvent in the case of the solution, and represented by % by weight relative to the GDH enzyme in powdered dry matter. For example, in powdered dry matter, when a stabilizing agent at 60% is added to 40 mg/mL of GDH, 24 mg of the stabilizing agent is added, and at that time, the concentration in the solution is about 2.4%. In an experiment for examining the stabilization in powder, the GDH in the powder was stabilized within a concentration range in which thermal stabilization was observed in the solution. It is easily presumed that the stabilization effect is exerted in the powder in the same concentration range as in the solution.

The concentration of each compound made to coexist is not particularly limited. In the solution, the lower limit is preferably 0.01 mM, more preferably 0.1 mM, and still more preferably 1 mM. The upper limit is preferably 10 M, more preferably 5 M, and still more preferably 1 M.

When a powder or lyophilized matter is produced, it is possible to acquire a dry preparation having the same effect as the solution by drying the composition containing the compound at a concentration equivalent to that in the solution.

The concentration of the compound described in the Experiments is a final concentration when stored by coexisting with the GDH enzyme.

The GDH-containing composition of the third concept can be provided in a liquid form, but can be powderized by lyophilization, vacuum drying, spray drying, or the like. At that time, the GDH can be dissolved in the buffer for use, and further sugars/sugar alcohols, amino acids, proteins, peptides, etc., other than the above compounds can be added as excipients or stabilizing agents. The GDH can be further granulated after being powderized.

The composition of the buffer used for the extraction, purification and powderization of the GDH described above and a stability test is not particularly limited, and may be those having a buffer capacity in the range from pH 5 to 8, and, for example, buffers such as boric acid, Tris hydrochloride, potassium phosphate, etc., and Good's buffers such as BES, Bicine, Bis-Tris, CHES, EPPS, HEPES, HEPPSO, MES, MOPS, MOPSO, PIPES, POPSO, TAPS, TAPSO, TES and Tricine are included. Also buffers based on dicarboxylic acid such as phthalic acid, maleic acid, glutaric acid, etc., can also be included.

Among them, only one may be applied or two or more may be used. Furthermore, the buffer may be a composite composition of one or more containing one other than the above.

The concentration of these buffers to be added is not particularly limited as long as it is in a range having buffering capacity. The upper limit is preferably 100 mM or less, and more preferably 50 mM or less. The preferable lower limit is 5 mM or more.

The content of the buffer in the powder or the lyophilized matter is not particularly limited, and the buffer is used in the range of preferably 0.1% (weight ratio) or more, and more preferably 0.1 to 80% (weight ratio).

As these, various commercially available reagents can be used.

It is desirable that the various compounds described above are added before making a reagent for measuring the glucose level, a glucose assay kit or a glucose sensor, but may be added upon measurement. They can also be added in a step solution in each step of extracting, purifying or powderizing GDH.

Enhancement of the thermal stability referred to herein means an increase in the residual ratio (%) of the GDH enzyme kept after applying the treatment with heat at a certain temperature for a certain time period to the composition comprising the GDH enzyme. In the present third concept, the residual ratio of the enzyme is calculated by comparing the activity in the GDH solution after treatment with heat at a certain temperature for a certain time period with the activity kept nearly complete in the sample stored at 4°C, which is taken as 100%. When this residual ratio was increased compared with that when the compound had not been added, it was determined that the thermal stability of GDH was enhanced.

Specifically, whether the stability was enhanced or not was determined as follows.

In the method for measuring the activity to be described in the method for measuring the GDH enzyme activity described later, the GDH activity value (a) of the solution stored at 4°C and the GDH activity value (b) after treatment with heat at a certain temperature for a certain time period were measured, a relative value [(b)/(a) x 100] when the activity value (a) was made 100 was calculated. This relative value was made the residual ratio (%). Comparing the presence with the absence of the added compound, when the residual ratio was increased by the addition of a compound, it was determined that the thermal stability was enhanced.

The effect of the third concept becomes remarkable in a system comprising a mediator. The mediator applicable to the method of the third concept is not particularly limited, and includes the combination of phenazine methosulfate (PMS) with 2,6-dichlorophenolindophenol (DCPIP), the combination of PMS with nitroblue tetrazolium, (NBT), DCPIP alone, ionic ferricyanide (as the compound, potassium ferricyanide) alone, ferrocene alone and nitrosoaniline alone. Among them, the ionic ferricyanide (as the compound, potassium ferricyanide) is preferable.

These mediators are variously different in sensitivity. Thus, the concentration of the mediator to be added is not necessarily defined uniformly, and generally it is desirable to add at a concentration of 1 mM or more.

These mediators may be added upon measurement or can also be previously contained when producing the reagent for measuring the glucose level, the glucose assay kit or the glucose sensor described later. At that time, the mediator can be added so that it is dissociated to become ions regardless of a liquid state or a dry state.

In the third concept, it is possible to make various components coexist if necessary. For example, the surfactant and the like may be added.

In the third concept, the glucose level can be measured by the following various methods.

The reagent for measuring the glucose level, the glucose assay kit or the glucose sensor of the third concept can take various forms such as a liquid (aqueous solution, suspension, etc.), a powderized form by vacuum drying or spray drying, a lyophilized form, etc. The drying method is not particularly limited, and may be performed in accordance with standard methods. The composition comprising the enzyme of the third concept is not limited to lyophilized matter, and may be in a solution state obtained by re-dissolving the dry matter.

In the third concept, the glucose level can be measured by the following various methods.

Reagent for measuring glucose level

The reagent for measuring the glucose level disclosed herein typically includes the reagents such as GDH, a buffer and mediator required for the measurement, glucose standard solutions for making a calibration curve, and instructions for use. The kit disclosed herein can provide the lyophilized reagent or the solution in an appropriate storage solution. Preferably, the GDH as disclosed herein is provided as a holoenzyme, but can be provided as an apoenzyme and converted into the holoenzyme before use.

Glucose assay kit

Disclosed herein isa glucose assay kit containing GDH as disclosed herein The glucose assay kit disclosed herein contains GDH as disclosed hereinin a sufficient amount for at least one assay. Typically, the kit includes a buffer and a mediator essential for the assay in addition to GDH, glucose standard solutions for making the calibration curve, and instructions for use. The GDH as disclosed hereincan be provided in various forms, for example, as a lyophilized reagent or as a solution in an appropriate storage solution. Preferably, the GDH as disclosed herein is provided as a holoenzyme, but can be provided as an apoenzyme and converted into the holoenzyme before use.

Glucose sensor

Disclosed herein is also a glucose sensor using the GDH as disclosed herein. As an electrode, a carbon electrode, a gold electrode, a platinum electrode and the like are used, and the enzyme disclosed herein is immobilized on this electrode. As a method for immobilization, a method of using a crosslinking reagent, a method of enfolding in a polymer matrix, a method of covering with a dialysis membrane, photo-crosslinkable polymers, conductive polymers, redox polymers, etc., are available. Preferably, the GDH as disclosed herein is immobilized on the electrode as a holoenzyme, or it is possible to immobilize it as an apoenzyme and supply a coenzyme as another layer or in a solution. Typically, the GDH as disclosed herein is immobilized on a carbon electrode using glutaraldehyde, and subsequently glutaraldehyde is blocked by treating with a reagent having an amine group.

The glucose concentration can be measured as follows. The buffer is placed in a cell at constant temperature, a mediator is added and the temperature is kept constant. As an action electrode, the electrode on which GDH, as disclosed herein, has been immobilized is used, and a counter electrode (e.g., platinum electrode) and a reference electrode (e.g., Ag/AgCl electrode) are used. A certain voltage is applied to the carbon electrode and the current becomes constant, and subsequently an increase in the current is measured by adding the sample containing glucose. According to the calibration curve made from the glucose solutions at standard concentrations, the glucose concentration in the sample can be calculated.

The mediator used for the composition for measuring the glucose level, the glucose assay kit, the glucose sensor or the method for measuring the glucose level is not particularly limited, and preferably 2,6-dichlorophenol-indophenol (abbreviated as DCPIP) and ferrocene or derivatives thereof (e.g., potassium ferricyanide, phenazine methosulfate) could be used. As these mediators, commercially available products can be obtained.

Test Experiment

In the present disclosure, the activity of glucose dehydrogenase is measured as follows.

<Reagents>

50 mM PIPES buffer pH 6.5 (containing 0.1% Triton X-100) 14 mM 2,6-dichlorophenol-indophenol (DCPIP) solution 1 M D-glucose solution

The reaction reagent is made by mixing 15.8 mL of the PIPES buffer, 0.2 mL of the DCPIP solution and 4 mL of the D-glucose solution described above.

<Measurement conditions>

The reaction reagent 2.9 mL is preliminarily heated at 37°C for 5 minutes. The GDH solution (0.1 mL) is added and gently mixed, subsequently the change in absorbance at 600 nm is recorded for 5 minutes using a spectrophotometer controlled at 37°C using water as the control, and the change in absorbance per one minute ($\Delta OD_{TEST}$) is calculated from the linear portion of the record. As a blank test, the solvent in which GDH is dissolved is added to the reagent mixture in place of the GDH solution, and the change in absorbance ($\Delta OD_{BLANK}$) per one minute is measured. The GDH activity is calculated from these values according to the following formula. One unit (U) in GDH activity is defined as the amount of enzyme that reduces 1 μM DCPIP for one minute in the presence of 200 mM D-glucose.

$$\text{Activity (U/mL)} = [-(\Delta OD_{TEST} - \Delta OD_{BLANK}) \times 3.0 \times \text{dilution scale}]/(16.3 \times 0.1 \times 1.0)$$

In the above formula, 3.0 represents a liquid amount (mL) of the reaction reagent + the enzyme solution, 16.3 represents a millimolar molecular absorbance coefficient ($cm^2/\mu mol$) in the conditions for measuring this enzyme, 0.1 represents the liquid amount of the enzyme solution (mL), and 1.0 represents a light path length (cm) of the cell.

EXPERIMENTAL

The present invention will be more specifically described below with reference to some of the Experiments, but is not limited to these Experiments.

Experiments 1A-16A

Experiment 1A

Acquisition of GDH Derived from Filamentous Fungi Belonging to Genus *Penicillium*

Using *Penicillium lilacinoechinulatum* NBRC6231 and *Penicillium italicum* NBRC32032 (purchased from the Independent Administrative Institution, National Institute of Technology and Evaluation) as GDH-producing fungi, an L-dried sample of each fungal strain was inoculated on a potato dextrose agar medium (supplied by Difco) and incubated at 25°C to restore. Fungal threads restored on the plate were collected together with the agar and then suspended in filter-sterilized water. Six liters of a production medium (1% malt extract, 1.5% soybean peptide, 0.1% $MgSO_4 \cdot 7H_2O$, 2% glucose, pH 6.5) was prepared in two 10 L jar fermenters and sterilized in an autoclave at 120°C for 15 minutes. Then, the above fungal thread suspension was added thereto, and culture was started. The culture was performed under the conditions of a temperature of 30°C, an aeration amount of 2 L/minute and a stirring frequency of 380 rpm. The culture was stopped 64 hours after the start of the culture, and microbial cells from each fungal strain were collected on filter paper by suction filtration using a Nutsche filter. The microbial cells were resuspended in 3 L of 20 mM K-phosphate buffer (pH 6.5), and disrupted using a French press at a pressure of 130 MPa. The disrupted cell solution was dispensed into 500 mL centrifuge tubes and cell debris was precipitated by centrifuging at 8000 rpm for 15 minutes using a high speed cooling centrifuge supplied by Hitachi Ltd. The supernatant was concentrated to 1/10 amount using a hollow fiber module for ultrafiltration with a molecular weight cut-off of 10,000, and ammonium sulfate was added and dissolved in the concentrated solution to a final concentration of 60% saturation (456 g/L). Subsequently, the resulting solution was centrifuged at 8000 rpm for 15 minutes using a high speed cooling centrifuge supplied by Hitachi Ltd. to precipitate residue. Then, the supernatant was adsorbed onto an Octyl-Sepharose column, and fractions having GDH activity were collected by elution with a concentration gradient of ammonium sulfate from 0.6 to 0.0 saturation. Salting out was performed by subjecting the resulting GDH solution to gel filtration on a G-25 Sepharose column and collecting protein fractions. Ammonium sulfate corresponding to 0.6 saturation was added to the solution after the salting out. This mixture was adsorbed onto a Phenyl-Sepharose column, and fractions having GDH activity were collected by elution with a concentration gradient of ammonium sulfate from 0.6 to 0.0 saturation. The fractions were heated at 50°C for 45 minutes and centrifuged to yield a supernatant. The solutions obtained through the above steps were used as purified GDH samples.

Experiment 2A

Estimation of Molecular Weight

The GDH solution (25 μL) derived from each of NBRC6231 and NBRC32032 purified in Experiment 1A was applied to TSK-GEL G300SW (7.5 mm x 300 mm) supplied by Tosoh Corporation and buffered with 50 mM Tris-HCl (pH 7.5), and was fractionated at a flow rate of 0.5 mL/minute. For the elution of GDH derived from NBRC6231, the elution time was determined from the position at which the peak appeared, by monitoring the absorbance at 280 nm. The eluate of GDH derived from NBRC32032 was collected using a fraction collector, the GDH activity in each fraction was measured according to Test Experiment 1, the peak fraction was specified, and the time required for the elution was calculated therefrom. Based on the elution time calculated, the molecular weight of the GDH protein was calculated from a standard curve previously made using standard protein solutions (MW-Marker (MW 12,400 to 290,000) supplied by Oriental Yeast Co., Ltd.). As a result, it was estimated that GDH derived from NBRC6231 and GDH derived from NBRC32032 have molecular weights of about 270 kDa and 79 to 93 kDa, respectively.

Experiment 3A

Optimal Reaction Temperature

In order to know the optimal reaction temperature of the purified GDH solutions derived from NBRC6231 and NBRC32032 obtained in Experiment 1A, by making the preliminary heating temperature and the temperature of the reaction reagent during the reaction 25, 37, 40, 45, 50, 55, 60 and 65°C, the activity under each condition was measured. FIG. 1 is a graph showing the relative activity when the maximum activity value was made 100. From the above, it was found that the optimal reaction temperature of GDH derived from NBRC6231 is in the range higher than 45°C and lower than 55°C and is about 50°C and that the optimal reaction temperature of GDH derived from NBRC32032 is in the range higher than 55°C and lower than 65°C and is about 60°C.

Experiment 4A

Optimal Reaction pH

In order to know the optimal reaction pH of the purified GDH solutions derived from NBRC6231 and NBRC32032 obtained in Experiment 1A, reaction reagents were prepared using 50 mM K-phosphate buffers in the range from pH 5.5 to 8.0 instead of the PIPES buffer in the reagent shown in the above Test Experiment, and the activity was measured using these reagents according to the procedure in the Test Experiment. FIG. 2 is a graph showing the relative activity at each pH when the maximum activity value was made 100. Both GDHs derived from NBRC6231 and NBRC32032 exhibited maximum activity at around pH 6.5. From the above, it was found that the optimal reaction pH of both GDHs derived from NBRC6231 and NBRC32032 is in the range higher than 6.0 and lower than 7.0 and is about 6.5.

Experiment 5A

Temperature Stability

In order to know the temperature stability of the purified GDH solutions derived from NBRC6231 and NBRC32032 obtained in Experiment 1A, each GDH solution was diluted with 20 mM K-phosphate buffer (pH 6.5) to have an activity of 1 U/mL, this diluted GDH solution was heated at each temperature in the range of 37°C to 65°C for 15 minutes using a heat bath, and the activities before and after the treatment were compared. The activity was measured according to the Test Experiment described above. FIG. 3 is a graph showing the residual activity after heating relative to the activity before heating. In GDH derived from NBRC6231, the residual activity was 93% or 44% after treatments at 55°C or 60°C, respectively. In GDH derived from NBRC32032, the residual activity was 98% or 73% after treatments at 55°C or 60°C, respectively.

Experiment 6A

pH Stability

In order to know the pH stability of the purified GDH solutions derived from NBRC6231 and NBRC32032 obtained in Experiment 1A, buffers for pH 3.3 to 8.5 (pH 3 to 6: acetate buffer, pH 6 to 7: PIPES buffer, pH 7 to 8.5: Tris hydrochloride buffer) were prepared, and using these buffers, each GDH solution was diluted to have an enzyme activity of 1 U/mL. The diluted GDH solutions were incubated at 25°C for 16 hours and the activities before and after incubation were compared. FIGs. 4 (derived from NBRC6231) and 5 (derived from NBRC32032) are graphs showing the residual activity after incubation relative to the activity before incubation. GDH derived from NBRC6231 exhibited a residual activity of 80% or more in the range of pH 5 to 8 and exhibited good stability. GDH derived from NBRC32032 exhibited a residual activity of 80% or more in the range of pH 5 to 8.5, but when using the PIPES buffer, the residual activity at pH 7.4 was 77%.

Experiment 7A

In order to know the substrate specificity of the purified GDH solutions derived from NBRC6231 and NBRC32032 obtained in Experiment 1A, the activity was measured using reaction reagents in which the substances shown in Table 1A had been dissolved to a final concentration of 4 mM. Table 1A shows the activity for each substrate when the activity for glucose as the substrate was made 100%. It was found that both enzymes acted upon 2-deoxy-D-glucose and xylose, among substrates other than glucose. However, both enzymes exhibited reactivity to other sugars at levels with no practical problems.

Table 1A

| | Relative Activity | |
|---|---|---|
| Substrate (final concentration 4 mM) | NBRC6231 | NBRC32032 |
| Glucose | 100 | 100 |
| Maltose | 0.5 | 0.2 |
| Fructose | 0.2 | 0.3 |
| Arabinose | 0.2 | 0.2 |
| Glycerin | 0.1> | 1.7 |
| Sucrose | 0.6 | 1.1 |
| Melezitose | 0.2 | 0.5 |
| Sorbose | 0.1> | 0.5 |
| Ribose | 0.1> | 0.2 |
| Maltotriose | 0.2 | 1.0 |
| Maltotetraose | 0.1> | 1.6 |
| Galactose | 1.0 | 0.6 |
| Mannose | 1.6 | 0.9 |
| Xylose | 10.1 | 10.4 |
| 2-Deoxy-D-glucose | 14.3 | 17.2 |
| Trehalose | 0.3 | 0.1> |

Experiment 8A

In order to know the effects of chemicals on the purified GDH solutions derived from NBRC32032 and NBRC6231 obtained in Experiment 1A, each substance shown in Table 2 was added to a final concentration of 2 mM to the reaction reagent shown in the Test Experiment, and using the resulting mixture, the GDH activity was measured. The relative activity when the activity value in the case of no chemical being added was made 100 is shown in Table 2A. Substances commonly exhibiting a strong inhibitory effect were copper sulfate, cadmium acetate and silver nitrate. An inhibitory effect was commonly observed in zinc chloride, monoiodoacetic acid, N-ethylmaleimide and hydroxylamine. It was also found that GDH derived from NBRC6231 was inhibited by sodium azide and that GDH derived from NBRC32032 was inhibited by iron (III) chloride.

Table 2A

| | Relative activity | |
|---|---|---|
| Substance added (final concentration 2 mM) | NBRC6231 | NBRC32032 |
| $MgCl_2$ | 99.7 | 102 |
| $CaCl_2$ | 103 | 103 |
| $Ba(OAc)_2$ | 106 | 106 |
| $FeCl_3$ | 106 | 77.5 |
| $CoCl_2$ | 94.1 | 102 |
| $MnCl_2$ | 93.4 | 99.1 |
| $ZnCl_2$ | 76.2 | 75.1 |
| $Cd(OAc)_2$ | 41.0 | 41.4 |
| $NiCl_2$ | 96.6 | 101 |
| $CuSO_4$ | 2.2 | 4.5 |
| $AgNO_3$ | 20.9 | 15.0 |
| Monoiodoacetic acid | 68.7 | 73.3 |
| N-ethylmaleimide | 86.8 | 87.8 |
| Iodoacetamide | 111 | 101 |
| Hydroxylamine | 81.5 | 79.1 |
| EDTA | 113 | 104 |
| o-Phenanthroline | 106 | 103 |
| $\alpha,\alpha'$-dipyridyl | 109 | 102 |
| Boric acid | 111 | 102 |
| NaF | 111 | 102 |
| $NaN_3$ | 89.3 | 97.8 |

Experiment 9A

Km Value for D-glucose

The activity of GDHs derived from NBRC6231 and NBRC32032 was measured by changing the D-glucose concentration in the range of 200 mM or lower in the reaction reagent composition described in the Test Experiment. The Km value was calculated according to the Lineweaver-Burk plot method. As a result, GDH derived from NBRC6231 had a Km value of 13 mM and GDH derived from NBRC32032 had a Km value of 7 mM.

Experiment 10A

Application to Glucose Electrode

Carbon graphite (0.5 g) was placed in a mortar, 0.3 mL of liquid paraffin was added, and the mixture was kneaded using a pestle to make a carbon paste. The carbon paste made was kneaded into a platinum electrode, 10 μL of the purified GDH solution (500 U/mL, derived from the deposit number NBRC6231) made according to Experiment 1A was added, and air-drying was performed at room temperature for 30 minutes. A cellulose semipermeable membrane for dialysis was placed on the air-dried enzyme electrode, and secured with a plastic O-ring. The glucose electrode made in this way was used after being immersed for 30 minutes in 50 mM potassium phosphate buffer (pH 7.0) previously ice-cooled. In a cuvette warmed at 25°C, 20 mL of a reaction solution was placed, the glucose electrode (action electrode), the platinum electrode as the counter electrode and an Ag/AgCl electrode as the reference electrode were immersed therein, and a voltage of +0.35 V was applied. After the current value became constant, glucose was added, and the current value in response thereto was monitored. A detector converted 1 μA of current value into 0.1 V of voltage and outputted the resulting value, and the change in outputted voltage values with time was graphed. The voltage increase from the background value to the steady state value after the addition of glucose was designated as the response value (V). FIG. 6 is a graph plotting the response value obtained at each glucose concentration by changing the final concentration of glucose to be added in the range of 5 to 40 mM. From these results, a glucose concentration-dependent increase in response value was observed, demonstrating that the GDH as disclosed herein is applicable to quantification of glucose and usable as a glucose sensor.

Experiment 11A

Preparation of cDNA

*Penicillium lilacinoechinulatum* strain NBRC6231 and *Penicillium italicum* strain NBRC32032 were cultured according to the method in Experiment 1 (except that the culture in the jar fermenter was performed for 24 hours), and the fungal threads were collected on filter paper using a Nutsche filter. The obtained fungal threads were immediately frozen in liquid nitrogen and were disrupted using Cool Mill supplied by Toyobo Co., Ltd. Total RNA was immediately extracted from the disrupted microbial cells using Sepasol RNA I supplied by Nacalai Tesque Inc. in accordance with the protocol of this kit. mRNA was purified from the resulting total RNA using Origotex-dt30 (supplied by Daiichi Pure Chemicals Co., Ltd.), and RT-PCR with the mRNA as the template was performed using ReverTra-Plus™ supplied by Toyobo Co., Ltd. The resulting product was electrophoresed on agarose gel and a portion corresponding to a chain length of 0.5 to 4.0 kb was cut out. cDNA was extracted from the cut out gel fragment using MagExtractor-PCR&Gel Clean Up supplied by Toyobo Co., Ltd. and purified to obtain a cDNA sample.

Experiment 12A

Determination of GDH Gene Sequence

Purified GDH derived from NBRC6231 was dissolved in Tris-HCl buffer (pH 6.8) containing 0.1% SDS and 10% glycerol, and partially digested by adding Glu specific V8 endoprotease to a final concentration of 10 μg/mL and incubating at 37°C for 16 hours. This sample was electrophoresed on 16% acrylamide gel to separate peptides. Peptide molecules present in the gel were transferred on a PVDF membrane using a blotting buffer (1.4% glycine, 0.3% Tris and 20% ethanol) using a semi-dry method. The peptides transferred on the PVDF membrane were stained using a CBB staining kit (GelCode Blue Stain Reagent supplied by PIERCE), two band portions of the visualized peptide fragments were cut out, and the internal amino acid sequences were analyzed using a peptide sequencer. The resulting amino acid sequences were IGGVVDTSLKVYGT (SEQ ID NO: 5) and WGGGTKQTVRAGKALGGTST (SEQ ID NO: 6). Based on these sequences, degenerate primers containing mixed bases were made, and PCR was performed using the cDNA derived from NBRC6231 made in Experiment 11A as the template. An amplified product was obtained, and was detected as a single band of about 1.4 kb by agarose gel electrophoresis. This band was cut out, and extracted and purified using MagExtractor-PCR&Gel Clean Up supplied by Toyobo Co., Ltd. The purified DNA fragment was TA-cloned using TArget Clone-Plus supplied by Toyobo Co., Ltd., and *Escherichia coli* JM 109 competent cells (Competent High JM109 supplied by Toyobo Co., Ltd.) were transformed with the resulting vector by heat shock. Among the transformed clones, from colonies in which an insert had been identified by blue-white determination, plasmids were extracted and purified by miniprep using MagExtractor-Plasmid supplied by Toyobo Co., Ltd., and the base sequence of the insert was determined using plasmid sequence specific primers. Based on the determined partial sequence of the GDH gene, 5'- and 3'-flanking regions of the partial sequence were determined by the RACE method. The sequence from the initiation codon to the termination codon in the determined gene region is shown in SEQ ID NO: 1, and the amino acid sequence deduced from this sequence is shown in SEQ ID NO: 2. Likewise, the sequence of the GDH gene derived from NBRC32032 was determined, and its base sequence is shown in SEQ ID NO: 3. The amino acid sequence deduced from this sequence is also shown in SEQ ID NO: 4. Based on the amino acid sequences deduced from these base sequences, a homology search was conducted on the "NCBI BLAST" website (http://www.ncbi.nlm.nih.gov/BLAST/). The GDH sequence derived from NBRC6231 had the highest homology of 55% to glucose oxidase derived from *Botryotinia fuckeliana*. The GDH sequence derived from NBRC32032 had the highest homology of 54% to an unnamed protein product (amino acid sequence deduced from ORF) derived from *Aspergillus oryzae.* The homology between GDH derived from NBRC6231 and GDH derived from NBRC32032 was 80%. These two types of GDH have a common characteristic of high heat stability, and it was found that the homology between the amino acid sequences of the two types of GDH is extremely high, and that there is no known amino acid sequence having a homology of more than 55% to the amino acid sequences of these two types of GDH. Therefore, one of the indicators that characterize the GDH as disclosed herein is the high homology to the amino acid sequence represented by SEQ ID NO: 2 or 4. It can be presumed that a protein having a homology of 80% or more, more preferably 85% or more, and still more preferably 90% or more is industrially advantageous.

Experiment 13A

Preparation of GDH Gene Recombinant Plasmid and Transformed Microorganism

Primers were made by adding an NdeI site to the 28 bases at the 5' end side and adding a BamHI site to the 28 bases at the 3' end side in the base sequence represented by SEQ ID NO: 3. Using these primers, PCR with cDNA derived from NBRC32032 as the template was performed. For the amino acid sequence represented by SEQ ID NO: 4, the signal sequence in the N terminal sequence was analyzed on a SignalP ver. 3.0 server, and it was concluded that the sequence up to Ala at position 15 or Ala at position 19 was the potential sequence for the secretory signal. Thus, N terminal restriction enzyme site-added primers were made so as to amplify the sequence in which 15 codons (45 bases) in the N terminal region had been deleted and the initiation codon (ATG) had been added, and the sequence in which 19 codons (57 bases) at the N terminal region had been deleted and the initiation codon (ATG) had been added, and PCR amplification product was obtained. The resulting PCR product was electrophoresed on agarose gel, and extracted and purified from the gel using MagExtractor-PCR&Gel Clean Up supplied by Toyobo Co., Ltd., and then treated with restriction enzymes, NdeI and BamHI. The treated PCR product was mixed with pBluescript KSN(+) subjected to the same restriction enzyme treatment. The resulting mixed solutions were ligated by adding Ligation High supplied by Toyobo Co., Ltd. in the same amount as the mixed solution and incubating at 16°C for 30 minutes. The ligated product was introduced by heat shock to *Escherichia coli* JM109 strain, which was then applied to LB agar medium containing 50 μg/mL of sodium ampicillin, and cultured at 37°C overnight to obtain transformed colonies. A plasmid was extracted by miniprep from the liquid culture product of a colony having an insert, and the base sequence of the insert was identified. In this way, three types of recombinant plasmids were made: pPIGDH1 (the full length from the initiation codon of SEQ ID NO: 3 was introduced), pPIGDH2 the sequence obtained by deleting 15 codons in the N terminal region and adding an initiation codon in SEQ ID NO: 3 had been introduced), and pPIGDH3 the sequence obtained by deleting 19 codons in the N terminal region and adding an initiation codon in SEQ ID NO: 3 had been introduced).

Experiment 14A

Culture of Transformed Microorganisms and Expression of GDH

Each of the recombinant plasmids obtained in Experiment 13A and a plasmid containing no insert as the control were introduced into *Escherichia coli* C600 strain by electroporation. Each of the resulting transformed strains was inoculated to 5 mL of LB medium (containing 50 μg/mL of sodium ampicillin) in a test tube, and cultured with shaking at 30°C for 16 hours. Subsequently, 50 μL of this culture medium was added to 5 mL of LB medium (containing 50 μg/mL of sodium ampicillin), and cultured with shaking at 30°C for 20 hours. The GDH activity in the culture medium obtained by culturing the transformant with the plasmid containing no insert was subtracted as a blank. The resultant GDH activity in the culture medium of each GDH gene-introduced strain was as in Table 3.

Table 3A

| Plasmid | pPIGDH1 | pPIGDH2 | pPIGDH3 |
|---|---|---|---|
| GDH activity (U/L) | 6.1 | 17.8 | 48.5 |

In this way, the expression of the GDH gene in *Escherichia coli* was identified. Furthermore, the *Escherichia coli* C600 strain transformed with pPIGDH3 was cultured with shaking at 25°C for 20 hours in a rich medium (2.4% yeast extract, 2.4% polypeptone, 1.25% dipotassium monohydrogen phosphate, 0.23% monopotassium dihydrogen phosphate, 0.4% glycerol, 50 μg/mL of sodium ampicillin, pH 7.0), and consequently the activity in the culture medium reached 1,000 U/L.

Experiment 15A

Purification of Expressed Recombinant GDH

The *Escherichia coli* C600 strain transformed with pPIGDH3 was inoculated to 60 mL of LB medium (containing 50 μg/mL of sodium ampicillin) in a 500 mL shaking flask, and cultured with shaking at 30°C for 16 hours. The culture was placed in a rich medium (2.4% yeast extract, 2.4% polypeptone, 1.25% dipotassium monohydrogen phosphate, 0.23% monopotassium dihydrogen phosphate, 0.4% glycerol, 50 μg/mL of sodium ampicillin, pH 7.0) in a 10 L jar fermenter, and cultured at 25°C with an aeration amount of 2 L/minute and a stirring rotation frequency of 330 rpm for 24 hours. The cultured microbial cells were collected by centrifugation, suspended in 50 mM phosphate buffer (pH 6.5) so that the microbial cell turbidity at 660 nm was about 50, and disrupted using a French press at a pressure of 65 MPa. The disrupted solution was centrifuged to obtain a supernatant, polyethyleneimine was added to the supernatant to a final concentration of 9% to precipitate nucleic acids and other substances, and the resulting mixture was centrifuged to obtain a supernatant. Ammonium sulfate in a saturated amount was dissolved in the supernatant to precipitate the target protein, and the precipitate collected by centrifugation was re-dissolved in 50 mM phosphate buffer (pH 6.5). Gel filtration on a G-25 Sepharose column and hydrophobic chromatography on an Octyl-Sepharose column and a Phenyl-Sepharose column (the peak fraction was extracted by elution with a concentration gradient of ammonium sulfate from 25% saturation to 0%) were carried out, and ammonium sulfate was removed by gel filtration on a G-25 Sepharose column to yield a recombinant GDH sample.

Experiment 16A

Application of Expressed Recombinant GDH to Glucose Electrode

Using the recombinant GDH obtained in Experiment 12A, a glucose electrode was made and the glucose concentration was quantified by following the procedure of Experiment 10A. The results are shown in FIG. 7. As shown in the graph, the glucose electrode made using the recombinant GDH also exhibited glucose concentration-dependent responses, and was confirmed to be applicable to glucose concentration quantification.

Experiments 1B-3B:

The outline of the procedure to acquire a GDH gene derived from *Aspergillus oryzae* described in the Experiments is as follows.

In order to acquire an *Aspergillus oryzae* GDH gene, purification of GDH from culture supernatants of *Aspergillus oryzae* and *Aspergillus terreus* was tried using salting out, chromatography and the like, but it was difficult to yield GDH with high purity (Experiment 1B [1])

Therefore, we had to abandon cloning using a partial amino acid sequence, which is one of the standard methods to acquire genes.

Thus, we searched for GDH-producing microorganisms other than the above microorganisms, and as a result of extensive study, we found that *Penicillium lilacinoechinulatum* NBRC6231 produced GDH, and successfully obtained a highly purified enzyme from a culture medium of this fungal strain (Experiment 1B [2]).

Subsequently, we successfully determined a partial amino acid sequence using the above enzyme, acquired a part of a GDH gene derived from *P. lilacinoechinulatum* NBRC6231 by PCR based on the determined amino acid sequence, and determined its base sequence (1356 bp) (Experiment 1B [3] and [4]).

Finally, based on this base sequence, a GDH gene derived from *Aspergillus* oryzae was deduced (Experiment 1B [5]) from the published database of the *Aspergillus* oryzae genome, and acquired.

Experiment 1B

Estimation of glucose dehydrogenase gene derived from *Aspergillus oryzae*

[1] Acquisition of GDH derived from *Aspergillus oryzae*

*Aspergillus* oryzae obtained from soils and stored as L-dried microbial cells according to a standard method was used. This is referred to as *Aspergillus* oryzae TI strain below.

The L-dried microbial cells of *Aspergillus* oryzae TI strain were inoculated to a potato dextrose agar medium (supplied by Difco) and incubated at 25°C to restore the cells. Fungal threads restored on the plate were collected together with the agar and then suspended in filter-sterilized water. In two 10 L jar fermenters, 6 L of a production medium (1% malt extract, 1.5% soybean peptide, 0.1% $MgSO_4 \cdot 7H_2O$, 2% glucose, pH 6.5) was prepared, sterilized in an autoclave at 120°C for 15 minutes, and allowed to cool. Thereafter, the above fungal thread suspension was inoculated and cultured with aeration and stirring at 30°C. The culture was stopped 64 hours after the start of the culture, and the fungal threads were removed by filtration to obtain a filtrate having GDH activity. Low molecular substances were removed from the collected supernatant using a ultrafiltration membrane (molecular weight cut-off of 10,000). Then, ammonium sulfate was added and dissolved at 60% saturation to perform ammonium sulfate fractionation. A supernatant having GDH activity was collected by centrifugation, adsorbed onto an Octyl-Sepharose column, and eluted with a gradient of ammonium from 60% saturation to 0% to collect fractions having GDH activity. The resulting GDH solution was applied to a G-25 Sepharose column to perform salting out. Ammonium sulfate at 60% saturation was added and dissolved, and the resulting solution was adsorbed onto a Phenyl-Sepharose column and eluted with a gradient of ammonium sulfate from 60% saturation to 0% to collect fractions having GDH activity. The fractions were heated at 50°C for 45 minutes, and then centrifuged to yield a supernatant. The solution obtained through the above steps was used as a purified GDH preparation (AOGDH). In the above purification process, a 20 mM potassium phosphate buffer (pH 6.5) was used as the buffer. In order to determine a partial amino acid sequence of the AOGDH, further purification was tried using various procedures such as ion exchange chromatography and gel filtration chromatography, but no purified preparation capable of being subjected to partial amino acid sequencing could be obtained.

Also, we independently searched and obtained a microorganism belonging to *Aspergillus terreus,* and tried purification from its culture supernatant in the same manner as above by salting out, adsorption onto Octyl-Sepharose, etc., but no purified preparation capable of being subjected to partial amino acid sequencing could be obtained as was the case with *Aspergillus oryzae.* It was presumed that one of the causes of not being able to acquire a GDH preparation having high purity and clearly detectable on SDS-PAGE by conventional purification methods is a sugar chain thought to be bound to the enzyme protein. Therefore, we had no choice but to abandon cloning utilizing a partial amino acid sequence of the protein, which is one of the standard methods to acquire the gene.

[2] Acquisition of GDH derived from filamentous fungus belonging to the genus *Penicillium*

Using *Penicillium lilacinoechinulatum* NBRC6231 as a GDH producing fungus derived from a filamentous fungus belonging to the genus *Penicillium*, culture and purification were performed in the same manner as for *Aspergillus* oryzae to obtain a purified preparation found to be nearly homogeneous by SDS electrophoresis.

[3] Preparation of cDNA

*Penicillium lilacinoechinulatum* NBRC6231 was cultured according to the above method (except that the culture in the jar fermenter was performed for 24 hours), and the fungal threads were collected by filtration on filter paper. The collected fungal threads were immediately frozen in liquid nitrogen, and disrupted using Cool Mill (supplied by Toyobo Co., Ltd.). The total RNA was immediately extracted from the disrupted microbial cells using Sepasol RNA I (supplied by Nacalai Tesque) according to the protocol of this kit. mRNA was purified from the resulting total RNA using Origotex-dt30 (supplied by Daiichi Pure Chemicals Co., Ltd.), and RT-PCR with the mRNA as the template was performed using ReverTra-Plus™ supplied by Toyobo Co., Ltd. The resulting product was electrophoresed on agarose gel and the portion corresponding to a chain length of 0.5 to 4.0 kb was cut out. cDNA was extracted and purified from the cutout gel fragment using MagExtractor-PCR&Gel Clean Up supplied by Toyobo Co., Ltd., and used as a cDNA sample.

[4] Determination of partial GDH gene sequence

The purified GDH derived from NBRC6231 was dissolved in a Tris-HCl buffer (pH 6.8) containing 0.1% SDS and 10% glycerol, and partially digested by adding Glu specific V8 endoprotease to a final concentration of 10 μg/mL thereto and incubating at 37°C for 16 hours. This sample was electrophoresed on 16% acrylamide gel to separate peptides. Peptide molecules present in this gel were transferred on a PVDF membrane using a blotting buffer (1.4% glycine, 0.3% Tris and 20% ethanol) using a semi-dry method. The peptides transferred onto the PVDF membrane were stained using a CBB staining kit (GelCode Blue Stain Reagent supplied by PIERCE), two band portions of the visualized peptide fragments were cut out and the internal amino acid sequences were analyzed using a peptide sequencer. The resulting amino acid sequences were IGGVVDTSLKVYGT (SEQ ID NO: 5) and WGGGTKQTVRAGKALGGTST (SEQ ID NO: 6). Based on these sequences, degenerate primers containing mixed bases were made, and PCR was performed using the cDNA derived from NBRC6231 as the template. An amplified product was obtained, and was detected as a single band of about 1.4 kb by agarose gel electrophoresis. This band was cut out, and extracted and purified using MagExtractor-PCR&Gel Clean Up (supplied by Toyobo Co., Ltd.). The purified DNA fragment was TA-cloned using TArget Clone-Plus supplied by Toyobo Co., Ltd., and *Escherichia coli* JM 109 competent cells (supplied by Toyobo Co., Ltd.) were transformed with the resulting vector by heat shock. Among the transformed clones, from colonies in which an insert had been identified by blue-white determination, plasmids were extracted and purified by miniprep using MagExtractor-Plasmid, and the base sequence (1356 bp) of the insert was determined using plasmid sequence specific primers.

[5] Estimation of AOGDH gene

Based on the determined base sequence, a homology search was conducted on the "NCBI BLAST" website (http://www.ncbi.nlm.nih.gov/BLAST/), and the AOGDH gene was estimated from multiple candidate sequences in consideration of the homology to publicly known glucose oxidation enzymes. The homology of the AOGDH estimated from the search to the partial sequence of GDH derived from *P. lilacinoechinulatum* NBRC6231 was 49% at an amino acid level.

Experiment 2B

Acquisition of AOGDH gene and introduction into *Escherichia coli*

To obtain the AOGDH gene, mRNA was prepared from microbial cells of *Aspergillus* oryzae TI strain, and cDNA was synthesized. Two types of oligo DNA represented by SEQ ID NOs: 12 and 13 were synthesized, and the AOGDH gene was amplified using the prepared cDNA as the template and using KOD Plus DNA polymerase (supplied by Toyobo Co., Ltd.). The resulting DNA fragment was treated with restriction enzymes NdeI and BamHI and inserted into Nde I-Bam HI sites in pBluescript (the NdeI site was introduced to match the NdeI recognition sequence ATG to the translation initiation codon ATG of LacZ) to construct a recombinant plasmid. Using the recombinant plasmid, *Escherichia coli* DH5α (supplied by Toyobo Co., Ltd.) was transformed. The plasmid was extracted from the transformant according to the standard method, and the base sequence of the AOGDH gene was determined (SEQ ID NO: 11). As a result, it was found that the amino acid sequence deduced from the cDNA sequence was composed of 593 amino acid residues (SEQ ID NO: 10). GDH predicted from the RIB40 strain registered in the database is composed of 588 amino acid residues (SEQ ID NO: 9), suggesting that the GDH from the RIB40 strain and the GDH from the TI strain are different in amino acid residue number. For the gene, the sequence was identified using TI strain genomic DNA, and the gene flanking regions were identified using the RACE method. Since it was suggested that the RIB40 strain in the database and the TI strain were different in GDH gene sequence, a recombinant plasmid containing the GDH gene sequence predicted from the sequence of the database RIB40 strain was made using the recombinant plasmid containing TI strain GDH gene as the template and using QuickChange Site Directed Mutagenesis Kit (supplied by Stratagene), and a transformant were acquired. These transformants were cultured with shaking at 30°C for 16 hours in a liquid medium (Terrific broth) containing 100 μg/mL of ampicillin. When the GDH activity was measured in disrupted microbial cell solutions, no GDH activity could be identified in the transformant with the GDH sequence derived from the RIB40 strain whereas the high GDH activity of 8.0 U per mL of the culture medium was obtained in the transformant with the GDH sequence derived from the TI strain. The GDH activity in the culture supernatant of *Aspergillus oryzae* TI strain in Experiment 1B was 0.2 U/mL. These results suggested that the GDH gene predicted from the RIB40 database sequence did not function as GDH. From a comparison between the gene sequences of the TI strain and RIB40 strain, this is presumably caused by the partial deletion in the GDH gene sequence derived from the RIB40 strain.

Experiment 3B

Cells of the *Escherichia coli* DH5α transformant with the GDH sequence derived from the TI strain cultured in Experiment 2B were collected by centrifugation, suspended in 20 mM potassium phosphate buffer (pH 6.5), and then disrupted using a French press to extract GDH. The extracted GDH was treated by the same procedure as for the AOGDH purified in Experiment 1B to yield a purified enzyme preparation (rAOGDH). The properties of the rAOGDH purified enzyme preparation were compared with those of the AOGDH purified enzyme preparation.

[1] Substrate specificity

When a blood glucose level is measured using a blood glucose sensor, use of a glucose-specific enzyme is required to avoid misdiagnosis. Thus, the reactivity of rAOGDH to various sugars was examined. The-results are shown in Table 1B. It was demonstrated that rAOGDH and AOGDH exhibited the equivalent substrate specificity and did not act upon maltose to which reactivity is particularly problematic when the sensor is used for a patient receiving a transfusion.

Table 1B

| | *Aspergillus oryzae* AOGDH | Recombinant rAOGDH |
|---|---|---|
| Glucose | 100.0 | 100.0 |
| Maltose | 0.4 | 0 |
| Fructose | 0 | 0.2 |
| Arabinose | 0 | 0.3 |
| Glycerin | 0.1 | 0.2 |
| Sucrose | 0.1 | -0 |
| Melezitose | 0 | 0.5 |
| Sorbose | 0 | 0 |
| Ribose | 0 | 0.1 |
| Maltotriose | 0.2 | 0.2 |
| Maltotetraose | 0.7 | 0.2 |
| Galactose | 0.4 | 0.6 |
| Mannose | 0.8 | 2.1 |
| Trehalose | 0.5 | 1.0 |

[2] Maltose degradability

Even if GDH itself used for the blood glucose sensor does not act upon maltose, the presence of a component that degrades maltose into glucose in the enzyme preparations or the like potentially leads to misdiagnosis. That is, it is extremely important that maltose-degrading components are not contained in the GDH enzyme preparations. Thus, the contamination of the GDH enzyme preparations with maltose-degrading components was examined. A test for such contamination with maltose-degrading components was performed as follows. 8 mM Maltose (50 μL) was added to 50 μL of each purified enzyme solution prepared at 10 U/mL, and was reacted at 37°C. After completion of the reaction, the concentration of glucose contained in the reaction solution was examined using Liquitec glucose HK test (supplied by Roche Diagnostics). A standard curve for calculating the glucose concentration was made by measuring glucose solutions for setting a standard coefficient (supplied by Wako Pure Chemical Industries Inc.). The results are shown in Table 2B. In the AOGDH purified preparation, after treating at 37°C for 30 seconds, 90% of maltose had already degraded into glucose, and after reacting for 10 minutes, nearly 100% maltose had degraded. Thus, the same measurement was performed using the preparation obtained by highly purifying AOGDH for the purpose of the analysis of the partial amino acid sequence in Experiment 1B, but the activity to degrade maltose was identified. In contrast, in the rAOGDH preparation, even after treating at 37°C for 10 minutes, no accumulation of glucose was observed at all. *Aspergillus oryzae* has been utilized in the fermentation industry for a long time, and has been known to produce sugar-related enzymes such as amylase and glucoamylase in large amounts. Under such circumstances, a high degree of purification of GDH alone is believed to be extremely difficult.

From these results, it is believed that the use of GDH prepared from a recombinant gene is essential when using GDH derived from *Aspergillus oryzae* in a blood glucose sensor.

Table 2B

| | Purified enzyme | Reaction time | | | |
|---|---|---|---|---|---|
| | | 30 sec | 60 sec | 3 min | 10 min |
| Free glucose concentration (mM) | AOGDH | 7.10 | 7.15 | 7.37 | 7.89 |
| | rAOGDH (recombinant) | 0 | 0.030 | 0.005 | 0 |
| Maltose degradation ratio | AOGDH | 89% | 89% | 92% | 99% |
| | rAOGDH (recombinant) | 0% | 0.38% | 0.06% | 0% |

Experiments 1C-2C:

The DNA (gene) composed of the base sequence described in SEQ ID NO: 8 comprises DNA (gene) encoding *Aspergillus oryzae* RIB40 strain-derived glucose dehydrogenase, which is predicted from the NCBI database and obtained by removing the intron from the genomic gene sequence including the intron. SEQ ID NO: 9 represents the amino acid sequence corresponding thereto.

The gene encoding the protein composed of the amino acid sequence described in SEQ ID NO: 9 indicates the full sequence of the glucose dehydrogenase gene predicted from the NCBI database.

The DNA (gene) composed of the base sequence described in SEQ ID NO: 11 indicates the full sequence of the DNA (gene) encoding the protein with glucose dehydrogenase activity derived from *Aspergillus* oryzae TI strain described later and identified by the present inventors. SEQ ID NO: 10 represents the amino acid sequence corresponding thereto.

DNA (gene) that hybridizes with DNA composed of the base sequence complementary to the base sequence described in SEQ ID NO: 11 under stringent conditions and encodes a protein having glucose dehydrogenase activity is included in the applicable scope of the present invention.

The gene encoding the protein composed of the amino acid sequence described in SEQ ID NO: 10 indicates the full sequence of the DNA (gene) encoding the protein having the glucose dehydrogenase activity derived from the *Aspergillus* oryzae TI strain described later. DNA (gene) encoding the protein composed of an amino acid sequence having one or more amino acid deletions, substitutions or additions (insertions) in the amino acid sequence described in SEQ ID NO: 10 and having glucose dehydrogenase activity is included in the applicable scope of the present invention.

The *Aspergillus oryzae* GDH gene was obtained according to Experiment 1B.

Experiment 1C

Introduction of Glucose Dehydrogenase Gene Derived from *Aspergillus oryzae* into *Escherichia coli*

For the AOGDH gene, mRNA was prepared from *Aspergillus* oryzae microbial cells, and cDNA was synthesized. Two types of oligo DNA shown in SEQ ID NOs: 12 and 13 were synthesized, and the AOGDH gene (wild type) was amplified using the prepared cDNA as the template and using KOD-Plus (supplied by Toyobo Co. Ltd.). The resulting DNA fragment was treated with restriction enzymes NdeI and BamHI, and inserted into NdeI-BamHI sites in pBluescript (the NdeI site had been introduced to match the NdeI recognition sequence ATG to the translation initiation codon ATG of LacZ) to construct a recombinant plasmid. This plasmid was introduced using Competent High DH5$\alpha$ (supplied by Toyobo Co., Ltd.). The plasmid was extracted according to the standard method, and the base sequence of the AOGDH gene was determined (SEQ ID NO: 11). The amino acid sequence deduced from the cDNA sequence was composed of 593 amino acid residues (SEQ ID NO: 10).

When FAD-GDH after cleaving the signal peptide was referred to as mFAD-GDH, the form in which only M had been added to the N-terminal of mFAD-GDH and thus the N-terminal of mFAD-GDH had been extended by one amino acid was expressed as S2. The form in which K at the N-terminal of mFAD-GDH had been substituted with M and thus the total number of the amino acid residues was the same as mFAD-GDH was expressed as S3. For S2, PCR was performed using the oligonucleotide of SEQ ID NO: 7 as the N-terminal primer, in combination with the primer of SEQ ID NO: 6, and by the same procedure, a recombinant plasmid having the DNA sequence encoding S2 was constructed and the transformant was likewise acquired. For S3, PCR was performed using the oligonucleotide of SEQ ID NO: 16 as the primer for the N terminal side, in combination with the primer of SEQ ID NO: 13, and by the same procedure, the recombinant plasmid having the DNA sequence encoding S2 was constructed and the transformant was likewise acquired. It was confirmed by DNA sequencing that the plasmid having the DNA sequence for each modified FAD-GDH had no error in its sequence.

SEQ ID NO: 17 represents the DNA sequence encoding the signal peptide-deleted mutant S2 determined above. SEQ ID NO: 18 represents the amino acid sequence corresponding thereto.

These transformants were cultured in liquid TB medium using 10 L jar fermenters for 1 to 2 days. The microbial cells at each culture phase were collected, and disrupted with ultrasonic waves to identify GDH activity. The relationship of the culture phase of each transformant with OD, pH and GDH activity are shown in Tables 1C, 2C and 3C and FIGs. 9, 10 and 11. *Aspergillus oryzae* wild type strain was cultured in a liquid medium (1% malt extract, 1.5% soybean peptide, 0.1% $MaSO_4 \cdot 7H_2O$, 2% glucose, 0.05 mM p-benzoquinone, 0.1 mM EDTA, pH 6.5) using a 10 L jar fermenter at 30°C for one day, and the GDH activity in or out of the microbial cells was measured. As a result, the GDH activity was about 0.2 U/mL of the medium (mL, broth) in all cases.

In this specification, the expressed amounts are compared in terms of GDH activity value per 1 mL of the medium.

In the wild type FAD-GDH (WT), the peak (6.6 U/mL-b (enzyme unit (U) per 1 mL of medium)) was observed at 16 to 18 hours of the culture, and then activity decreased. In the modified FAD-GDH S2, the peak (72 to 73 U/mL-b) was observed at 22 to 25 hours of the culture; in S3, the peak (74 to 75 U/mL-b) was observed at 20 to 23 hours of the culture, and GDH activity thereafter decreased as was the case with WT.

Comparing culture titers in the respective peaks, it was revealed that the GDH productivity was increased by 10 times or more by deleting the amino acid sequence predicted to be the signal peptide.

Comparing the specific activity (U/mg) of FAD-GDH purified preparations before and after deleting the signal peptide, the specific activity was 270 U/mg in the wild type FAD-GDH (WT) whereas it was 670 U/mg in the modified FAD-GDH S2 after the deletion. Thus, the specific activity was increased by 2.5 times, suggesting that the specific activity is also increased by deleting the signal peptide.

Even considering the increase in specific activity, the activity is believed to be increased by at least 4.4 times.

Furthermore, as a result of extensive study, we discovered that FAD-GDH derived from the filamentous fungus had reduced stability in a pH range of pH 7.1 or higher. Further, the present study revealed that it was important to control the pH at 7.1 to 7.3 or lower, and preferably 7.1 or lower in culture for production of the recombinant FAD-GDH derived from the filamentous fungus. It was confirmed that the peak of GDH activity can be kept by controlling the culture at the above pH value or lower.

In the method for producing recombinant glucose dehydrogenase derived from the filamentous fungi, when a mutation is introduced into the signal peptide sequence present in the N-terminal region, the controlled pH of the medium can be raised to 7.3.

Experiment 2C

Introduction of Glucose Dehydrogenase Gene Derived from *Aspergillus* terreus (hereinafter abbreviated as ATGDH) into *Escherichia coli*

For the ATGDH gene, mRNA was prepared from microbial cells of *Aspergillus* terreus (deposited under accession number NBRC33026 at the Biological Resource Center, the National Institute of Technology and Evaluation), and cDNA was synthesized. Two types of oligo DNA shown in SEQ ID NOs: 21 and 22 were synthesized, and the ATGDH gene (gene sequence in which the predicted signal peptide sequence had been deleted) was amplified using the prepared cDNA as the template and using KOD-Plus (supplied by Toyobo Co., Ltd.). The resulting DNA fragment was treated with the restriction enzymes NdeI and BamHI, and inserted into NdeI-BamHI sites in pBluescript (the NdeI site had been introduced to match the NdeI recognition sequence ATG to the translation initiation codon ATG of LacZ) to construct a recombinant plasmid. This plasmid was introduced using Competent High DH5$\alpha$ (supplied by Toyobo Co., Ltd.). The plasmid was extracted according to the standard method, and the base sequence of the ATGDH gene was determined (SEQ ID NO: 19). The amino acid sequence deduced from the DNA sequence was composed of 568 amino acids (SEQ ID NO: 20).

Each of these transformants were cultured in 50 mL of LB medium containing 100 $\mu$g/mL of ampicillin at 30°C overnight, and again cultured in 50 mL of LB medium containing 100 $\mu$g/mL of ampicillin at 30°C for 8 hours to prepare an inoculum.

The prepared inoculum was cultured in liquid TB medium containing 100 $\mu$g/mL of ampicillin in the range of Kd·P 0.5 to 1.5 in a 10 L jar fermenter for 6 days. The microbial cells at each culture phase were collected, and disrupted with ultrasonic waves to identify GDH activity. The relationships of the culture phase with OD, pH and GDH activity are shown in Tables 4C, 5C, 6C and 7C and FIGs. 12, 13, 14 and 15. *Aspergillus terreus* wild type strain was cultured in a liquid medium (1% malt extract, 1.5% soybean peptide, 0.1% $MaSO_4 \cdot 7H_2O$, 2% glucose, 0.05 mM p-benzoquinone, 0.1 mM EDTA, pH 6.5) using a 10 L jar fermenter at 30°C for one day, and GDH activity in the microbial cells was measured. As a result, about 0.1 U/mL of medium was the peak.

In the recombinant FAD-GDH, activity of about 9 to 21 U/mL-b was observed in the peaks, and the culture titers were increased by about 100 to 200 times. Examining the culture conditions, it was found that when Kd·P was set to a low value of 0.5, the culture titer was increased by about 1.5 times more than in the case of a Kd·P of 0.75, and by about 2 times than in the case of a Kd·P of 1 to 1.5. It was confirmed that when Kd·P was set to 2 or more, the culture titer was about 5 U/mL-b.

With respect to the culture temperature, the relationships of the culture phase with OD, pH and GDH activity are shown in Tables 8C, 9C, 10C and 11C and FIGs. 16, 17, 18 and 19. A culture temperature of 26 to 28°C is optimal, and it is necessary to culture at least at 23 to 28°C. Culturing at 29°C or higher tends to result in the reduction of the culture titer by half.

Also in the culture of the recombinant ATGDH gene, it is extremely important to keep the pH of the medium at 7.3 or lower in order to keep the enzyme activity stable. In ATGDH, it appeared to be necessary to terminate the culture at a lower pH than in AOGDH.

In the ATGDH gene, the DNA sequence of the predicted signal peptide (MLGKLSFLSALSLAVAATLSNSTSA) (SEQ ID NO: 23) sequence was deleted from the DNA sequence encoding FAD-GDH derived from *Aspergillus* terreus. In the transformant containing the signal peptide, the expression amount of FAD-GDH was poor as was the case with FAD-GDH derived from A. oryzae. Thus, the culture was initially studied using a transformant containing no signal peptide.

Table 1C

| Sampling time (hr) | Wild type OD | pH | Act (U/ml) |
|---|---|---|---|
| 16 | 11.9 | 6.8 | 6.5 |
| 18 | 14.2 | 7.1 | 6.6 |
| 20 | 15.2 | 7.3 | 4.9 |
| 22 | 15.7 | 7.5 | 1.7 |
| 23 | 16.2 | 7.6 | 1.1 |
| 24 | 16.8 | 7.7 | 0.9 |
| 25 | 16.8 | 7.7 | 0.6 |
| 26 | 16.7 | 7.8 | 0.4 |
| 28 | 17.8 | 8.0 | 0.3 |
| 30 | 18.1 | 8.1 | 0.2 |

Table 2C

| Sampling time (hr) | S2 OD | pH | Act (U/ml) |
|---|---|---|---|
| 16 | 5.8 | 6.7 | 14.1 |
| 18 | 9.3 | 6.6 | 47.0 |
| 20 | 11.4 | 6.7 | 63.5 |
| 22 | 14.0 | 7.0 | 72.2 |
| 23 | 14.5 | 7.1 | 72.8 |
| 24 | 15.1 | 7.2 | 73.3 |
| 25 | 15.8 | 7.3 | 71.6 |
| 26 | 16.5 | 7.5 | 49.9 |
| 28 | 17.3 | 7.7 | 36.2 |
| 30 | 17.5 | 7.9 | 24.4 |

Table 3C

| Sampling time (hr) | S3 OD | pH | Act (U/ml) |
|---|---|---|---|
| 16 | 9.8 | 6.5 | 53.4 |
| 18 | 11.8 | 6.8 | 70.0 |
| 20 | 14.4 | 7.0 | 74.6 |

(continued)

| Sampling time (hr) | S3 OD | pH | Act (U/ml) |
|---|---|---|---|
| 22 | 15.3 | 7.2 | 74.6 |
| 23 | 15.6 | 7.3 | 74.0 |
| 24 | 15.9 | 7.5 | 63.5 |
| 25 | 16.0 | 7.6 | 46.7 |
| 26 | 16.2 | 7.7 | 29.8 |
| 28 | 17.6 | 7.9 | 24.5 |
| 30 | 16.9 | 8.3 | 1.4 |

Table 4C

| Time | FAD-GLD U/ml | OD660 | U/OD | pH |
|---|---|---|---|---|
| 16 | 0.8 | 3.0 | 0.28 | 6.76 |
| 20 | 1.9 | 4.7 | 0.41 | 6.5 |
| 24 | 3.0 | 7.1 | 0.42 | 6.51 |
| 28 | 6.7 | 9.8 | 0.68 | 6.45 |
| 32 | 9.2 | 12.4 | 0.74 | 6.39 |
| 36 | 11.5 | 13.2 | 0.87 | 6.3 |
| 40 | 15.5 | 14.0 | 1.11 | 6.4 |
| 44 | 13.8 | 16.0 | 0.86 | 6.48 |
| 48 | 14.7 | 17.5 | 0.84 | 6.56 |
| 52 | 15.7 | 17.1 | 0.92 | 6.63 |
| 56 | 17.4 | 17.8 | 0.98 | 6.73 |
| 60 | 17.4 | 19.4 | 0.90 | 6.8 |
| 64 | 16.3 | 20.7 | 0.79 | 6.84 |
| 68 | 13.6 | 19.6 | 0.69 | 6.93 |
| 72 | 14.6 | 21.5 | 0.68 | 6.94 |
| 76 | 15.2 | 21.9 | 0.70 | 7.02 |
| 80 | 13.9 | 23.2 | 0.60 | 7.09 |
| 84 | 13.4 | 24.6 | 0.55 | 7.19 |
| 88 | 10.2 | 20.2 | 0.51 | 7.23 |

Table 5C

| Time | FAD-GLD U/ml | OD660 | U/OD | pH |
|---|---|---|---|---|
| 16 | 0.4 | 2.2 | 0.19 | 6.8 |
| 20 | 2.0 | 6.1 | 0.34 | 6.51 |
| 24 | 3.2 | 9.1 | 0.35 | 6.47 |
| 28 | 4.7 | 13.7 | 0.35 | 6.37 |

(continued)

| Time | FAD-GLD U/ml | OD660 | U/OD | pH |
|---|---|---|---|---|
| 32 | 7.3 | 15.7 | 0.46 | 6.34 |
| 36 | 12.4 | 17.1 | 0.72 | 6.46 |
| 40 | 11.3 | 18.1 | 0.63 | 6.63 |
| 44 | 11.6 | 20.8 | 0.56 | 6.76 |
| 48 | 11.7 | 20.7 | 0.56 | 6.87 |
| 52 | 10.2 | 20.1 | 0.51 | 6.96 |
| 56 | 9.8 | 21.0 | 0.47 | 7.11 |
| 60 | 8.9 | 21.9 | 0.41 | 7.23 |
| 64 | 7.8 | 23.6 | 0.33 | 7.34 |
| 68 | 6.6 | 24.5 | 0.27 | 7.47 |
| 72 | 5.5 | 24.5 | 0.22 | 7.51 |
| 76 | 4.3 | 26.0 | 0.16 | 7.63 |
| 80 | 3.1 | 27.1 | 0.11 | 7.71 |
| 84 | 2.2 | 27.1 | 0.08 | 7.86 |
| 88 | 1.2 | 23.9 | 0.05 | 7.97 |

Table 6C

| Time | FAD-GLD U/ml | OD660 | U/OD | pH |
|---|---|---|---|---|
| 16 | 0.2 | 2.1 | 0.09 | 6.83 |
| 20 | 2.3 | 7.0 | 0.33 | 6.51 |
| 24 | 3.7 | 11.0 | 0.33 | 6.49 |
| 28 | 9.9 | 15.8 | 0.63 | 6.27 |
| 32 | 10.0 | 18.4 | 0.54 | 6.44 |
| 36 | 9.8 | 20.9 | 0.47 | 6.74 |
| 40 | 8.1 | 21.2 | 0.38 | 6.92 |
| 44 | 6.5 | 21.6 | 0.30 | 7.1 |
| 48 | 5.8 | 21.9 | 0.26 | 7.32 |
| 52 | 4.4 | 20.5 | 0.22 | 7.46 |
| 56 | 2.9 | 20.4 | 0.14 | 7.62 |
| 60 | 1.6 | 22.3 | 0.07 | 7.75 |
| 64 | 0.7 | 23.6 | 0.03 | 7.86 |
| 68 | 0.4 | 24.6 | 0.02 | 8.18 |

Table 7C

| Time | FAD-GLD U/ml | OD660 | U/OD | pH |
|---|---|---|---|---|
| 16 | 0.2 | 2.1 | 0.10 | 6.83 |
| 20 | 3.2 | 8.4 | 0.38 | 6.54 |
| 24 | 4.5 | 14.5 | 0.31 | 6.44 |
| 28 | 9.4 | 17.7 | 0.53 | 6.66 |
| 32 | 8.3 | 19.3 | 0.43 | 6.94 |
| 36 | 4.5 | 20.5 | 0.22 | 7.3 |
| 40 | 3.0 | 19.3 | 0.16 | 7.6 |
| 44 | 1.8 | 19.2 | 0.09 | 7.74 |
| 48 | 1.0 | 19.4 | 0.05 | 7.8 |
| 52 | 0.4 | 18.6 | 0.02 | 8.01 |
| 56 | 0.1 | 19.1 | 0.01 | 8.09 |
| 60 | 0.0 | 20.2 | 0.00 | 8.11 |
| 64 | 0.0 | 19.6 | 0.00 | 8.22 |
| 68 | 0.0 | 18.9 | 0.00 | 8.42 |

Table 8C

| Time | FAD-GLD U/ml | OD660 | U/OD | pH |
|---|---|---|---|---|
| 18 | 0.0 | 0.1 | 0.01 | 6.81 |
| 24 | 0.0 | 0.2 | 0.02 | 6.81 |
| 28.5 | 0.0 | 0.4 | 0.02 | 6.8 |
| 39 | 0.0 | 1.4 | 0.01 | 6.78 |
| 46 | 0.2 | 3.2 | 0.07 | 6.68 |
| 50 | 1.4 | 5.8 | 0.24 | 6.53 |
| 56 | 2.7 | 8.2 | 0.33 | 6.45 |
| 62 | 3.4 | 10.5 | 0.32 | 6.28 |
| 70 | 8.9 | 11.5 | 0.77 | 6.24 |
| 76 | 8.0 | 15.7 | 0.51 | 6.44 |
| 82 | 9.1 | 18.2 | 0.50 | 6.57 |
| 88 | 8.4 | 18.8 | 0.45 | 6.75 |
| 94 | 10.2 | 20.6 | 0.49 | 6.89 |
| 100 | 9.0 | 21.2 | 0.43 | 7.05 |
| 106 | 12.9 | 19.7 | 0.66 | 7.14 |
| 112 | 14.0 | 20.7 | 0.68 | 7.31 |
| 130 | 8.0 | 19.5 | 0.41 | 7.69 |
| 136 | 6.0 | 18.2 | 0.33 | 7.76 |

Table 9C

| Time | FAD-GLD U/ml | OD660 | U/OD | pH |
|---|---|---|---|---|
| 18 | 0.0 | 0.4 | 0.01 | 6.81 |
| 24 | 0.0 | 1.3 | 0.01 | 6.79 |
| 28.5 | 0.7 | 2.9 | 0.24 | 6.7 |
| 39 | 4.4 | 7.1 | 0.61 | 6.37 |
| 46 | 7.1 | 9.3 | 0.76 | 6.22 |
| 50 | 8.9 | 11.2 | 0.80 | 6.19 |
| 56 | 10.0 | 12.7 | 0.79 | 6.36 |
| 62 | 10.0 | 13.7 | 0.73 | 6.53 |
| 70 | 12.7 | 13.7 | 0.93 | 6.72 |
| 76 | 13.1 | 14.4 | 0.91 | 6.85 |
| 82 | 14.4 | 16.1 | 0.89 | 6.97 |
| 88 | 14.2 | 17.1 | 0.83 | 7.1 |
| 94 | 14.3 | 17.4 | 0.82 | 7.25 |
| 100 | 13.1 | 17.6 | 0.74 | 7.4 |
| 106 | 10.8 | 17.5 | 0.62 | 7.45 |
| 112 | 8.8 | 19.1 | 0.46 | 7.57 |
| 130 | 5.2 | 17.4 | 0.30 | 7.96 |
| 136 | 2.2 | 17.4 | 0.13 | 8.03 |

Table 10C

| Time | FAD-GLD U/ml | OD660 | U/OD | pH |
|---|---|---|---|---|
| 18 | 0.8 | 2.3 | 0.37 | 6.69 |
| 24 | 0.6 | 4.9 | 0.12 | 6.43 |
| 28.5 | 4.5 | 6.2 | 0.72 | 6.35 |
| 39 | 6.3 | 8.7 | 0.72 | 6.18 |
| 46 | 9.5 | 10.6 | 0.89 | 6.28 |
| 50 | 11.0 | 11.8 | 0.93 | 6.41 |
| 56 | 6.7 | 15.4 | 0.44 | 6.52 |
| 62 | 11.5 | 12.8 | 0.90 | 6.63 |
| 70 | 16.9 | 12.3 | 1.37 | 6.77 |
| 76 | 19.4 | 13.3 | 1.46 | 6.89 |
| 82 | 21.3 | 15.6 | 1.37 | 7.02 |
| 88 | 19.8 | 15.6 | 1.27 | 7.1 |
| 94 | 19.8 | 16.2 | 1.22 | 7.22 |
| 100 | 17.6 | 17.4 | 1.01 | 7.28 |

(continued)

| Time | FAD-GLD U/ml | OD660 | U/OD | pH |
|---|---|---|---|---|
| 106 | 16.8 | 16.3 | 1.03 | 7.28 |
| 112 | 13.8 | 17.1 | 0.81 | 7.34 |
| 130 | 4.1 | 17.2 | 0.24 | 7.77 |
| 136 | 1.3 | 17.2 | 0.08 | 7.94 |

Table 11C

| Time | FAD-GLD U/ml | OD660 | U/OD | pH |
|---|---|---|---|---|
| 18 | 2.4 | 5.6 | 0.43 | 6.31 |
| 24 | 5.0 | 7.0 | 0.71 | 6.28 |
| 28.5 | 7.9 | 7.9 | 1.01 | 6.21 |
| 39 | 7.4 | 10.0 | 0.74 | 6.42 |
| 46 | 7.8 | 12.1 | 0.64 | 6.55 |
| 50 | 8.0 | 13.1 | 0.61 | 6.66 |
| 56 | 9.2 | 14.8 | 0.62 | 6.75 |
| 62 | 6.7 | 14.7 | 0.45 | 6.85 |
| 70 | 9.6 | 14.8 | 0.65 | 6.98 |
| 76 | 9.0 | 16.1 | 0.56 | 7.12 |
| 82 | 8.0 | 18.3 | 0.44 | 7.17 |
| 88 | 7.6 | 21.1 | 0.36 | 7.27 |
| 94 | 6.3 | 21.8 | 0.29 | 7.38 |
| 100 | 3.7 | 20.0 | 0.18 | 7.58 |
| 106 | 2.3 | 22.1 | 0.10 | 7.78 |
| 112 | 0.4 | 21.1 | 0.02 | 7.93 |
| 130 | 0.1 | 22.5 | 0.00 | 8.18 |
| 136 | 0.0 | 22.8 | 0.00 | 8.2 |

Tables 1C to 3C show the relationships of the culture phase of each mutant in the 10 L jar fermenter culture with the microbial cell turbidity (OD), pH and GDH activity of the medium. Tables 1C, 2C and 3C correspond to FIGs. 9, 10 and 11, respectively.

Tables 4C to 7C show the relationships of the culture phase at Kd·P 0.5, 0.75, 1 and 1.5 in the 10 L jar fermenter culture with the microbial cell turbidity (OD), pH and GDH activity in the medium. Tables 4C, 5C, 6C and 7C correspond to FIGs. 12, 13, 14 and 15, respectively.

Tables 8C to 11C show the relationships of the culture phase at culture temperatures 20, 23, 26 and 30°C in the 10 L jar fermenter culture with the microbial cell turbidity (OD), pH and GDH activity of the medium. Tables 8C, 9C, 10C and 11C correspond to FIGs. 16, 17, 18 and 19, respectively.

Experiments 1D:9D

Test Experiments 1D: Method for Measuring FAD-Dependent GDH Activity

The activity of FAD-dependent GDH is measured as follows.

Reagents:

50 mM PIPES buffer pH 6.5 (containing 0.1% Triton X-100)
163 mM PMS solution
6.8 mM 2,6-dichlorophenol-indophenol (DCPIP) solution
1 M D-glucose solution

The reaction reagent is made by mixing 15.6 mL of the PIPES buffer, 0.2 mL of the DCPIP solution and 4 mL of the D-glucose solution.

Measurement conditions:

The reaction reagent (3 mL) is preliminarily heated at 37°C for 5 minutes. The GDH solution (0.1 mL) is added and gently mixed, subsequently the change of absorbance at 600 nm is recorded for 5 minutes using a spectrophotometer maintained at 37°C using water as the control, and the change of absorbance per one minute ($\Delta OD_{TEST}$) is calculated from the linear portion of the record. As a blank test, a solvent that dissolves GDH is added in place of the GDH solution to the reagent mixture, and the change of absorbance ($\Delta OD_{BLANK}$) per one minute is measured. The GDH activity is calculated from these values according to the following formula. One unit (U) of GDH activity is defined as the amount of enzyme that reduces 1 μmol of DCPIP in one minute in the presence of 200 mM D-glucose.

$$\text{Activity (U/mL)} = [-(\Delta OD_{TEST} - \Delta OD_{BLANK}) \times 3.0 \times \text{dilution scale}]/(16.3 \times 0.1 \times 1.0)$$

In the above formula, 3.0 is the liquid amount (mL) of the reaction reagent + the enzyme solution, 16.3 is the millimolar molecular absorbance coefficient ($cm^2/\mu mol$) in the conditions for measuring the activity, 0.1 is the liquid amount of the enzyme solution (mL), and 1.0 is the light path length (cm) of the cell.

Experiment 1D: Preparation of Recombinant Glucose Dehydrogenase Preparation Derived from Filamentous Fungus

mRNA was prepared from microbial cells of *Aspergillus oryzae* TI strain (obtained from soils and stored as L-dried microbial cells according to the standard method; hereinafter referred to as *Aspergillus oryzae* TI strain) and *Aspergillus terreus* NBRC33026 strain, and cDNA was synthesized. Four types of oligo DNA shown in SEQ ID NOs: 12 and 13 and SEQ ID NOs: 21 and 22 were synthesized. Using each cDNA prepared from each mRNA as the template, the GDH (AOGDH) genes of *Aspergillus oryzae* and *Aspergillus terreus* were amplified using KOD-Plus (supplied by Toyobo Co., Ltd). The resulting DNA fragments were treated with restriction enzymes NdeI and BamHI, and inserted into NdeI-BamHI sites in pBluescript (the NdeI site had been introduced to match the NdeI recognition sequence ATG to the translation initiation codon ATG of LacZ) to construct two recombinant plasmids (pAOGDH, pATGDH). These recombinant plasmids were introduced using Competent High DH5α (supplied by Toyobo Co., Ltd.). The plasmids were extracted according to the standard method, and the base sequences of the AOGDH gene and the ATGDH gene were determined (SEQ ID NOs: 11 and 19). The amino acid sequence deduced from the cDNA sequence was composed of 593 amino acids (SEQ ID NO: 10) in *Aspergillus* oryzae and 568 amino acids (SEQ ID NO: 20) in *Aspergillus* terreus. By the same techniques, a transformant was obtained by introducing a recombinant plasmid (pPIGDH) containing the GDH gene derived from *Penicillium italicum* (PIGDH).

These transformants were cultured in TB medium (2.4% yeast extract, 1.2% polypeptone, 1.25% dipotassium monohydrogen phosphate, 0.23% monopotassium dihydrogen phosphate, 0.4% glycerol, 50 μg/mL of sodium ampicillin, pH 7.0) using a 10 L jar fermenter at a culture temperature of 25°C, an aeration amount of 2 L/minute and a stirring rotation speed of 170 rpm for 48 hours.

The cultured microbial cells were collected by centrifugation, suspended in 50 mM phosphate buffer (pH 5.5) so that the microbial cell turbidity at 660 nm was about 50, and disrupted with a homogenizer at a pressure of 65 MPa. The disrupted solution was centrifuged to obtain a supernatant, polyethyleneimine was added to the supernatant to a final concentration of 9% to precipitate nucleic acids and other substances, and the resulting mixture was centrifuged to obtain a supernatant. Ammonium sulfate in a saturated amount was dissolved in the supernatant to precipitate the target protein, and the precipitate collected by centrifugation was re-dissolved in 50 mM phosphate buffer (pH 5.5). Gel filtration on a G-25 Sepharose column and hydrophobic chromatography on an Octyl-Sepharose column and a Phenyl-Sepharose column (the peak fraction was extracted by elution with a concentration gradient of ammonium sulfate from 25% saturation to 0%) were carried out, and further ammonium sulfate was removed by gel filtration on a G-25 Sepharose column to prepare a recombinant GDH preparation.

Experiment 2D: Preparation of FAD-GDH Preparation Derived from Wild Type Filamentous Fungus

Using *Aspergillus* terreus NBRC33026 strain and *Aspergillus oryzae* TI strain as FAD-dependent GDH-producing fungi derived from wild type filamentous fungi, an L-dried sample of each fungal strain was inoculated on a potato dextrose agar medium (supplied by Difco) and incubated at 25°C to restore. Fungal threads restored on the plate were collected together with the agar, and then suspended in filter-sterilized water. In two 10 L jar fermenters, 6 L of a production medium (1% malt extract, 1.5% soybean peptide, 0.1% $MgSO_4 \cdot 7H_2O$, 2% glucose, pH 6.5) was prepared and sterilized in an autoclave at 120°C for 15 minutes. Then, the above fungal thread suspension was added thereto, and culture was started. The culture was performed under the conditions of a temperature of 30°C, an aeration amount of 2 L/minute and a stirring frequency of 380 rpm. The culture was stopped 64 hours after the start of the culture, and microbial cells from each fungal strain were collected on filter paper by suction filtration using a Nutsche filter. The culture medium (5 L) was concentrated to 1/10 amount using a hollow fiber module for ultrafiltration with a molecular weight cut off of 10,000, and ammonium sulfate was added to and dissolved in each concentrated solution to a final concentration of 60% saturation (456 g/L). Subsequently, the mixture was centrifuged at 8000 rpm for 15 minutes using a high speed cooling centrifuge supplied by Hitachi Ltd. to precipitate the cell debris. Then, the supernatant was adsorbed onto an Octyl-Sepharose column, and fractions with GDH activity were collected by elution with a concentration gradient of ammonium sulfate from 0.6 to 0.0 saturation. Salting out was performed by applying the resulting GDH solution onto a G-25 Sepharose column for gel filtration and collecting protein fractions. Ammonium sulfate corresponding to 0.6 saturation was added to the solution after salting out. This mixture was adsorbed onto a Phenyl Sepharose column, and fractions with GDH activity were collected by elution with a concentration gradient of ammonium sulfate from 0.6 to 0.0 saturation. The resulting GDH solution was subjected to gel filtration on a G25 Sepharose column to collect the protein fraction. The acquired purified enzyme was used as a preparation for evaluating FAD-dependent GDH.

The mediator used for the glucose level measurement composition, glucose assay kit, glucose sensor or glucose level measurement method as disclosed herein is not particularly limited. It is preferable to use 2,6-dichlorophenol-indophenol (abbreviated as DCPIP), ferrocene or a derivative thereof (e.g., potassium ferricyanide, phenazine methosulfate or the like). Such mediators are commercially available.

Experiment 3D: Study of FAD-GDH Thermal Stabilization Effect of Various Stabilizing Agents using Glucose Measurement System

The study was performed in accordance with the method for measuring the FAD-GDH activity in Test Experiment 1 described above.

First, 50 mL of an enzyme solution in which the *Aspergillus oryzae*-derived recombinant FAD-GDH preparation (rAO-FAD-GDH) obtained in Experiment 1 had been dissolved at 2 U/mL in an enzyme dilution solution (50 mM potassium phosphate buffer (pH 5.5), 0.1% Triton X-100) was prepared. Two tubes were prepared in which each of the stabilizing agents shown in Table 1D had been added at each final concentration to 0.9 mL of the above enzyme solution to make a total volume of 1.0 mL were prepared. As the control, two tubes in which 0.1 mL of distilled water had been added in place of each compound were prepared.

Of the two tubes, one was stored at 4°C and the other was treated at 50°C for 15 minutes. Then, the FAD-GDH activity in each tube was measured. The enzyme activity in the tube stored at 4°C was made 100, and, comparing with this, the activity value after treatment at 50°C for 15 minutes was calculated as the residual activity ratio (%).

These experiments revealed that the thermal stability of FAD-GDH was increased by adding sugars and certain types of amino acids, which were not the substrates of FAD-GDH (Table 1D).

A higher thermal stabilization effect was observed with the sugars than with the amino acids. Among them, a high effect was observed with trehalose, mannose, sodium gluconate, galactose, methyl-α-D-glucoside and α-D-melibiose.

Table 1D

| | Final concentration | Stabilizing agent | Residual activity ratio (%) after treatment at 50°C for 15 min |
|---|---|---|---|
| | 1% | Control (no stabilizing agent) | 19.3 |
| Sugars | 1% | D-(-)-arabinose | 23.0 |
| | 1% | 1,4-sorbitane | 26.7 |
| | 1% | 2-deoxy-D-glucose | 69.6 |
| | 1% | D-(+)-xylose | 68.5 |
| | 1% | D-(+)-trehalose dihydrate | 64.7 |
| | 1% | D-(+)-mannose | 65.3 |
| | 1% | D-(+)-melezitose | 44.7 |
| | 1% | D-(-)-fructose | 25.9 |
| | 1% | Sodium gluconate | 33.5 |
| | 1% | D-sodium glucuronate | 56.2 |
| | 1% | D-$\alpha$-galacturonic acid | 0.0 |
| | 1% | Inulin | 24.7 |
| | 1% | Galactose | 60.6 |
| | 1% | Glucono-1,5-lactone | 0.0 |
| | 1% | Methyl-$\alpha$-D-glucoside | 53.6 |
| | 1% | $\alpha$-cyclodextrin | 44.1 |
| | 1% | $\alpha$-D-(+)-melibiose | 59.0 |
| | 1% | Sucrose | 44.1 |
| Amino acids | 1% | Sodium L-aspartate | 23.0 |
| | 1% | L-aspartic acid | 12.2 |
| | 0.20% | L-asparagine | 22.8 |
| | 1% | Glycine | 26.4 |
| | 0.10% | D-glutamic acid | 23.2 |
| | 0.10% | D-phenylalanine | 23.6 |
| | 1% | D-proline | 24.8 |
| | 1% | D-$\alpha$-alanine | 27.1 |
| | 0.25% | DL-isoleucine | 23.4 |
| | 0.25% | L-glutamine | 22.6 |
| | 1% | L-(-)-proline | 25.2 |
| | 1% | L-arginine | 1.1 |
| | 1% | L-serine | 27.0 |
| | 0.10% | L-triptophan | 24.3 |
| | 0.50% | L-valine | 25.5 |
| | 0.25% | L-histidine | 11.4 |
| | 0.20% | L-phenylalanine | 22.3 |
| | 1% | L-lysine hydrochloride | 23.4 |
| | 0.10% | L-leucine | 22.2 |
| | 1% | Sarcosine | 25.2 |
| | 1% | Taurine | 23.8 |

Experiment 4D: Study on Effective Concentration of Trehalose for FAD-GDH Thermal Stabilization Effect

Subsequently, concerning trehalose exhibiting a high thermal stabilization effect, the effective concentration at which its effect was exerted was examined. The method was in accordance with Experiment 3D described above. As a result, as the concentration of added trehalose was increased, the effect tended to increase. It was revealed that even when trehalose was added to a final concentration of 0.01%, a stabilization effect was exerted (Table 2D).

Table 2D

| Final concentration of stabilizing agent | GDH residual activity ratio (%) after treatment at 50°C for 30 min |
|---|---|
| Control (no stabilizing agent) | 3.9 |
| 0.01% trehalose<br>0.1% trehalose<br>1% trehalose | 11.2<br>20.4<br>42.3 |

Experiment 5D: Study on Other Synergistic Effects

In accordance with Experiment 3D above for the basic method, examination was carried out on whether a synergistic effect was observed by combining the other stabilizing agents. As a result, a noticeable thermal stabilization effect was confirmed in the combinations of serine and BSA (Table 3D), trehalose and mannose, trehalose and glycine, and mannose and glycine (Table 4D), as compared with individual use.

Table 3D

| Final concentration of stabilizing agent | GDH residual activity ratio (%) after treatment at 50°C for 30 min |
|---|---|
| 1% cellobiose<br>1% trehalose | 42.2<br>42.4 |
| 1% cellobiose + 1% sericin<br>1% trehalose + 1% sericin | 60.6<br>57.0 |

Table 4D

| GDH sample containing 1% trehalose and 1% sericin as stabilizing agents | | |
|---|---|---|
| GDH activity (U/ml) after storage at 4°C | GDH activity (U/ml) after treatment at 50°C for 30 min | GDH residual activity ratio (%) |
| 9.52 | 5.47 | 57.4 |
| 898.6 | 518.6 | 57.7 |

Experiment 6D: Study on Effective Concentrations of Trehalose and Glycine, and Mannose and Glycine

Concerning the combinations of trehalose and glycine, and glycine and mannose, which exhibited a high thermal stabilization effect in Experiment 5D, the effective concentration at which the effect was exerted was examined. The method was in accordance with Experiment 3 above. As a result, also in these combinations, as the concentration of the added compounds was increased, the effect tended to increase. Even when the compounds were added to a final concentration of 0.01%, a residual activity of about 20% was observed after treatment at 50°C for 30 minutes, and nearly double the stabilization effect was observed compared with using 0.01% trehalose alone (Table 5D).

Table 5D

| Final concentration of stabilizing agent | GDH Residual activity ratio (%) after treatment at 50°C for 15 min | GDH residual activity ratio (%) after treatment at 50°C for 30 min |
|---|---|---|
| Control (no stabilizing agent) | 13 | 0.5 |
| 2.5%BSA<br>10%serine<br>10%serine+2.5%BSA | 20<br>33<br>51 | 3.5<br>11.5<br>25.4 |

Experiment 7D: Study on Thermal Stabilization Effect of Stabilizing Agent on Various Types of FAD-GDH

In accordance with Experiment 3D above for the basic method, the thermal stabilization effect of a stabilizing agent on various types of FAD-GDH was examined. As the stabilizing agent, a mixed composition of 4% sodium D-glucuronate and 4% glycine was used. As a result, it was demonstrated that the effect of the stabilizing agent was observed regardless of whether the GDH is wild type-derived GDH or recombinant GDH (Table 6D).

Table 6D

| Final concentration of stabilizing agent | GDH residual activity ratio (%) after treatment at 50°C for 30 min |
|---|---|
| Control (no stabilizing agent) | 3.50 |
| 1% trehalose | 39.10 |
| 1% mannose | 47.78 |
| 1% glycine | 4.84 |
| 1% trehalose + 1% mannose | 49.54 |
| 1% trehalose + 1% glycine | 51.93 |
| 1% mannose + 1% glycine | 63.41 |

Experiment 8D: Study of Storage Stability Using Glucose Measurement System

The study was performed using the *Aspergillus oryzae*-derived recombinant FAD-GDH preparation (rAO-FAD-GDH) obtained in Experiment 1D in accordance with the method for measuring FAD-GDH activity in Test Experiment 1 above. The amount of FAD-GDH protein in the FAD-GDH enzyme solution was measured, and 1 mL of the solution in which a stabilizing agent had been dissolved in a proportion of 60% or 30% relative to the protein was prepared. For example, when BSA in a proportion of 60% was added to an enzyme solution containing 10 mg of FAD-GDH, 6 mg of BSA was dissolved.

Several vials in which 0.2 mL of each of the enzyme solutions containing the stabilizing agents had been correctly dispensed were prepared. As the control, a vial containing no stabilizing agent was prepared. The prepared vials were subjected to vacuum freeze-drying (FDR) to completely evaporate the water. Immediately thereafter, only two of the samples to which the same stabilizing agent had been added were subjected to the measurement of activity. The remaining vials were treated at 25°C at a humidity of 70% for several hours, and then stored at 37°C for one week. Subsequently, the residual activity was measured. The residual activity ratio (%) was calculated by making the average activity immediately after FDR 100% and measuring the average activity of the samples after storing at 37°C. A higher residual activity ratio was regarded as an enhanced storage stability.

As a result, BSA, serine or trehalose alone was observed to enhance the storage stability of the powdered enzyme, but a further storage stabilization effect was observed by combining BSA and serine. Due to the limited amount of the enzyme preparations, only a few combinations were analyzed, but presumably, compounds that had exhibited a thermal stabilization effect likewise have a storage stabilization effect (Tables 7D and 8D).

Table 7D

| Final concentration of stabilizing agent | GDH residual activity ratio (%) after treatment at 50°C for 30 min | |
|---|---|---|
| | Trehalose + glycine | Mannose + glycine |
| Each 2% | 63.1 | 71.3 |
| Each 1% | 53.7 | 63.8 |
| Each 0.1% | 29.4 | 44.7 |
| Each 0.01% | 18.7 | 22.9 |
| No stabilizing agent | 3.5 | |

Table 8D

| | GDH residual activity ratio (%) after treatment at 50°C for 30 min | |
|---|---|---|
| | Stabilizing agent (-) | Stabilizing agent (+) |
| GDH derived from wild type *A. terreus* subspecies | 70.0 | 89.2 |
| Recombinant GDH derived from *P. italicum* | 2.7 | 92.9 |
| GDH derived from wild type *A. oryzae* | 73.2 | 94.2 |
| Recombinant GDH derived from *A. oryzae* | 2.9 | 71.8 |

Experiment 9D: Study on FAD-GDH Thermal Stabilization Effect of Various Stabilizing Agents Using the Glucose Measurement System

The study was performed in accordance with the method for measuring the FAD-GDH activity in Test Experiment 1 above.

First, 50 mL of an enzyme solution in which the *Aspergillus terreus*-derived recombinant FAD-GDH preparation (rAT-FAD-GDH) obtained in Experiment 1D had been dissolved at about 2 U/mL of in an enzyme dilution solution (50 mM potassium phosphate buffer (pH 5.5), 0.1% Triton X-100) was prepared. Two tubes were prepared in which each of the stabilizing agents shown in Table 1D had been added at each final concentration to 0.9 mL of the above enzyme solution to make a total volume of 1.0 mL As the control, two tubes in which 0.1 mL of distilled water had been added in place of each compound were prepared.

Of the two tubes, one was stored at 4°C and the other was treated at 50°C for 15 minutes. Then, the FAD-GDH activity in each tube was measured. The enzyme activity in the tube stored at 4°C was made 100, and comparing with it, the activity value after treatment at 50°C for 15 minutes was calculated as the residual activity ratio (%).

These experiments revealed that the thermal stability of FAD-GDH was increased by adding sugars and certain types of amino acids, which were not the substrates of FAD-GDH (Tables 9D and 10D).

A higher thermal stabilization effect was observed with the sugars than with the amino acids. Among them, high effects were observed with trehalose, mannose, melezitose, sodium gluconate, sodium glucuronate, galactose, methyl-O-D-glycoside, □-D-melibiose, sucrose, glycine, alanine, serine, sodium chloride, sodium sulfate, trisodium citrate, ammonium sulfate, succinic acid, malonic acid, glutaric acid, arabinose, sorbitan, 2-deoxy-D-glucose, xylose, fructose, sodium aspartate, glutamic acid, phenylalanine, proline, lysine hydrochloride, sarcosine and taurine.

Table 9D

| Stabilizing agent | GDH activity (kU/vial) | | GDH residual activity ratio (%) |
|---|---|---|---|
| | Immediately after pulverization | After 1 week at 37°C | |
| Control(no stabilizing agent) | 18.3 | 1.6 | 8.9 |
| 60% BSA | 16.7 | 4.3 | 25.9 |
| 60% serine | 19.8 | 8.2 | 41.3 |
| 60% BSA x 60% serine | 19.5 | 14.0 | 71.6 |
| * After treatment at 70% humidity, 25°C , 24 h, and then allowed to stand at 37°C for 1 week. | | | |

Table 10D

| Stabilizing agent | GDH activity (U/vial) | | | GDH residual activity ratio (%) |
|---|---|---|---|---|
| | Immediately before pulverization | Immediately after pulverization | At 37°C for 1 week | |
| Control (no stabilizing agent) | 403 | 352 | 6 | 1.6 |
| 60% trehalose | 397 | 401 | 195 | 48.6 |

(continued)

| Stabilizing agent | GDH activity (U/vial) | | | GDH residual activity ratio (%) |
|---|---|---|---|---|
| | Immediately before pulverization | Immediately after pulverization | At 37°C for 1 week | |
| 30% trehalose + 30% sericin | 405 | 385 | 288 | 74.7 |
| * After treatment at 70% humidity, 25°C , 7 h, and then allowed to stand at 37°C for 1 week. | | | | |

INDUSTRIAL APPLICABILITY

The present invention can be useful in a composition for measuring a glucose level or a method for measuring a glucose level. The composition for measuring a glucose level or the method for measuring a glucose level can be used for a glucose assay kit and a glucose sensor.

The present invention makes it possible to produce glucose dehydrogenase derived from *Aspergillus oryzae* on a large scale by the use of recombinant *Escherichia coli.* According to the present invention, it is possible to produce glucose dehydrogenase that does not act upon maltose in a broad sense and is suitable as a glucose sensor and the like.

The present invention makes it possible to produce glucose dehydrogenase derived from *Aspergillus oryzae* on a large scale by the use of recombinant *Escherichia* coli. By the present invention, it becomes possible to produce glucose dehydrogenase that does not act upon maltose in a broad sense and is suitable as a glucose sensor and the like.

As disclosed herein, by enhancing the stability of the GDH composition, it becomes possible to reduce thermal deactivation upon production of a glucose measurement reagent, a glucose assay kit and a glucose sensor to reduce the amount of enzyme to be used and enhance the accuracy of the measurement. It also becomes possible to provide a reagent for measuring a blood glucose level using the GDH composition that has excellent storage stability.

## Claims

**1.** A gene composed of the following DNA (a), (b), (c) or (d) :

(a) DNA composed of a base sequence of SEQ ID NO: 11;
(b) DNA composed of a base sequence of SEQ ID NO: 14, comprising the base sequence of SEQ ID NO: 11 and an intron;
(c) DNA which hybridizes with DNA composed of a base sequence complementary to DNA (a) under stringent conditions and encodes a protein having glucose dehydrogenase activity;
(d) DNA which hybridizes with DNA composed of a base sequence complementary to DNA (b) under stringent conditions and comprises a region encoding a protein having glucose dehydrogenase activity.

**2.** A gene encoding the following protein (a) or (b):

(a) a protein composed of an amino acid sequence of SEQ ID NO: 10;
(b) a protein composed of an amino acid sequence having one or a few amino acid deletions, substitutions, additions or insertions in the amino acid sequence of SEQ ID NO: 10, and
having glucose dehydrogenase activity.

**3.** A recombinant vector comprising the gene according to claim 1 or 2.

**4.** A transformant transformed with the recombinant vector according to claim 3.

**5.** The transformant according to claim 4 wherein a host is *Escherichia coli.*

**6.** A method for producing a protein having glucose dehydrogenase activity, **characterized in that** the transformant according to claim 4 or 5 is cultured in a nutrient medium and the protein having glucose dehydrogenase activity is collected.

**7.** A glucose dehydrogenase comprising an amino acid sequence of SEQ ID NO: 10.

**Patentansprüche**

1. Gen, das aus den folgenden DNAs (a), (b), (c) oder (d) besteht:

   (a) einer DNA, die aus einer Basensequenz gemäß SEQ ID Nr.: 11 besteht;
   (b) einer DNA, die aus einer Basensequenz gemäß SEQ ID Nr.: 14 besteht und die Basensequenz gemäß SEQ ID Nr.: 11 und ein Intron aufweist;
   (c) einer DNA, die mit einer DNA hybridisiert, die aus einer zur DNA (a) unter stringenten Bedingungen komplementären Basensequenz besteht und für ein Protein mit Glucosedehydrogenase-Aktivität codiert;
   (d) einer DNA, die mit einer DNA hybridisiert, die aus einer zur DNA (b) unter stringenten Bedingungen komplementären Basensequenz besteht und eine Region aufweist, die für ein Protein mit Glucesedehydregenase-Aktivitäz codiert.

2. Gen, das für das folgende Protein (a) oder (b) codiert:

   (a) ein Protein, das aus einer Aminosäuresequenz gemäß SEQ ID Nr.: 10 besteht;
   (b) ein Protein, das aus einer Aminosäuresequenz mit einer oder mehreren Aminosäurcdeletionen, -substitutionen, -additionen oder -insertionen in der Aminosäuresequenz gemäß SEQ ID Nr.: 10 besteht und Glucosedehydrogenase-Aktivität aufweist

3. Rekombinantes Vektor, der das Gen nach Anspruch 1 oder 2 aufweist.

4. Transformante, die mit dem rekombinanten Vektor nach Anspruch 3 transformiert ist.

5. Transformant nach Anspruch 4, wobei ein Wirt Escherichia coli ist.

6. Verfahren zur Erzeugung eines Proteins mit Glucosedehydrogenase-Aktivität, **dadurch gekennzeichnet, dass** die Transformante nach Anspruch 4 oder 5 in einem Nährmedium kultiviert wird und das Protein mit Glucosedehydrogenase-Aktivität gewonnen wird.

7. Glucosedehydrogenase, die eine Aminosäuresequenz gemäß SEQ ID Nr.: 10 aufweist.

**Revendications**

1. Gène composé de l'ADN (a), (b), (c) ou (d) suivant:

   (a) ADN composé d'une séquence de bases de la SEQ ID NO:11;
   (b) ADN composé d'une séquence de bases de la SEQ ID NO:14, comprenant la séquence de bases de la SEQ ID NO:11 et un intron;
   (c) ADN qui s'hybride avec un ADN composé d'une séquence de bases complémentaire à l'ADN (a) dans des conditions rigoureuses et code pour une protéine possédant une activité de glucose déshydrogénase;
   (d) ADN qui s'hybride avec un ADN composé d'une séquence de bases complémentaire à l'ADN (b) dans des conditions rigoureuses et comprend une région codant pour une protéine possédant une activité de glucose déshydrogénase.

2. Gène codant pour la protéine (a) ou (b) suivante:

   (a) une protéine composée d'une séquence d'acides aminés de la SEQ ID NO:10;
   (b) une protéine composée d'une séquence d'acides aminés possédant une ou quelques délétions, substitutions, additions ou insertions d'acide aminé dans la séquence d'acides aminés de la SEQ ID NO:10 et possédant une activité de glucose déshydrogénase.

3. Vecteur recombinant comprenant le gène selon la revendication 1 ou 2.

4. Transformant transformé avec le vecteur recombinant selon la revendication 3.

5. Transformant selon la revendication 4, dans lequel un hôte est l'*Escherichia coli*.

6. procédé pour la production d'une protéine possédant une activité de glucose déshydrogénase, **caractérisé en ce que** le transformant selon la revendication 4 ou 5 est cultivé dans un milieu nutritif et la protéine possédant une activité de glucose déshydrogénase est récupérée.

7. Glucose déshydrogénase comprenait une séquence d'acides aminés de la SEQ ID NO:10.

Fig.1

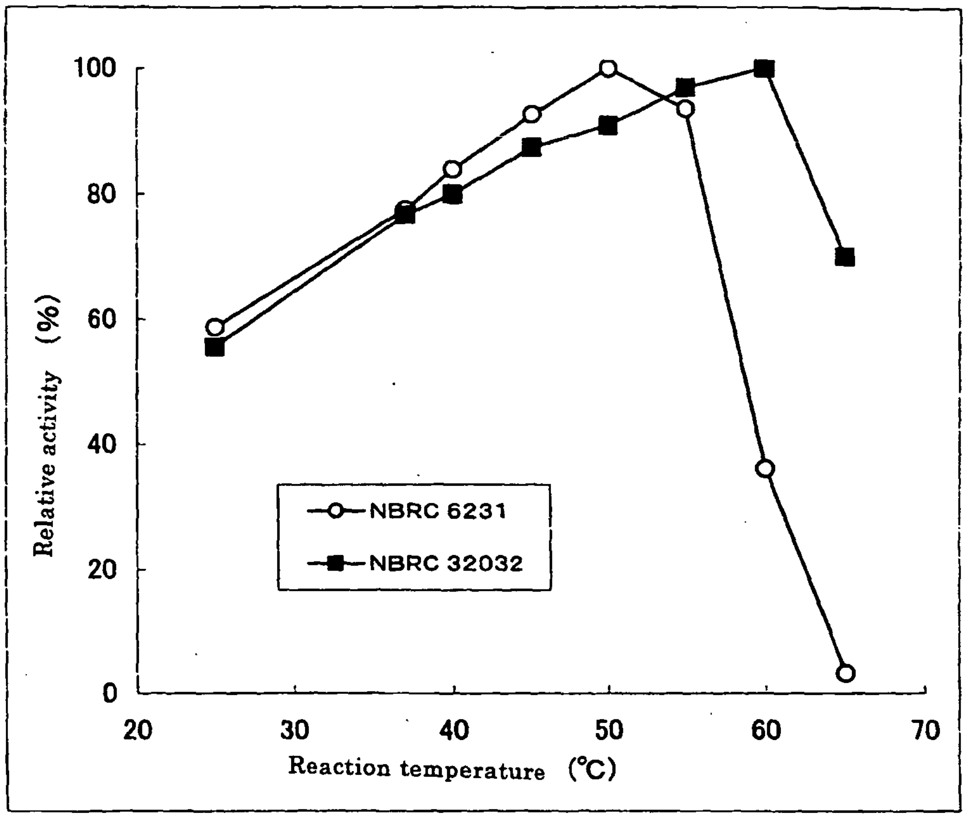

100
80
60
40
20
0
Relative activity (%)
NBRC 6231
NBRC 32032
20
30
40
50
60
70
Reaction temperature (°C)


Fig.2

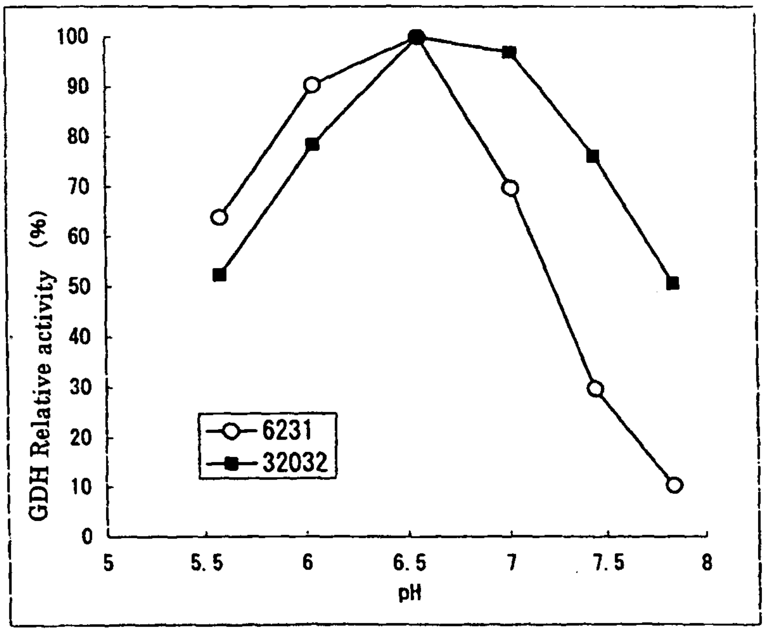

100
90
80
70
60
50
40
30
20
10
0
GDH Relative activity (%)
6231
32032
5
5.5
6
6.5
7
7.5
8
pH

Fig.3

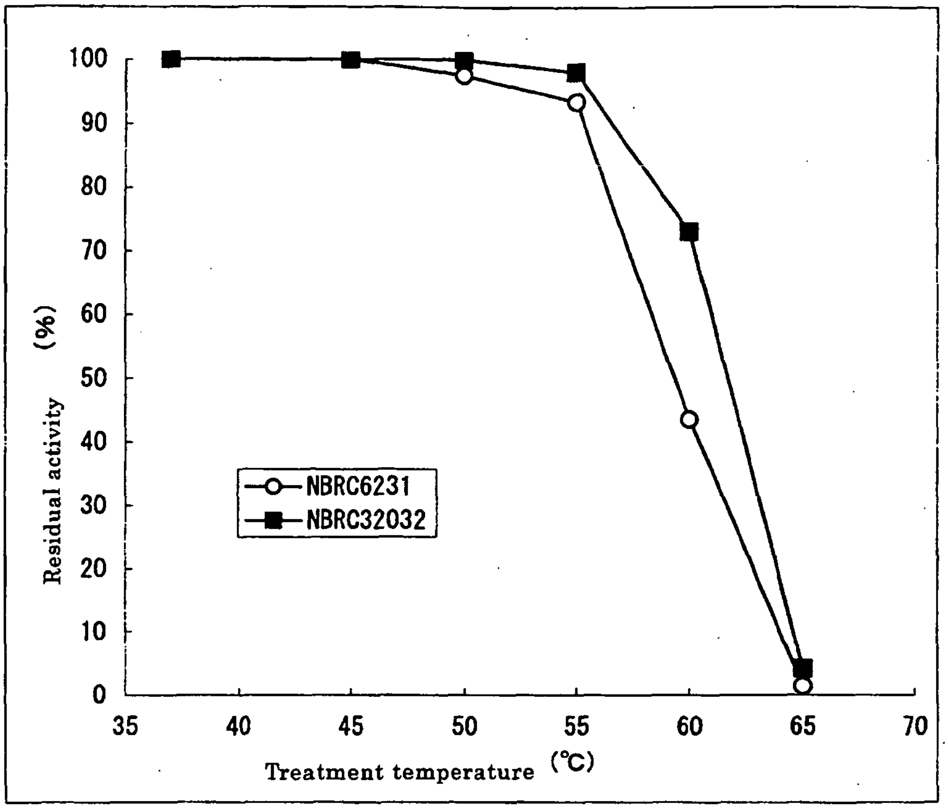
100
90
80
70
60
50
40
30
20
10
0
Residual activity (%)
NBRC6231
NBRC32032
35
40
45
50
55
60
65
70
Treatment temperature (℃)

Fig.4

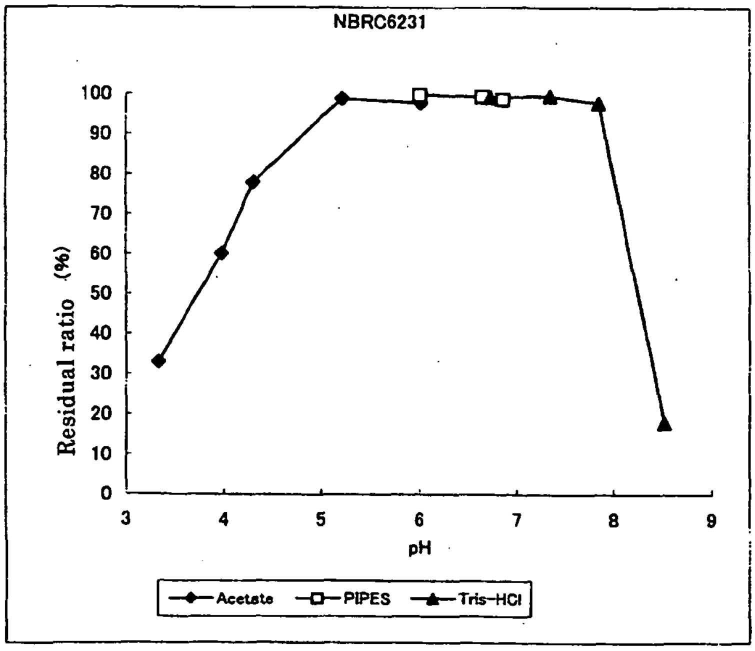
NBRC6231
100
90
80
70
60
50
40
30
20
10
0
Residual ratio (%)
3
4
5
6
7
8
9
pH
Acetate
PIPES
Tris-HCl

Fig.5

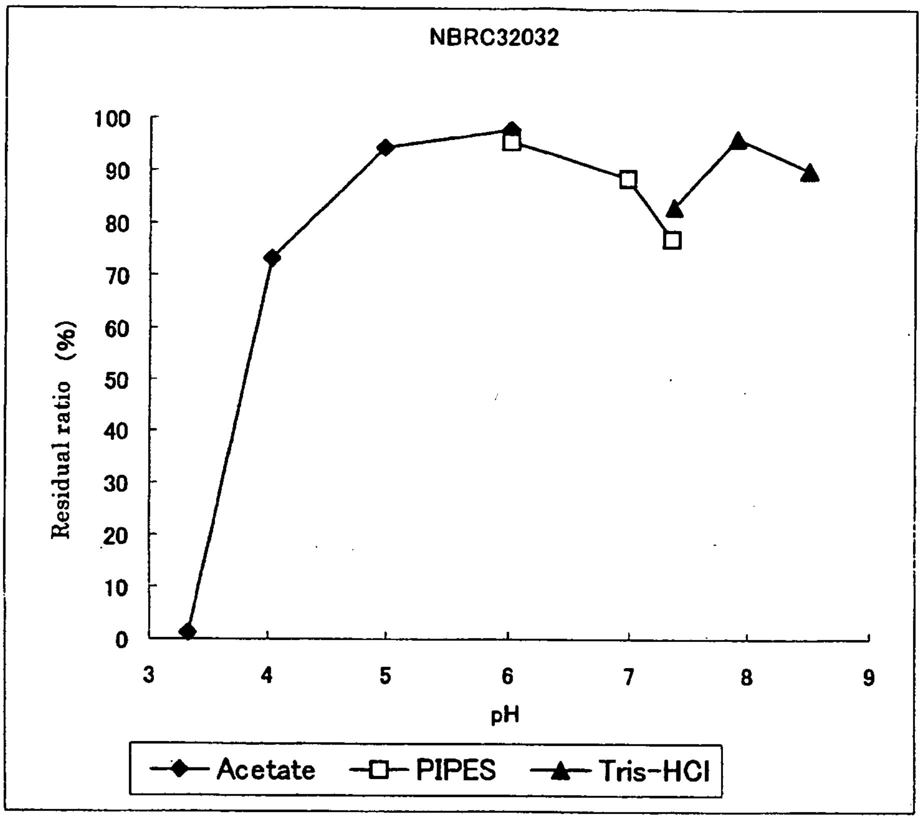
NBRC32032
Residual ratio (%)
100
90
80
70
60
50
40
30
20
10
0
3
4
5
6
7
8
9
pH
Acetate
PIPES
Tris-HCl

Fig.6

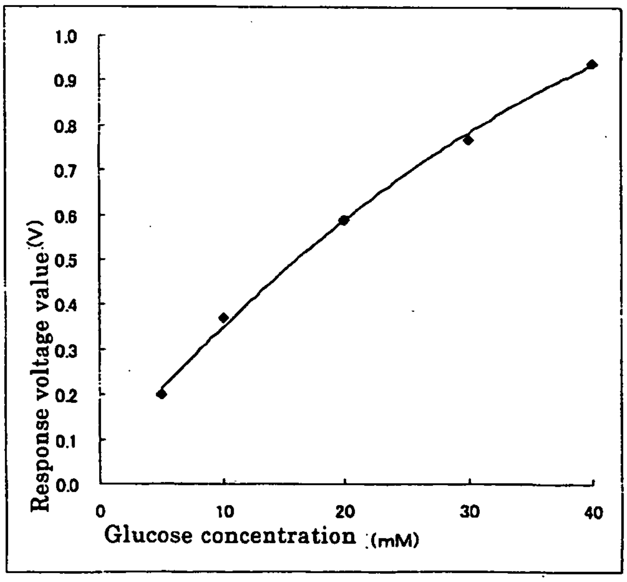
Response voltage value (V)
1.0
0.9
0.8
0.7
0.6
0.5
0.4
0.3
0.2
0.1
0.0
0
10
20
30
40
Glucose concentration (mM)

Fig.7

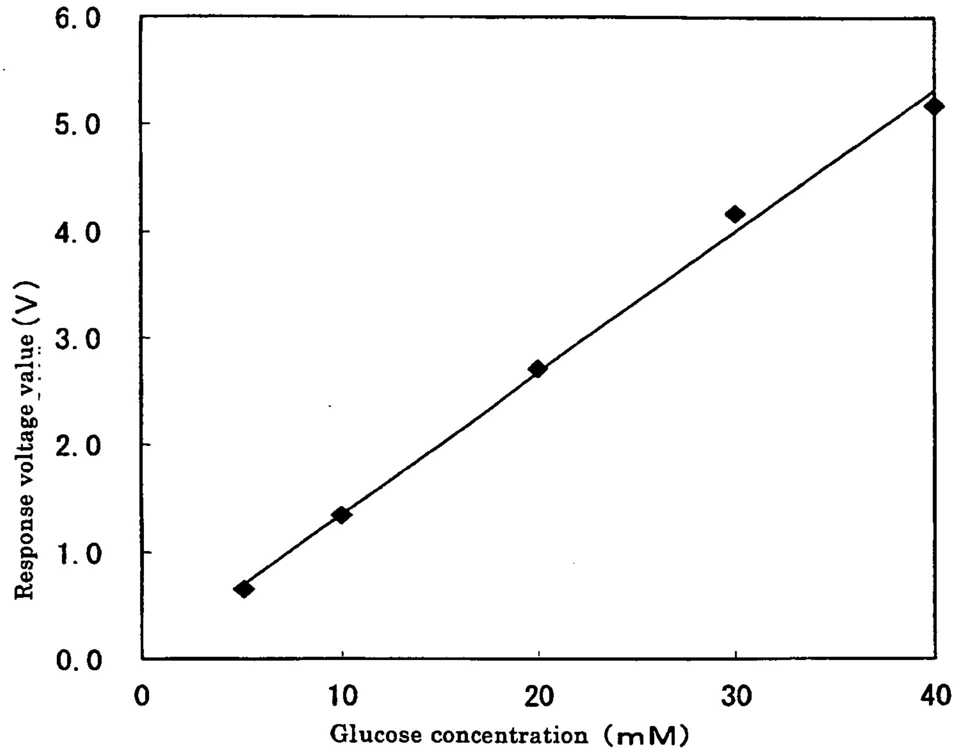
6.0
5.0
4.0
3.0
2.0
1.0
0.0
Response voltage value (V)
0
10
20
30
40
Glucose concentration (mM)

Fig.8

SignalP-NN result:

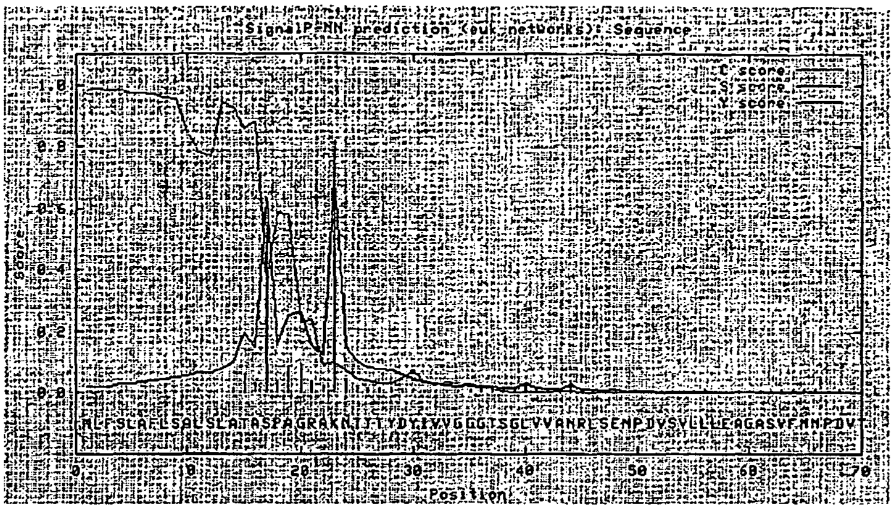


>Sequence length = 70

| # Measure | Position | Value | Cutoff | signal peptide? | |
|---|---|---|---|---|---|
| max. C | 23 | 0.814 | 0.32 | YES | (Cleavage site) |
| max. Y | 23 | 0.700 | 0.33 | YES | (Score of cleavage site considering S score) |
| max. S | 2 | 0.990 | 0.87 | YES | (Signal peptide region) |
| mean S | 1-22 | 0.767 | 0.48 | YES | |
| D | 1-22 | 0.733 | 0.43 | YES | |

# Most likely cleavage site between pos. 22 and 23: GRA-KN

SignalP-HMM result:

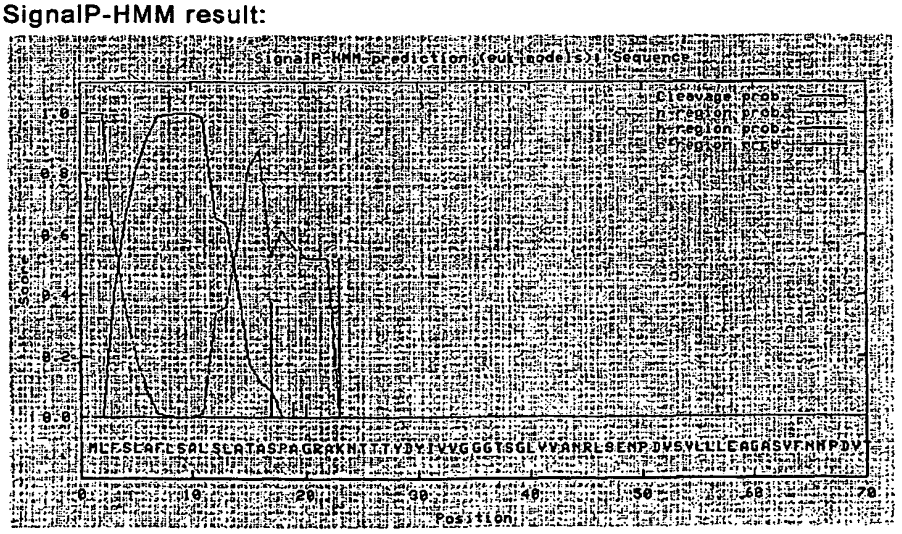


h-region prob.: (Hydrophobic region) c-region prob.: (Region from hydrophobic region to cleavage site)

>Sequence

Prediction: Signal peptide

Signal peptide probability: 1.000

Signal anchor probability: 0.000

Max cleavage site probability: 0.520 between pos. 22 and 23

Fig.9

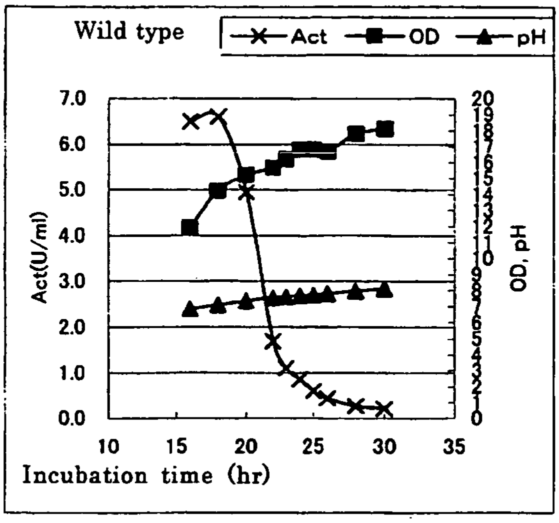
Wild type
Act
OD
pH
7.0
6.0
5.0
4.0
3.0
2.0
1.0
0.0
Act(U/ml)
20
19
18
17
16
15
14
13
12
11
10
9
8
7
6
5
4
3
2
1
0
OD, pH
10
15
20
25
30
35
Incubation time (hr)

Fig.10

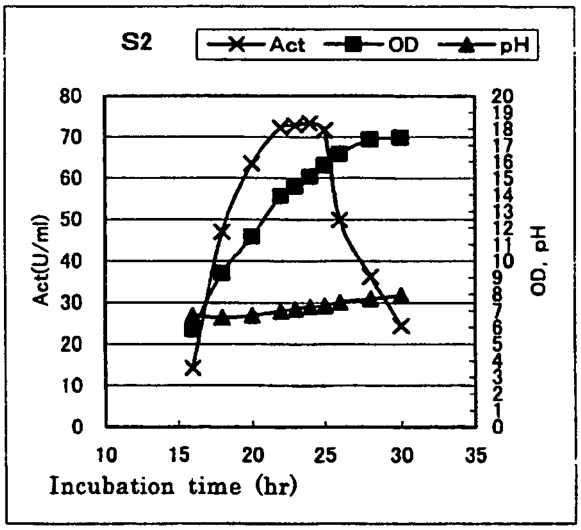
S2
Act
OD
pH
80
70
60
50
40
30
20
10
0
Act(U/ml)
20
19
18
17
16
15
14
13
12
11
10
9
8
7
6
5
4
3
2
1
0
OD, pH
10
15
20
25
30
35
Incubation time (hr)

Fig.11

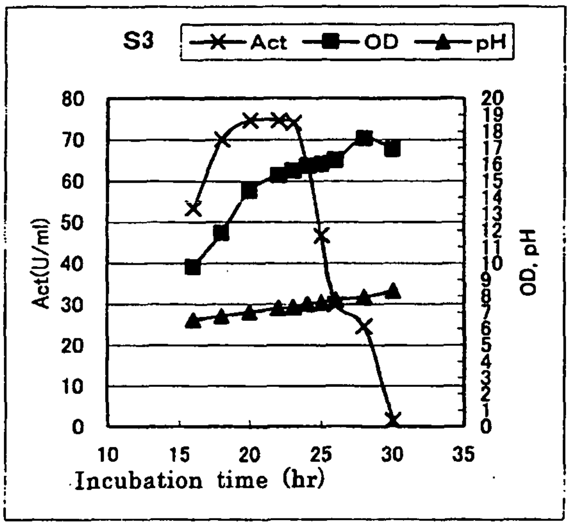
S3
Act
OD
pH
Act(U/ml)
OD, pH
Incubation time (hr)

Fig.12

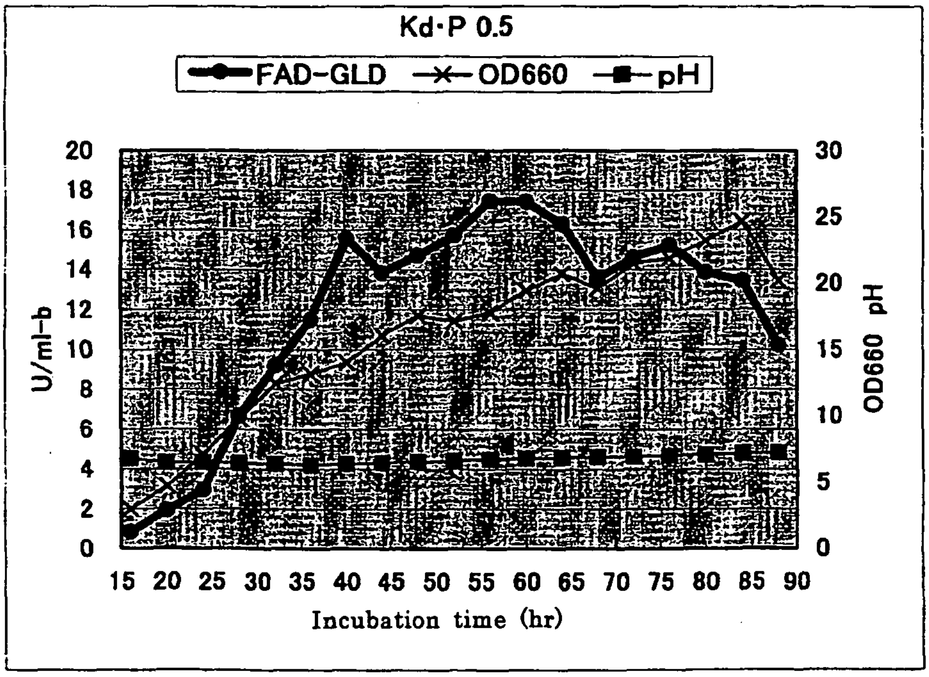
Kd·P 0.5
FAD-GLD
OD660
pH
U/ml-b
OD660 pH
Incubation time (hr)

Fig.13

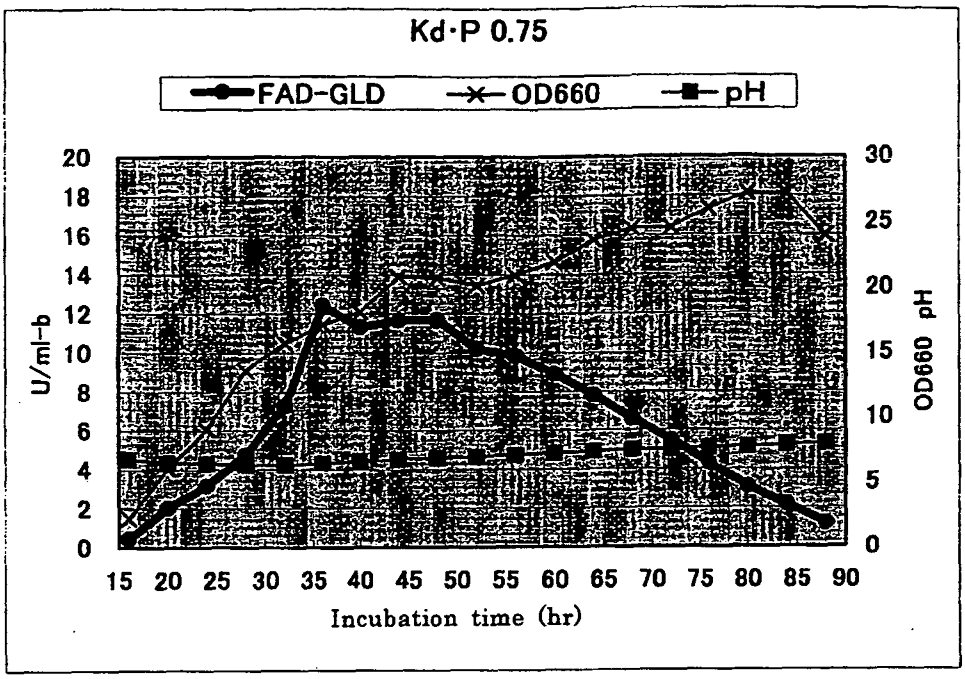
Kd·P 0.75
FAD-GLD
OD660
pH
U/ml-b
OD660 pH
0 2 4 6 8 10 12 14 16 18 20
0 5 10 15 20 25 30
15 20 25 30 35 40 45 50 55 60 65 70 75 80 85 90
Incubation time (hr)

Fig.14

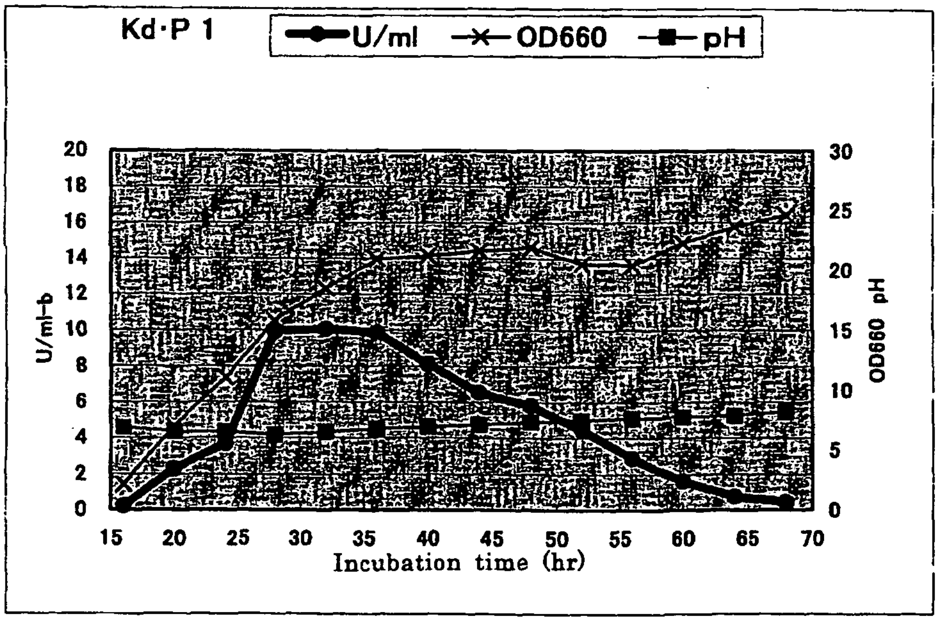
Kd·P 1
U/ml
OD660
pH
U/ml-b
OD660 pH
0 2 4 6 8 10 12 14 16 18 20
0 5 10 15 20 25 30
15 20 25 30 35 40 45 50 55 60 65 70
Incubation time (hr)

Fig.15

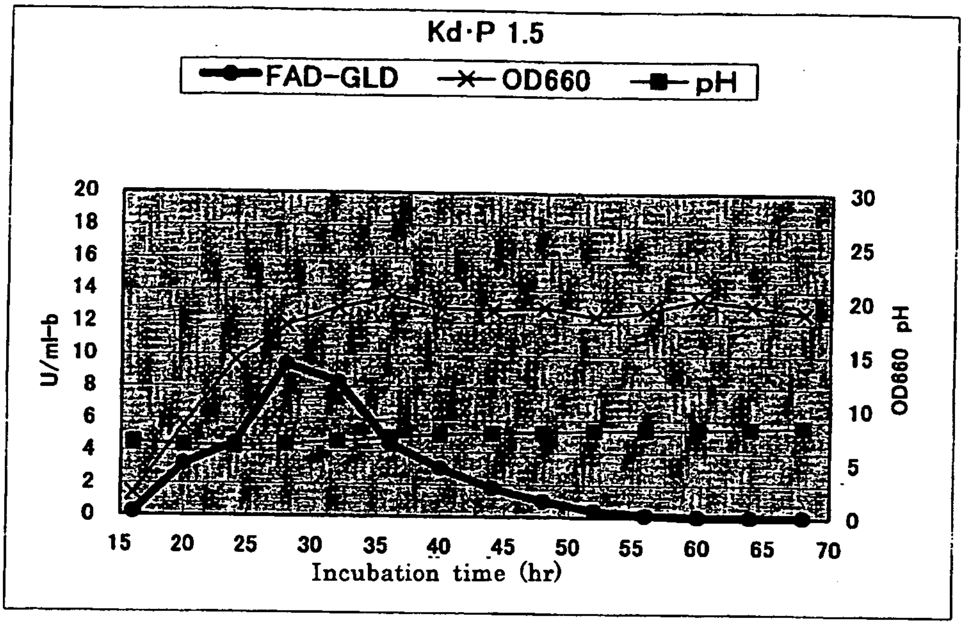
Kd·P 1.5
FAD-GLD
OD660
pH
20
18
16
14
12
10
8
6
4
2
0
U/ml-b
30
25
20
15
10
5
0
OD660 pH
15 20 25 30 35 40 45 50 55 60 65 70
Incubation time (hr)

Fig.16

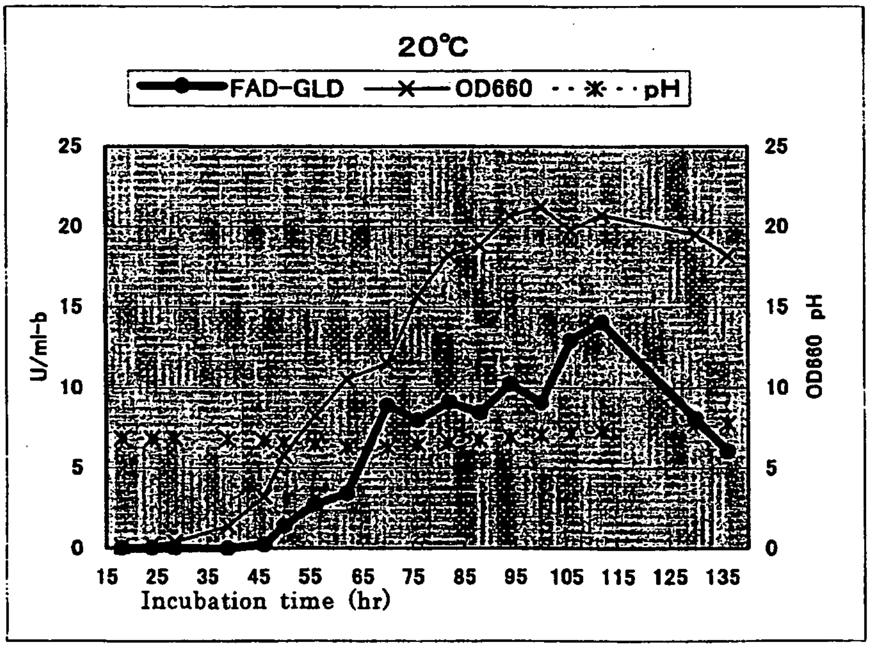
20℃
FAD-GLD
OD660
pH
25
20
15
10
5
0
U/ml-b
25
20
15
10
5
0
OD660 pH
15 25 35 45 55 65 75 85 95 105 115 125 135
Incubation time (hr)

Fig.17

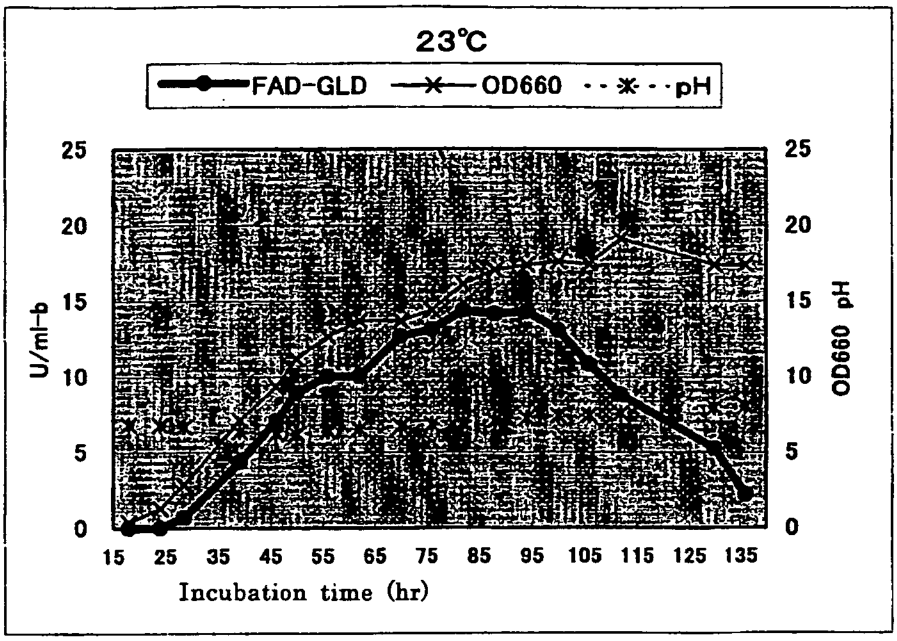
23℃
FAD-GLD
OD660
pH
25
20
15
10
5
0
U/ml-b
OD660 pH
15 25 35 45 55 65 75 85 95 105 115 125 135
Incubation time (hr)

Fig.18

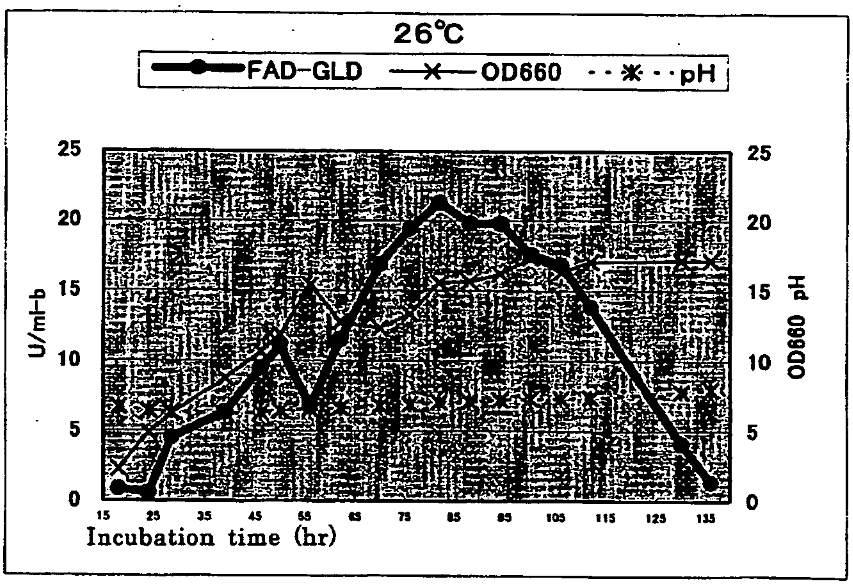
26℃
FAD-GLD
OD660
pH
25
20
15
10
5
0
U/ml-b
OD660 pH
15 25 35 45 55 65 75 85 95 105 115 125 135
Incubation time (hr)

Fig.19

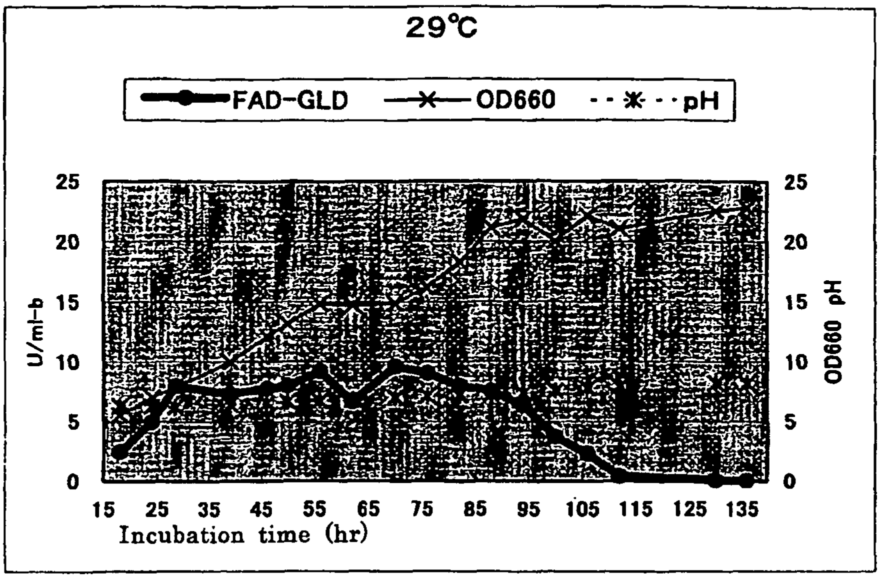
29℃
FAD-GLD
OD660
pH
U/ml-b
OD660 pH
25
20
15
10
5
0
15 25 35 45 55 65 75 85 95 105 115 125 135
Incubation time (hr)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004058958 A
- WO 2006101239 A
- WO 02072839 A
- US 60788252 B
- JP 2006319645 A
- JP 2006319644 A
- JP 2006319643 A
- JP 2006354168 A


### Non-patent literature cited in the description


- *Biochim Biophys Acta,* 11 July 1967, vol. 139 (2), 265-76
- *Biochim Biophys Acta,* 11 July 1967, vol. 139 (2), 277-93
- *Biochim Biophys Acta,* vol. 146 (2), 317-27
- *Biochim Biophys Acta,* vol. 146 (2), 328-35
- Tanpakushitsu Jikken Protocol Vol. 1, Functional Analysis. Structural Analysis. Shujunsha, vol. 2
- Revised Tanpakushitsu Jikken Note, Extraction and Separation/Purification. Yodosha
- Tanpakushitsu Jikken no Susumekata. Yodosha
- *Science,* 1981, vol. 214, 1205
- **Karlin et al.** *Proc. Natl. Acad. Sci. USA,* 1993, vol. 90, 5873-5877
- Tanpakushitsu Jikken Protocol. Functional Analysis Vol. 2, Structural Analysis. vol. 1, 2
- **Sambrook et al.** Molecular Cloning; A Laboratory Manual. Cold Spring Harbor Laboratory Press, 1989

SEQUENCE LISTING

<110> TOYO BOSEKI KABUSHIKI KAISHA

<120> GLUCOSE DEHYDROGENASE

<130> P07-26

<150> US 60/788,252
<151> 2006-03-31

<150> JP 2006-319645
<151> 2006-11-28

<150> JP 2006-319644
<151> 2006-11-28

<150> JP 2006-319643
<151> 2006-11-28

<150> JP 2006-354168
<151> 2006-12-28

<160> 23

<170> Patent In version 3.3

<210> 1
<211> 1773
<212> DNA

<213> Penicillium lilacinoechinulatum

<400> 1

```
atgaggagct tgataagcct agctctcttg cctttggcag ctgcagttcc ccatgtttca     60
cgtagctctg agactacata cgactacatt gtagttggag gtggaactag tggcctggtt    120
atagctaatc gactttctga gcttgaaaaa gtgaatgttc tcgttattga agccggtggc    180
tcagtgtaca acaatcctaa tgtaaccgat accgccggat atggaaaggc ctttggcact    240
gatattgatt gggcatatga gacagtcaag caagaatggg gaggaggtac caaacaaaca    300
gtaagagctg gaaaggctct cggaggtacc tcaaccatca acggaatggt ctatctgcgg    360
gctcagaaaa gtcagatcga tgcgtgggag aagatcggaa atgacggctg gaactggaag    420
aacctgttcc cttactatcg caagggagaa aaatttcaag ttccaaccga ctatgcattt    480
ttggaaggaa ccggcgttgc ctatgatcca gctttccatg ggtataacgg tcctctgaag    540
gtgggctgga cctcaacaca attgaacgat ggccttgccc aagtgatgaa ctctacttac    600
cagaatatgt cggtccctgt cccatacaac aaggatccaa acggtggaca aatggtcgga    660
tactcggttt accccaagac tgtcaactcg gaactcaata ttcgtgagga tgctgccaga    720
```

gcatactact acccctatca aaaccgaacc aaccttcatg tttggcttaa ttctcatgtc 780

aataagcttg tttggaagga tggagccaac atgaccgcag atggcgtgga agtcaagttc 840

tccaacggca caactgctac cgttaaggca gcgcgtgaag tgatccttgc ggcaggcgca 900

ctgaagtctc cgcttctact cgaattgtct ggagttggaa accctgacat cctctcgagg 960

cacggcatcg atacgaagat aaacctgcca accattggcg agaatctcca agatcaaatg 1020

aacaacggac tcgcttacac ctccaagaaa aactacacca aggcggcctc ttacgtcgca 1080

tacccttcag ccgaagaact ctttacaaac gcgaccacca ttggtgctca acttctgcgc 1140

aaacttcccg catacgcagc tcaggttgct tcagccaatg gtaatgtgac tcgggccgct 1200

gatatcgagc gcttcttcaa gatccagtgg gacttgatct tcaaatctca tatcccagtc 1260

gcagaaatct tgctggagcc atttggtttt acttatgact cggagtattg gggatcggtt 1320

ccattttcgc gtggcagcat tcacatctca tcttctgacc caactgcccc ggccatcatt 1380

gatcctaagt atttcatgtt ggattttgat ttccatgccc aggttgaggc agctcgcttt 1440

attcgtgagt tgtttaagac tgagccattt gccgatatgg caggtgccga gacaagcccg 1500

ggtctttcag ctgtctcttc caatgctgat gacgaagggt ggtcttcatt cctcaagtct 1560

aacttccgat cgaacttcca ccctatcacc acggctggca tgatgccaaa ggaaattggt 1620

ggtgttgtgg acacttcttt gaaggtctat ggaacttcga atgttcgtgt tgtcgacgcc 1680

tcggtgatcc cattccaggt ttgcggacac ttgcaaagca ctatctacgc ggttgccgag 1740

cgcgcagccg acatcataaa agctcaaatg tag 1773

<210> 2
<211> 590
<212> PRT
<213> Penicillium lilacinoechinulatum

<400> 2

Met Arg Ser Leu Ile Ser Leu Ala Leu Leu Pro Leu Ala Ala Ala Val
1 5 10 15

Pro His Val Ser Arg Ser Ser Glu Thr Thr Tyr Asp Tyr Ile Val Val
20 25 30

Gly Gly Gly Thr Ser Gly Leu Val Ile Ala Asn Arg Leu Ser Glu Leu
35 40 45

Glu Lys Val Asn Val Leu Val Ile Glu Ala Gly Gly Ser Val Tyr Asn
50 55 60

Asn Pro Asn Val Thr Asp Thr Ala Gly Tyr Gly Lys Ala Phe Gly Thr
65 70 75 80

Asp Ile Asp Trp Ala Tyr Glu Thr Val Lys Gln Glu Trp Gly Gly Gly
85 90 95

Thr Lys Gln Thr Val Arg Ala Gly Lys Ala Leu Gly Gly Thr Ser Thr
100 105 110

Ile Asn Gly Met Val Tyr Leu Arg Ala Gln Lys Ser Gln Ile Asp Ala
115 120 125

Trp Glu Lys Ile Gly Asn Asp Gly Trp Asn Trp Lys Asn Leu Phe Pro
130 135 140

Tyr Tyr Arg Lys Gly Glu Lys Phe Gln Val Pro Thr Asp Tyr Ala Phe
145 150 155 160

Leu Glu Gly Thr Gly Val Ala Tyr Asp Pro Ala Phe His Gly Tyr Asn
165 170 175

Gly Pro Leu Lys Val Gly Trp Thr Ser Thr Gln Leu Asn Asp Gly Leu
180 185 190

Ala Gln Val Met Asn Ser Thr Tyr Gln Asn Met Ser Val Pro Val Pro
195 200 205

Tyr Asn Lys Asp Pro Asn Gly Gly Gln Met Val Gly Tyr Ser Val Tyr
210 215 220

Pro Lys Thr Val Asn Ser Glu Leu Asn Ile Arg Glu Asp Ala Ala Arg
225 230 235 240

Ala Tyr Tyr Tyr Pro Tyr Gln Asn Arg Thr Asn Leu His Val Trp Leu
245 250 255

Asn Ser His Val Asn Lys Leu Val Trp Lys Asp Gly Ala Asn Met Thr
260 265 270

Ala Asp Gly Val Glu Val Lys Phe Ser Asn Gly Thr Thr Ala Thr Val

275 280 285

Lys Ala Ala Arg Glu Val Ile Leu Ala Ala Gly Ala Leu Lys Ser Pro
290 295 300

Leu Leu Leu Glu Leu Ser Gly Val Gly Asn Pro Asp Ile Leu Ser Arg
305 310 315 320

His Gly Ile Asp Thr Lys Ile Asn Leu Pro Thr Ile Gly Glu Asn Leu
325 330 335

Gln Asp Gln Met Asn Asn Gly Leu Ala Tyr Thr Ser Lys Lys Asn Tyr
340 345 350

Thr Lys Ala Ala Ser Tyr Val Ala Tyr Pro Ser Ala Glu Glu Leu Phe
355 360 365

Thr Asn Ala Thr Thr Ile Gly Ala Gln Leu Leu Arg Lys Leu Pro Ala
370 375 380

Tyr Ala Ala Gln Val Ala Ser Ala Asn Gly Asn Val Thr Arg Ala Ala
385 390 395 400

Asp Ile Glu Arg Phe Phe Lys Ile Gln Trp Asp Leu Ile Phe Lys Ser
405 410 415

His Ile Pro Val Ala Glu Ile Leu Leu Glu Pro Phe Gly Phe Thr Tyr
420 425 430

Asp Ser Glu Tyr Trp Gly Ser Val Pro Phe Ser Arg Gly Ser Ile His
435 440 445

Ile Ser Ser Ser Asp Pro Thr Ala Pro Ala Ile Ile Asp Pro Lys Tyr
450 455 460

Phe Met Leu Asp Phe Asp Phe His Ala Gln Val Glu Ala Ala Arg Phe
465 470 475 480

Ile Arg Glu Leu Phe Lys Thr Glu Pro Phe Ala Asp Met Ala Gly Ala
485 490 495

Glu Thr Ser Pro Gly Leu Ser Ala Val Ser Ser Asn Ala Asp Asp Glu
500 505 510

Gly Trp Ser Ser Phe Leu Lys Ser Asn Phe Arg Ser Asn Phe His Pro
515 520 525

Ile Thr Thr Ala Gly Met Met Pro Lys Glu Ile Gly Gly Val Val Asp
530 535 540

Thr Ser Leu Lys Val Tyr Gly Thr Ser Asn Val Arg Val Val Asp Ala
545 550 555 560

Ser Val Ile Pro Phe Gln Val Cys Gly His Leu Gln Ser Thr Ile Tyr
565 570 575

Ala Val Ala Glu Arg Ala Ala Asp Ile Ile Lys Ala Gln Met
580 585 590

<210> 3
<211> 1779
<212> DNA
<213> Penicillium italicum

<400> 3

atgcgcagcc tcatcggtct tgcactgctt ccactagcag ttgcagtccc ccatgcctca 60
cacaagtcag actctaccta tgactacatt attgttggag gtggcaccag tggcctcgtt 120
gttgcaaacc ggttgtccga gcaaaaggac accaccgtcc tcgtgatcga agccggcggc 180
tccgtatata acaacccaaa tgtgaccaac actctgggat acggtaaagc gttcggtaca 240
gatattgact gggcctacga gacgacagcc caagaacatg ccggtggatt cccacaaata 300
gtgcgtgccg gaaaggcact tggaggaaca tcgaccatca acggcatggc ctacctccgt 360
gcccaggcag cccagattga cgcatgggaa accgttggca acaagggctg gaactggaag 420
actctcctcc cctacttcaa gaagagcgag cagttccaag atccggcaaa gtacccattc 480
ttggatggat cgggtgtctc ctttgatccg gcctaccacg gctttactgg gcctttgaag 540
gttggctggt cttcaacaca gctgaacgat ggtctcgctc aaaagttgaa cgctacctac 600
cagagcctcg acgttcctgt tccgtacaac cgggacgcca atagcggaga catggttgga 660
tacagtgtgt atcccaagac agtcaatgct gatctcaaca tccgtgagga tgctgccccgt 720
gccttctatt atccttacca gaacagaaca aacctccacg tctggctcaa cacacacgcc 780
aacaagatta cctggaatga gggcagcgag gccaccgcaa atggtgtcga agtcactctt 840
tccaacggca aaaagacagt ggtgaaggct acccgtgaag tgattctcgc tgctggcgca 900

ttgaaatctc ccgtcctgct cgagctttct ggcgttggaa accccgacat tctttccaag 960

cacgggatta ccaccaagat taacctgcca actgtcggtg aaaacttgca ggaccaaatg 1020

aacaatggcc ttaagttcga gtcaaagaag acctacagta ccgataaggg tagttcctac 1080

gtggcctacc cctcagctga ccagctcttc cccaactcca ccgcgctggg agccgacctt 1140

cttcgcaagc ttcccgctta tgcagcccag gttgcatccg ccaacggcaa catcaccaaa 1200

gcccgcgaca tttaccgctt cttcaagatc cagtgggatt tgatctttaa ggatgaaatt 1260

cctgtcgcag agatcctgct ctcgggctcc ggagcctcat acagcggcga gtactggggt 1320

tctgttccgt tctctcgcgg cagcgttcac ctttcttccg cagaccccac ggcggcccct 1380

accattgacc ccaagtactt catgctggac tttgatctcc acgctcaggc acaggcggcg 1440

cggttcattc gtgaaatctt caagaccgag ccacttgctg acacggccgg tgctgaaacc 1500

acccccggtc tttctactgt tgctgctggc gctgatgatg aggcctggtc taaatttatc 1560

tacagtaaat accgatcgaa ctaccacccg attaccacag ctggcatgct gcctaaggag 1620

cttggtggtg ttgttgatac ctcgctgaag gtttatggaa cctccaatgt ccgtgttgtg 1680

gatgcttccg tcatgccttt ccaggtctgc ggtcaccttc agagcaccgt gtatgcggtt 1740

gccgagcgcg cggccgatat catcaaggga gagttgtaa 1779

<210> 4
<211 > 592
<212> PRT
<213> Penicillium italicum

<400> 4

Met Arg Ser Leu Ile Gly Leu Ala Leu Leu Pro Leu Ala Val Ala Val
1 5 10 15

Pro His Ala Ser His Lys Ser Asp Ser Thr Tyr Asp Tyr Ile Ile Val
20 25 30

Gly Gly Gly Thr Ser Gly Leu Val Val Ala Asn Arg Leu Ser Glu Gln
35 40 45

Lys Asp Thr Thr Val Leu Val Ile Glu Ala Gly Gly Ser Val Tyr Asn
50 55 60

Asn Pro Asn Val Thr Asn Thr Leu Gly Tyr Gly Lys Ala Phe Gly Thr
65 70 75 80

Asp Ile Asp Trp Ala Tyr Glu Thr Thr Ala Gln Glu His Ala Gly Gly
85 90 95

Phe Pro Gln Ile Val Arg Ala Gly Lys Ala Leu Gly Gly Thr Ser Thr
100 105 110

Ile Asn Gly Met Ala Tyr Leu Arg Ala Gln Ala Ala Gln Ile Asp Ala
115 120 125

Trp Glu Thr Val Gly Asn Lys Gly Trp Asn Trp Lys Thr Leu Leu Pro
130 135 140

Tyr Phe Lys Lys Ser Glu Gln Phe Gln Asp Pro Ala Lys Tyr Pro Phe
145 150 155 160

Leu Asp Gly Ser Gly Val Ser Phe Asp Pro Ala Tyr His Gly Phe Thr
165 170 175

Gly Pro Leu Lys Val Gly Trp Ser Ser Thr Gln Leu Asn Asp Gly Leu
180 185 190

Ala Gln Lys Leu Asn Ala Thr Tyr Gln Ser Leu Asp Val Pro Val Pro
195 200 205

Tyr Asn Arg Asp Ala Asn Ser Gly Asp Met Val Gly Tyr Ser Val Tyr
210 215 220

Pro Lys Thr Val Asn Ala Asp Leu Asn Ile Arg Glu Asp Ala Ala Arg
225 230 235 240

Ala Phe Tyr Tyr Pro Tyr Gln Asn Arg Thr Asn Leu His Val Trp Leu
245 250 255

Asn Thr His Ala Asn Lys Ile Thr Trp Asn Glu Gly Ser Glu Ala Thr
260 265 270

Ala Asn Gly Val Glu Val Thr Leu Ser Asn Gly Lys Lys Thr Val Val
275 280 285

Lys Ala Thr Arg Glu Val Ile Leu Ala Ala Gly Ala Leu Lys Ser Pro
290 295 300

Val Leu Leu Glu Leu Ser Gly Val Gly Asn Pro Asp Ile Leu Ser Lys
305 310 315 320

His Gly Ile Thr Thr Lys Ile Asn Leu Pro Thr Val Gly Glu Asn Leu
325 330 335

Gln Asp Gln Met Asn Asn Gly Leu Lys Phe Glu Ser Lys Lys Thr Tyr
340 345 350

Ser Thr Asp Lys Gly Ser Ser Tyr Val Ala Tyr Pro Ser Ala Asp Gln
355 360 365

Leu Phe Pro Asn Ser Thr Ala Leu Gly Ala Asp Leu Leu Arg Lys Leu
370 375 380

Pro Ala Tyr Ala Ala Gln Val Ala Ser Ala Asn Gly Asn Ile Thr Lys
385 390 395 400

Ala Arg Asp Ile Tyr Arg Phe Phe Lys Ile Gln Trp Asp Leu Ile Phe
405 410 415

Lys Asp Glu Ile Pro Val Ala Glu Ile Leu Leu Ser Gly Ser Gly Ala
420 425 430

Ser Tyr Ser Gly Glu Tyr Trp Gly Ser Val Pro Phe Ser Arg Gly Ser
435 440 445

Val His Leu Ser Ser Ala Asp Pro Thr Ala Ala Pro Thr Ile Asp Pro
450 455 460

Lys Tyr Phe Met Leu Asp Phe Asp Leu His Ala Gln Ala Gln Ala Ala
465 470 475 480

Arg Phe Ile Arg Glu Ile Phe Lys Thr Glu Pro Leu Ala Asp Thr Ala
485 490 495

Gly Ala Glu Thr Thr Pro Gly Leu Ser Thr Val Ala Ala Gly Ala Asp
500 505 510

Asp Glu Ala Trp Ser Lys Phe Ile Tyr Ser Lys Tyr Arg Ser Asn Tyr
515 520 525

His Pro Ile Thr Thr Ala Gly Met Leu Pro Lys Glu Leu Gly Gly Val
530 535 540

Val Asp Thr Ser Leu Lys Val Tyr Gly Thr Ser Asn Val Arg Val Val
545 550 555 560

Asp Ala Ser Val Met Pro Phe Gln Val Cys Gly His Leu Gln Ser Thr
565 570 575

Val Tyr Ala Val Ala Glu Arg Ala Ala Asp Ile Ile Lys Gly Glu Leu
580 585 590

<210> 5
<211> 14
<212> PRT
<213> Artificial Sequence

<220>
<223> The sequence of peptide fragment described in example 12A

<400> 5

Ile Gly Gly Val Val Asp Thr Ser Leu Lys Val Tyr Gly Thr
1 5 10

<210> 6
<211> 20
<212> PRT
<213> Artificial Sequence

<220>
<223> The sequence of peptide fragment described in example 12A

<400> 6

Trp Gly Gly Gly Thr Lys Gln Thr Val Arg Ala Gly Lys Ala Leu Gly
1 5 10 15

Gly Thr Ser Thr
20

<210> 7
<211> 1923
<212> DNA
<213> Aspergillus oryzae

<400> 7

atgctcttct cactggcatt cctgagtgcc ctgtcgctgg ccacggcatc accggctgga 60

cgggccaaga acactacgac atacgactac atcgttgtgg gaggcggcac aagtggtctt 120

gtggtcgcaa atcgcctttc tgagaacccc gatgtctccg ttcttctgct tgaggccggt 180
gcttctgtgt tcaacaaccc ggacgtaacc aacgctaacg gttatggatt ggcctttggc 240
tcggccatcg actggcagta ccagtctatt aaccaaagct atgcaggagg taaacagcaa 300
gttctgcgtg ctggtaaggc ccttggagga accagtacaa tcaatggtat gcttctatgg 360
atgatctctt agtcggcatg gaccactgac gaccacagga atggcctata cccgcgcaga 420
ggatgtccag attgacgttt ggcagaaact tggaaacgaa ggttggacgt ggaaagatct 480
cctaccatac tacctgaaga gtgaaaactt gacggcccct accagctctc aggttgctgc 540
tggcgctgct tataaccctg ccgtgaatgg aaaagaaggt cctctcaagg tcggctggtc 600
gggaagcctg gcctccggta atctgtcagt tgctctgaac cgtacgttcc aagccatgga 660
ggatgtcaat ggaggcaaga tgcgtggctt caacatctac ccatccaccc tcgacgttga 720
cctcaatgtc cgcgaagatg cagcccgggc atactacttc ccttatgatg acaggaagaa 780
ccttcacctg ctggagaaca ccactgccaa ccgccttttc tggaagaacg gctctgctga 840
ggaagctatt gcggatggtg tcgagatcac ctccgctgat ggcaaggtca ctcgtgtgca 900
tgcaaagaaa gaggtcatca tctctgctgg tgccctgcgg tctcctctca ttctcgagct 960
ttcaggagtt ggaaacccaa cgtaagtgtt ccactgatgc cagcccctct ctatcaccgt 1020
ctctgaccct cgtagcatcc tcaaaaagaa caacataacc ccacgtgtcg atctccccac 1080
cgttggggag aacctccaag accagttcaa caacggcatg gctggcgaag gatacggcgt 1140
ccttgccggt gcctcaaccg tgacctaccc ttccatctcc gacgtcttcg gtaacgagac 1200
tgactctatc gttgcatctc tccgatctca actctccgac tacgccgccg cgaccgtcaa 1260
ggtcagcaac ggccacatga agcaggagga ccttgagcgc ctctaccagc tccaatttga 1320
cctcatcgtc aaggacaagg tccctatcgc cgagatcctc ttccaccccg gtggtggaaa 1380
cgccgtgtcc tccgaattct ggggcttgct tcccttcgcc cgtggcaaca tccacattag 1440
ctccaatgac ccgactgctc ccgccgccat caaccctaac tactttatgt tcgaatggga 1500
cggcaagagc caggccggta tcgccaagta catcaggaag attctccgca gcgcaccatt 1560
gaacaaactt attgcgaagg aaaccaagcc cggtctctct gagattccgg ccactgctgc 1620
ggatgagaag tgggttgaat ggctcaaggc taactgtaag ttgaatcctt tcttggcttc 1680
gatggtgagt ctgacgtgag ctctctagat cgttccaact tccaccccgt cggaactgct 1740
gccatgatgc ctcgttccat tggtggcgtt gttgataacc gtctccgggt ctatggtacc 1800

agcaatgttc gcgtcgtaga tgcgtctgtc ctgcccttcc aggtttgcgg ccacttggtt 1860

agcacgcttt atgccgttgc cgagcgcgct tccgacttga ttaaggagga tgcgaagagt 1920

gct 1923

<210> 8
<211> 1767
<212> DNA
<213> Aspergillus oryzae

<400> 8

```
atgctcttct cactggcatt cctgagtgcc ctgtcgctgg ccacggcatc accggctgga     60
cgggccaaga acactacgac atacgactac atcgttgtgg gaggcggcac aagtggtctt    120
gtggtcgcaa atcgcctttc tgagaacccc gatgtctccg ttcttctgct tgaggccggt    180
gcttctgtgt tcaacaaccc ggacgtaacc aacgctaacg gttatggatt ggcctttggc    240
tcggccatcg actggcagta ccagtctatt aaccaaagct atgcaggagg taaacagcaa    300
gttctgcgtg ctggtaaggc ccttggagga accagtacaa tcaatggaat ggcctatacc    360
cgcgcagagg atgtccagat tgacgtttgg cagaaacttg gaaacgaagg ttggacgtgg    420
aaagatctcc taccatacta cctgaagagt gaaaacttga cggcccctac cagctctcag    480
gttgctgctg gcgctgctta taaccctgcc gtgaatggaa aagaaggtcc tctcaaggtc    540
ggctggtcgg gaagcctggc ctccggtaat ctgtcagttg ctctgaaccg tacgttccaa    600
gccatggagg atgtcaatgg aggcaagatg cgtggcttca acatctaccc atccaccctc    660
gacgttgacc tcaatgtccg cgaagatgca gcccgggcat actacttccc ttatgatgac    720
aggaagaacc ttcacctgct ggagaacacc actgccaacc gccttttctg gaagaacggc    780
tctgctgagg aagctattgc ggatggtgtc gagatcacct ccgctgatgg caaggtcact    840
cgtgtgcatg caaagaaaga ggtcatcatc tctgctggtg ccctgcggtc tcctctcatt    900
ctcgagcttt caggagttgg aaacccaacc atcctcaaaa agaacaacat aaccccacgt    960
gtcgatctcc ccaccgttgg ggagaacctc caagaccagt tcaacaacgg catggctggc   1020
gaaggatacg gcgtccttgc cggtgcctca accgtgacct acccttccat ctccgacgtc   1080
ttcggtaacg agactgactc tatcgttgca tctctccgat ctcaactctc cgactacgcc   1140
gccgcgaccg tcaaggtcag caacggccac atgaagcagg aggaccttga gcgcctctac   1200
cagctccaat ttgacctcat cgtcaaggac aaggtcccta tcgccgagat cctcttccac   1260
cccggtggtg gaaacgccgt gtcctccgaa ttctggggct tgcttccctt cgcccgtggc   1320
```

aacatccaca ttagctccaa tgacccgact gctcccgccg ccatcaaccc taactacttt 1380

atgttcgaat gggacggcaa gagccaggcc ggtatcgcca agtacatcag gaagattctc 1440

cgcagcgcac cattgaacaa acttattgcg aaggaaacca agcccggtct ctctgagatt 1500

ccggccactg ctgcggatga gaagtgggtt gaatggctca aggctaacta tcgttccaac 1560

ttccaccccg tcggaactgc tgccatgatg cctcgttcca ttggtggcgt tgttgataac 1620

cgtctccggg tctatggtac cagcaatgtt cgcgtcgtag atgcgtctgt cctgcccttc 1680

caggtttgcg gccacttggt tagcacgctt tatgccgttg ccgagcgcgc ttccgacttg 1740

attaaggagg atgcgaagag tgcttag 1767

<210> 9
<211> 588
<212> PRT
<213> Aspergillus oryzae

<400> 9

Met Leu Phe Ser Leu Ala Phe Leu Ser Ala Leu Ser Leu Ala Thr Ala
1 5 10 15

Ser Pro Ala Gly Arg Ala Lys Asn Thr Thr Thr Tyr Asp Tyr Ile Val
20 25 30

Val Gly Gly Gly Thr Ser Gly Leu Val Val Ala Asn Arg Leu Ser Glu
35 40 45

Asn Pro Asp Val Ser Val Leu Leu Leu Glu Ala Gly Ala Ser Val Phe
50 55 60

Asn Asn Pro Asp Val Thr Asn Ala Asn Gly Tyr Gly Leu Ala Phe Gly
65 70 75 80

Ser Ala Ile Asp Trp Gln Tyr Gln Ser Ile Asn Gln Ser Tyr Ala Gly
85 90 95

Gly Lys Gln Gln Val Leu Arg Ala Gly Lys Ala Leu Gly Gly Thr Ser
100 105 110

Thr Ile Asn Gly Met Ala Tyr Thr Arg Ala Glu Asp Val Gln Ile Asp
115 120 125

Val Trp Gln Lys Leu Gly Asn Glu Gly Trp Thr Trp Lys Asp Leu Leu

130 135 140

Pro Tyr Tyr Leu Lys Ser Glu Asn Leu Thr Ala Pro Thr Ser Ser Gln
145 150 155 160

Val Ala Ala Gly Ala Ala Tyr Asn Pro Ala Val Asn Gly Lys Glu Gly
165 170 175

Pro Leu Lys Val Gly Trp Ser Gly Ser Leu Ala Ser Gly Asn Leu Ser
180 185 190

Val Ala Leu Asn Arg Thr Phe Gln Ala Met Glu Asp Val Asn Gly Gly
195 200 205

Lys Met Arg Gly Phe Asn Ile Tyr Pro Ser Thr Leu Asp Val Asp Leu
210 215 220

Asn Val Arg Glu Asp Ala Ala Arg Ala Tyr Tyr Phe Pro Tyr Asp Asp
225 230 235 240

Arg Lys Asn Leu His Leu Leu Glu Asn Thr Thr Ala Asn Arg Leu Phe
245 250 255

Trp Lys Asn Gly Ser Ala Glu Glu Ala Ile Ala Asp Gly Val Glu Ile
260 265 270

Thr Ser Ala Asp Gly Lys Val Thr Arg Val His Ala Lys Lys Glu Val
275 280 285

Ile Ile Ser Ala Gly Ala Leu Arg Ser Pro Leu Ile Leu Glu Leu Ser
290 295 300

Gly Val Gly Asn Pro Thr Ile Leu Lys Lys Asn Asn Ile Thr Pro Arg
305 310 315 320

Val Asp Leu Pro Thr Val Gly Glu Asn Leu Gln Asp Gln Phe Asn Asn
325 330 335

Gly Met Ala Gly Glu Gly Tyr Gly Val Leu Ala Gly Ala Ser Thr Val
340 345 350

Thr Tyr Pro Ser Ile Ser Asp Val Phe Gly Asn Glu Thr Asp Ser Ile
355 360 365

Val Ala Ser Leu Arg Ser Gln Leu Ser Asp Tyr Ala Ala Ala Thr Val
370 375 380

Lys Val Ser Asn Gly His Met Lys Gln Glu Asp Leu Glu Arg Leu Tyr
385 390 395 400

Gln Leu Gln Phe Asp Leu Ile Val Lys Asp Lys Val Pro Ile Ala Glu
405 410 415

Ile Leu Phe His Pro Gly Gly Gly Asn Ala Val Ser Ser Glu Phe Trp
420 425 430

Gly Leu Leu Pro Phe Ala Arg Gly Asn Ile His Ile Ser Ser Asn Asp
435 440 445

Pro Thr Ala Pro Ala Ala Ile Asn Pro Asn Tyr Phe Met Phe Glu Trp
450 455 460

Asp Gly Lys Ser Gln Ala Gly Ile Ala Lys Tyr Ile Arg Lys Ile Leu
465 470 475 480

Arg Ser Ala Pro Leu Asn Lys Leu Ile Ala Lys Glu Thr Lys Pro Gly
485 490 495

Leu Ser Glu Ile Pro Ala Thr Ala Ala Asp Glu Lys Trp Val Glu Trp
500 505 510

Leu Lys Ala Asn Tyr Arg Ser Asn Phe His Pro Val Gly Thr Ala Ala
515 520 525

Met Met Pro Arg Ser Ile Gly Gly Val Val Asp Asn Arg Leu Arg Val
530 535 540

Tyr Gly Thr Ser Asn Val Arg Val Val Asp Ala Ser Val Leu Pro Phe
545 550 555 560

Gln Val Cys Gly His Leu Val Ser Thr Leu Tyr Ala Val Ala Glu Arg
565 570 575

Ala Ser Asp Leu Ile Lys Glu Asp Ala Lys Ser Ala
580 585

<210> 10

<211> 593
<212> PRT
<213> Aspergillus oryzae

<400> 10

Met Leu Phe Ser Leu Ala Phe Leu Ser Ala Leu Ser Leu Ala Thr Ala
1               5                   10                  15

Ser Pro Ala Gly Arg Ala Lys Asn Thr Thr Thr Tyr Asp Tyr Ile Val
            20                  25                  30

Val Gly Gly Gly Thr Ser Gly Leu Val Val Ala Asn Arg Leu Ser Glu
        35                  40                  45

Asn Pro Asp Val Ser Val Leu Leu Leu Glu Ala Gly Ala Ser Val Phe
    50                  55                  60

Asn Asn Pro Asp Val Thr Asn Ala Asn Gly Tyr Gly Leu Ala Phe Gly
65                  70                  75                  80

Ser Ala Ile Asp Trp Gln Tyr Gln Ser Ile Asn Gln Ser Tyr Ala Gly
                85                  90                  95

Gly Lys Gln Gln Val Leu Arg Ala Gly Lys Ala Leu Gly Gly Thr Ser
            100                 105                 110

Thr Ile Asn Gly Met Ala Tyr Thr Arg Ala Glu Asp Val Gln Ile Asp
        115                 120                 125

Val Trp Gln Lys Leu Gly Asn Glu Gly Trp Thr Trp Lys Asp Leu Leu
    130                 135                 140

Pro Tyr Tyr Leu Lys Ser Glu Asn Leu Thr Ala Pro Thr Ser Ser Gln
145                 150                 155                 160

Val Ala Ala Gly Ala Ala Tyr Asn Pro Ala Val Asn Gly Lys Glu Gly
                165                 170                 175

Pro Leu Lys Val Gly Trp Ser Gly Ser Leu Ala Ser Gly Asn Leu Ser
            180                 185                 190

Val Ala Leu Asn Arg Thr Phe Gln Ala Ala Gly Val Pro Trp Val Glu

195 200 205

Asp Val Asn Gly Gly Lys Met Arg Gly Phe Asn Ile Tyr Pro Ser Thr
210 215 220

Leu Asp Val Asp Leu Asn Val Arg Glu Asp Ala Ala Arg Ala Tyr Tyr
225 230 235 240

Phe Pro Tyr Asp Asp Arg Lys Asn Leu His Leu Leu Glu Asn Thr Thr
245 250 255

Ala Asn Arg Leu Phe Trp Lys Asn Gly Ser Ala Glu Glu Ala Ile Ala
260 265 270

Asp Gly Val Glu Ile Thr Ser Ala Asp Gly Lys Val Thr Arg Val His
275 280 285

Ala Lys Lys Glu Val Ile Ile Ser Ala Gly Ala Leu Arg Ser Pro Leu
290 295 300

Ile Leu Glu Leu Ser Gly Val Gly Asn Pro Thr Ile Leu Lys Lys Asn
305 310 315 320

Asn Ile Thr Pro Arg Val Asp Leu Pro Thr Val Gly Glu Asn Leu Gln
325 330 335

Asp Gln Phe Asn Asn Gly Met Ala Gly Glu Gly Tyr Gly Val Leu Ala
340 345 350

Gly Ala Ser Thr Val Thr Tyr Pro Ser Ile Ser Asp Val Phe Gly Asn
355 360 365

Glu Thr Asp Ser Ile Val Ala Ser Leu Arg Ser Gln Leu Ser Asp Tyr
370 375 380

Ala Ala Ala Thr Val Lys Val Ser Asn Gly His Met Lys Gln Glu Asp
385 390 395 400

Leu Glu Arg Leu Tyr Gln Leu Gln Phe Asp Leu Ile Val Lys Asp Lys
405 410 415

Val Pro Ile Ala Glu Ile Leu Phe His Pro Gly Gly Gly Asn Ala Val
420 425 430

Ser Ser Glu Phe Trp Gly Leu Leu Pro Phe Ala Arg Gly Asn Ile His
435 440 445

Ile Ser Ser Asn Asp Pro Thr Ala Pro Ala Ala Ile Asn Pro Asn Tyr
450 455 460

Phe Met Phe Glu Trp Asp Gly Lys Ser Gln Ala Gly Ile Ala Lys Tyr
465 470 475 480

Ile Arg Lys Ile Leu Arg Ser Ala Pro Leu Asn Lys Leu Ile Ala Lys
485 490 495

Glu Thr Lys Pro Gly Leu Ser Glu Ile Pro Ala Thr Ala Ala Asp Glu
500 505 510

Lys Trp Val Glu Trp Leu Lys Ala Asn Tyr Arg Ser Asn Phe His Pro
515 520 525

Val Gly Thr Ala Ala Met Met Pro Arg Ser Ile Gly Gly Val Val Asp
530 535 540

Asn Arg Leu Arg Val Tyr Gly Thr Ser Asn Val Arg Val Val Asp Ala
545 550 555 560

Ser Val Leu Pro Phe Gln Val Cys Gly His Leu Val Ser Thr Leu Tyr
565 570 575

Ala Val Ala Glu Arg Ala Ser Asp Leu Ile Lys Glu Asp Ala Lys Ser
580 585 590

Ala

<210> 11
<211> 1779
<212> DNA
<213> Aspergillus oryzae

<400> 11

atgctcttct cactggcatt cctgagtgcc ctgtcgctgg ccacggcatc accggctgga 60
cgggccaaga acactacgac atacgactac atcgttgtgg gaggcggcac aagtggtctt 120
gtggtcgcaa atcgcctttc tgagaacccc gatgtctccg ttcttctgct tgaggccggt 180

gcttctgtgt tcaacaaccc ggacgtaacc aacgctaacg gttatggatt ggcctttggc 240
tcggccatcg actggcagta ccagtctatt aaccaaagct atgcaggagg taaacagcaa 300
gttctgcgtg ctggtaaggc ccttggagga accagtacaa tcaatggaat ggcctatacc 360
cgcgcagagg atgtccagat tgacgtttgg cagaaacttg gaaacgaagg ttggacgtgg 420
aaagatctcc taccatacta cctgaagagt gaaaacttga cggcccctac cagctctcag 480
gttgctgctg gcgctgctta taaccctgcc gtgaatggaa aagaaggtcc tctcaaggtc 540
ggctggtcgg gaagcctggc ctccggtaat ctgtcagttg ctctgaaccg tacgttccaa 600
gccgctggtg ttccatgggt tgaggatgtc aatggaggca agatgcgtgg cttcaacatc 660
tacccatcca ccctcgacgt tgacctcaat gtccgcgaag atgcagcccg ggcatactac 720
ttcccttatg atgacaggaa gaaccttcac ctgctggaga acaccactgc caaccgcctt 780
ttctggaaga acggctctgc tgaggaagct attgcggatg gtgtcgagat cacctccgct 840
gatggcaagg tcactcgtgt gcatgcaaag aaagaggtca tcatctctgc tggtgccctg 900
cggtctcctc tcattctcga gctttcagga gttggaaacc caaccatcct caaaaagaac 960
aacataaccc cacgtgtcga tctccccacc gttggggaga acctccaaga ccagttcaac 1020
aacggcatgg ctggcgaagg atacggcgtc cttgccggtg cctcaaccgt gacctaccct 1080
tccatctccg acgtcttcgg taacgagact gactctatcg ttgcatctct ccgatctcaa 1140
ctctccgact acgccgccgc gaccgtcaag gtcagcaacg gccacatgaa gcaggaggac 1200
cttgagcgcc tctaccagct ccaatttgac ctcatcgtca aggacaaggt ccctatcgcc 1260
gagatcctct tccaccccgg tggtggaaac gccgtgtcct ccgaattctg ggcttgctt 1320
cccttcgccc gtggcaacat ccacattagc tccaatgacc cgactgctcc cgccgccatc 1380
aaccctaact actttatgtt cgaatgggac ggcaagagcc aggccggtat cgccaagtac 1440
atcaggaaga ttctccgcag cgcaccattg aacaaactta ttgcgaagga aaccaagccc 1500
ggtctctctg agattccggc cactgctgcg gatgagaagt gggttgaatg gctcaaggct 1560
aactatcgtt ccaacttcca ccccgtcgga actgctgcca tgatgcctcg ttccattggt 1620
ggcgttgttg ataaccgtct ccgggtctat ggtaccagca atgttcgcgt cgtagatgcg 1680
tctgtcctgc ccttccaggt ttgcggccac ttggttagca cgctttatgc cgttgccgag 1740
cgcgcttccg acttgattaa ggaggatgcg aagagtgct 1779

<210> 12
<211> 34

<212> DNA
<213> Artificial Sequence

<220>
<223> The sequence of designed polynucleotide described in Example 1B

<400> 12
ggaattccat atgctcttct cactggcatt cctg 34

<210> 13
<211> 35
<212> DNA
<213> Artificial Sequence

<220>
<223> The sequence of designed polynucleotide described in Example 1B

<400> 13
cgggatccga attggtacgg gacactgtcc ctacg 35

<210> 14
<211> 1938
<212> DNA
<213> Aspergillus oryzae

<400> 14

atgctcttct cactggcatt cctgagtgcc ctgtcgctgg ccacggcatc accggctgga 60
cgggccaaga acactacgac atacgactac atcgttgtgg gaggcggcac aagtggtctt 120
gtggtcgcaa atcgcctttc tgagaacccc gatgtctccg ttcttctgct tgaggccggt 180
gcttctgtgt tcaacaaccc ggacgtaacc aacgctaacg gttatggatt ggcctttggc 240
tcggccatcg actggcagta ccagtctatt aaccaaagct atgcaggagg taaacagcaa 300
gttctgcgtg ctggtaaggc ccttggagga accagtacaa tcaatggtat gcttctatgg 360
atgatctctt agtcggcatg gaccactgac gaccacagga atggcctata cccgcgcaga 420
ggatgtccag attgacgttt ggcagaaact tggaaacgaa ggttggacgt ggaaagatct 480
cctaccatac tacctgaaga gtgaaaactt gacggcccct accagctctc aggttgctgc 540
tggcgctgct tataaccctg ccgtgaatgg aaaagaaggt cctctcaagg tcggctggtc 600
gggaagcctg gcctccggta atctgtcagt tgctctgaac cgtacgttcc aagccgctgg 660
tgttccatgg gttgaggatg tcaatggagg caagatgcgt ggcttcaaca tctacccatc 720
caccctcgac gttgacctca atgtccgcga agatgcagcc cgggcatact acttccctta 780
tgatgacagg aagaaccttc acctgctgga gaacaccact gccaaccgcc ttttctggaa 840

gaacggctct gctgaggaag ctattgcgga tggtgtcgag atcacctccg ctgatggcaa 900
ggtcactcgt gtgcatgcaa agaaagaggt catcatctct gctggtgccc tgcggtctcc 960
tctcattctc gagctttcag gagttggaaa cccaacgtaa gtgttccact gatgccagcc 1020
cctctctatc accgtctctg accctcgtag catcctcaaa aagaacaaca taaccccacg 1080
tgtcgatctc cccaccgttg gggagaacct ccaagaccag ttcaacaacg gcatggctgg 1140
cgaaggatac ggcgtccttg ccggtgcctc aaccgtgacc tacccttcca tctccgacgt 1200
cttcggtaac gagactgact ctatcgttgc atctctccga tctcaactct ccgactacgc 1260
cgccgcgacc gtcaaggtca gcaacggcca catgaagcag gaggaccttg agcgcctcta 1320
ccagctccaa tttgacctca tcgtcaagga caaggtccct atcgccgaga tcctcttcca 1380
ccccggtggt ggaaacgccg tgtcctccga attctggggc ttgcttccct tcgcccgtgg 1440
caacatccac attagctcca atgacccgac tgctcccgcc gccatcaacc ctaactactt 1500
tatgttcgaa tgggacggca agagccaggc cggtatcgcc aagtacatca ggaagattct 1560
ccgcagcgca ccattgaaca aacttattgc gaaggaaacc aagcccggtc tctctgagat 1620
tccggccact gctgcggatg agaagtgggt tgaatggctc aaggctaact gtaagttgaa 1680
tcctttcttg gcttcgatgg tgagtctgac gtgagctctc tagatcgttc caacttccac 1740
cccgtcggaa ctgctgccat gatgcctcgt tccattggtg gcgttgttga taaccgtctc 1800
cgggtctatg gtaccagcaa tgttcgcgtc gtagatgcgt ctgtcctgcc cttccaggtt 1860
tgcggccact tggttagcac gctttatgcc gttgccgagc gcgcttccga cttgattaag 1920
gaggatgcga agagtgct 1938

<210> 15
<211> 37
<212> DNA
<213> Artificial sequence

<220>
<223> The sequence of designed polinucleotide described in Example 1C

<400> 15
ggaattccat atgaagaaca ctacgacata cgactac 37

<210> 16
<211> 37
<212> DNA
<213> Artificial sequence

<220>
<223> The sequence of designed polinucleotide described in Example 1C

<400> 16
cacacaggaa acacatatga acactacgac atacgac 37

<210> 17
<211> 1719
<212> DNA
<213> Aspergillus oryzae mutant

<400> 17

atgaagaaca ctacgacata cgactacatc gttgtgggag gcggcacaag tggtcttgtg 60
gtcgcaaatc gcctttctga gaacccgat gtctccgttc ttctgcttga ggccggtgct 120
tctgtgttca acaacccgga cgtaaccaac gctaacggtt atggattggc ctttggctcg 180
gccatcgact ggcagtacca gtctattaac caaagctatg caggaggtaa acagcaagtt 240
ctgcgtgctg gtaaggccct tggaggaacc agtacaatca atggaatggc ctatacccgc 300
gcagaggatg tccagattga cgtttggcag aaacttggaa acgaaggttg gacgtggaaa 360
gatctcctac catactacct gaagagtgaa aacttgacgg cccctaccag ctctcaggtt 420
gctgctggcg ctgcttataa ccctgccgtg aatggaaaag aaggtcctct caaggtcggc 480
tggtcgagga gcctggcctc cggtaatctg tcagttgctc tgaaccgtac gttccaagcc 540
gctggtgttc catgggttga ggatgtcaat ggaggcaaga tgcgtggctt caacatctac 600
ccatccaccc tcgacgttga cctcaatgtc cgcgaagatg cagcccgggc atactacttc 660
ccttatgatg acaggaagaa ccttcacctg ctggagaaca ccactgccaa ccgccttttc 720
tggaagaacg gctctgctga ggaagctatt gcggatggtg tcgagatcac ctccgctgat 780
ggcaaggtca ctcgtgtgca tgcaaagaaa gaggtcatca tctctgctgg tgccctgcgg 840
tctcctctca ttctcgagct ttcaggagtt ggaaacccaa ccatcctcaa aaagaacaac 900
ataaccccac gtgtcgatct ccccaccgtt ggggagaacc tccaagacca gttcaacaac 960
ggcatggctg gcgaaggata cggcgtcctt gccggtgcct caaccgtgac ctacccttcc 1020
atctccgacg tcttcggtaa cgagactgac tctatcgttg catctctccg atctcaactc 1080
tccgactacg ccgccgcgac cgtcaaggtc agcaacggcc acatgaagca ggaggacctt 1140
gagcgcctct accagctcca atttgacctc atcgtcaagg acaaggtccc tatcgccgag 1200
atcctcttcc accccggtgg tggaaacgcc gtgtcctccg aattctgggg cttgcttccc 1260
ttcgcccgtg gcaacatcca cattagctcc aatgacccga ctgctcccgc cgccatcaac 1320
cctaactact ttatgttcga atgggacggc aagagccagg ccggtatcgc caagtacatc 1380

```
aggaagattc tccgcagcgc accattgaac aaacttattg cgaaggaaac caagcccggt    1440
ctctctgaga ttccggccac tgctgcggat gagaagtggg ttgaatggct caaggctaac    1500
tatcgttcca acttccaccc cgtcggaact gctgccatga tgcctcgttc cattggtggc    1560
gttgttgata accgtctccg ggtctatggt accagcaatg ttcgcgtcgt agatgcgtct    1620
gtcctgccct tccaggtttg cggccacttg tgcagcacgc tttatgccgt tgccgagcgc    1680
gcttccgact tgattaagga ggatgcgaag agtgcttag                           1719
```

<210> 18
<211> 572
<212> PRT
<213> Aspergillus oryzae mutant

<400> 18

```
Met Lys Asn Thr Thr Thr Tyr Asp Tyr Ile Val Val Gly Gly Gly Thr
1               5                   10                  15

Ser Gly Leu Val Val Ala Asn Arg Leu Ser Glu Asn Pro Asp Val Ser
            20                  25                  30

Val Leu Leu Leu Glu Ala Gly Ala Ser Val Phe Asn Asn Pro Asp Val
        35                  40                  45

Thr Asn Ala Asn Gly Tyr Gly Leu Ala Phe Gly Ser Ala Ile Asp Trp
    50                  55                  60

Gln Tyr Gln Ser Ile Asn Gln Ser Tyr Ala Gly Gly Lys Gln Gln Val
65                  70                  75                  80

Leu Arg Ala Gly Lys Ala Leu Gly Gly Thr Ser Thr Ile Asn Gly Met
                85                  90                  95

Ala Tyr Thr Arg Ala Glu Asp Val Gln Ile Asp Val Trp Gln Lys Leu
            100                 105                 110

Gly Asn Glu Gly Trp Thr Trp Lys Asp Leu Leu Pro Tyr Tyr Leu Lys
        115                 120                 125

Ser Glu Asn Leu Thr Ala Pro Thr Ser Ser Gln Val Ala Ala Gly Ala
    130                 135                 140
```

Ala Tyr Asn Pro Ala Val Asn Gly Lys Glu Gly Pro Leu Lys Val Gly
145 150 155 160

Trp Ser Arg Ser Leu Ala Ser Gly Asn Leu Ser Val Ala Leu Asn Arg
165 170 175

Thr Phe Gln Ala Ala Gly Val Pro Trp Val Glu Asp Val Asn Gly Gly
180 185 190

Lys Met Arg Gly Phe Asn Ile Tyr Pro Ser Thr Leu Asp Val Asp Leu
195 200 205

Asn Val Arg Glu Asp Ala Ala Arg Ala Tyr Tyr Phe Pro Tyr Asp Asp
210 215 220

Arg Lys Asn Leu His Leu Leu Glu Asn Thr Thr Ala Asn Arg Leu Phe
225 230 235 240

Trp Lys Asn Gly Ser Ala Glu Glu Ala Ile Ala Asp Gly Val Glu Ile
245 250 255

Thr Ser Ala Asp Gly Lys Val Thr Arg Val His Ala Lys Lys Glu Val
260 265 270

Ile Ile Ser Ala Gly Ala Leu Arg Ser Pro Leu Ile Leu Glu Leu Ser
275 280 285

Gly Val Gly Asn Pro Thr Ile Leu Lys Lys Asn Asn Ile Thr Pro Arg
290 295 300

Val Asp Leu Pro Thr Val Gly Glu Asn Leu Gln Asp Gln Phe Asn Asn
305 310 315 320

Gly Met Ala Gly Glu Gly Tyr Gly Val Leu Ala Gly Ala Ser Thr Val
325 330 335

Thr Tyr Pro Ser Ile Ser Asp Val Phe Gly Asn Glu Thr Asp Ser Ile
340 345 350

Val Ala Ser Leu Arg Ser Gln Leu Ser Asp Tyr Ala Ala Ala Thr Val
355 360 365

Lys Val Ser Asn Gly His Met Lys Gln Glu Asp Leu Glu Arg Leu Tyr

370 375 380

Gln Leu Gln Phe Asp Leu Ile Val Lys Asp Lys Val Pro Ile Ala Glu
385 390 395 400

Ile Leu Phe His Pro Gly Gly Gly Asn Ala Val Ser Ser Glu Phe Trp
405 410 415

Gly Leu Leu Pro Phe Ala Arg Gly Asn Ile His Ile Ser Ser Asn Asp
420 425 430

Pro Thr Ala Pro Ala Ala Ile Asn Pro Asn Tyr Phe Met Phe Glu Trp
435 440 445

Asp Gly Lys Ser Gln Ala Gly Ile Ala Lys Tyr Ile Arg Lys Ile Leu
450 455 460

Arg Ser Ala Pro Leu Asn Lys Leu Ile Ala Lys Glu Thr Lys Pro Gly
465 470 475 480

Leu Ser Glu Ile Pro Ala Thr Ala Ala Asp Glu Lys Trp Val Glu Trp
485 490 495

Leu Lys Ala Asn Tyr Arg Ser Asn Phe His Pro Val Gly Thr Ala Ala
500 505 510

Met Met Pro Arg Ser Ile Gly Gly Val Val Asp Asn Arg Leu Arg Val
515 520 525

Tyr Gly Thr Ser Asn Val Arg Val Val Asp Ala Ser Val Leu Pro Phe
530 535 540

Gln Val Cys Gly His Leu Cys Ser Thr Leu Tyr Ala Val Ala Glu Arg
545 550 555 560

Ala Ser Asp Leu Ile Lys Glu Asp Ala Lys Ser Ala
565 570

<210> 19
<211> 1704
<212> DNA
<213> Aspergillus terreus

<400> 19

atgaaatatg attatatcgt tattggaggc ggtaccagcg gtttggccgt cgcaaaccgt 60

ctatcggagg acccaagcgt gaacgtactc attctggagg ccggtggctc ggtctggaac 120

aatcccaatg tcacaaacgt gaatggctat gggcttgcat ttgggtctga cattgactgg 180

caataccagt ccgtcaacca gccatatgga ggcaacgtca gtcaagtgct gcgtgccggc 240

aaggcccttg gtggtactag tactattaac ggtatggcct atacccgcgc cgaggatgtc 300

cagatcgacg cctgggaaac cattggcaac acaggatgga cgtggaagaa tctgttccct 360

tactatcgga agagcgagaa cttcactgtc cctaccaaat cgcagacttc tcttggagcg 420

tcgtatgaag ctggagccca cggccacgag ggtccccttg acgttgcctt cactcagatc 480

gagtcgaaca acctgaccac ctacctcaac cgtaccttcc agggcatggg actcccatgg 540

actgaggacg tcaatggcgg aaagatgcgc ggctttaacc tataccccctc caccgtgaat 600

cttgaggagt atgttcgcga agacgccgct cgtgcatact actggcctta caagtcccgt 660

cccaacctgc atgtcctgct caacactttt gccaaccgga ttgtgtggga cggcgaagcc 720

cgtgatggcg acatcactgc cagtggtgtc gagatcactt ccaggaacgg cactgttcgt 780

gttatcaatg cggagaagga agtcattgtc tctgccggcg ccttgaagtc cccggctatc 840

cttgaacttt ccggaattgg caaccctagc gttcttgaca agtacaacat ccccgtcaag 900

gtcaacctcc ctactgtagg tgagaacctt caggaccagg tgaacagcca catggatgcg 960

tcgggcaaca cttccatctc tggaaccaag gcagtctctt accccgatgt ctatgacgtc 1020

ttcggtgacg aagccgagtc ggtcgccaaa cagatccgtg ccagcctgaa gcaatacgcc 1080

gccgacaccg cccaggccaa cggaaacatc atgaaggccg ccgatctgga gcgtctcttc 1140

gaggtccagt atgaccttat tttcaagggc agagtcccaa ttgcagaagt cctcaactat 1200

cctggcagcg cgacgtccgt gtttgcagaa ttctgggccc tccttccctt cgctcgggga 1260

agtgttcaca tcggttcttc aaacccggtc gagtttcctg tcatcaaccc caactatttc 1320

atgctcgact gggacgcgaa gagctacgtc gccgttgcaa agtatatccg ccgctcgttc 1380

gagagctacc ctctcagcag catcgttaag gagtctaccc ctggctatga tgttatcccc 1440

cggaacgctt ctgaacagag ctggaaagaa tgggtctttg ataagaacta tcgttctaac 1500

ttccatcccg tcggcacggc tgccatgatg cctcgtgaaa ttggcggtgt cgtggacgag 1560

cgtctgaatg tctatggtac tacgaacgtc agagttgtcg atgcctcggt gcttccgttc 1620

caggtctgcg gtcatttggt gagcacccta tacgctgtgg ccgaacgggc agcggatctc 1680

atcaaggccg atgctggtcg tcgt 1704

<210> 20
<211> 568
<212> PRT
<213> Aspergillus terreus

<400> 20

Met Lys Tyr Asp Tyr Ile Val Ile Gly Gly Gly Thr Ser Gly Leu Ala
1               5                   10                  15

Val Ala Asn Arg Leu Ser Glu Asp Pro Ser Val Asn Val Leu Ile Leu
            20                  25                  30

Glu Ala Gly Gly Ser Val Trp Asn Asn Pro Asn Val Thr Asn Val Asn
        35                  40                  45

Gly Tyr Gly Leu Ala Phe Gly Ser Asp Ile Asp Trp Gln Tyr Gln Ser
    50                  55                  60

Val Asn Gln Pro Tyr Gly Gly Asn Val Ser Gln Val Leu Arg Ala Gly
65                  70                  75                  80

Lys Ala Leu Gly Gly Thr Ser Thr Ile Asn Gly Met Ala Tyr Thr Arg
                85                  90                  95

Ala Glu Asp Val Gln Ile Asp Ala Trp Glu Thr Ile Gly Asn Thr Gly
            100                 105                 110

Trp Thr Trp Lys Asn Leu Phe Pro Tyr Tyr Arg Lys Ser Glu Asn Phe
        115                 120                 125

Thr Val Pro Thr Lys Ser Gln Thr Ser Leu Gly Ala Ser Tyr Glu Ala
    130                 135                 140

Gly Ala His Gly His Glu Gly Pro Leu Asp Val Ala Phe Thr Gln Ile
145                 150                 155                 160

Glu Ser Asn Asn Leu Thr Thr Tyr Leu Asn Arg Thr Phe Gln Gly Met
                165                 170                 175

Gly Leu Pro Trp Thr Glu Asp Val Asn Gly Gly Lys Met Arg Gly Phe
            180                 185                 190

Asn Leu Tyr Pro Ser Thr Val Asn Leu Glu Glu Tyr Val Arg Glu Asp
195 200 205

Ala Ala Arg Ala Tyr Tyr Trp Pro Tyr Lys Ser Arg Pro Asn Leu His
210 215 220

Val Leu Leu Asn Thr Phe Ala Asn Arg Ile Val Trp Asp Gly Glu Ala
225 230 235 240

Arg Asp Gly Asp Ile Thr Ala Ser Gly Val Glu Ile Thr Ser Arg Asn
245 250 255

Gly Thr Val Arg Val Ile Asn Ala Glu Lys Glu Val Ile Val Ser Ala
260 265 270

Gly Ala Leu Lys Ser Pro Ala Ile Leu Glu Leu Ser Gly Ile Gly Asn
275 280 285

Pro Ser Val Leu Asp Lys Tyr Asn Ile Pro Val Lys Val Asn Leu Pro
290 295 300

Thr Val Gly Glu Asn Leu Gln Asp Gln Val Asn Ser His Met Asp Ala
305 310 315 320

Ser Gly Asn Thr Ser Ile Ser Gly Thr Lys Ala Val Ser Tyr Pro Asp
325 330 335

Val Tyr Asp Val Phe Gly Asp Glu Ala Glu Ser Val Ala Lys Gln Ile
340 345 350

Arg Ala Ser Leu Lys Gln Tyr Ala Ala Asp Thr Ala Gln Ala Asn Gly
355 360 365

Asn Ile Met Lys Ala Ala Asp Leu Glu Arg Leu Phe Glu Val Gln Tyr
370 375 380

Asp Leu Ile Phe Lys Gly Arg Val Pro Ile Ala Glu Val Leu Asn Tyr
385 390 395 400

Pro Gly Ser Ala Thr Ser Val Phe Ala Glu Phe Trp Ala Leu Leu Pro
405 410 415

Phe Ala Arg Gly Ser Val His Ile Gly Ser Ser Asn Pro Val Glu Phe

420 425 430

Pro Val Ile Asn Pro Asn Tyr Phe Met Leu Asp Trp Asp Ala Lys Ser
435 440 445

Tyr Val Ala Val Ala Lys Tyr Ile Arg Arg Ser Phe Glu Ser Tyr Pro
450 455 460

Leu Ser Ser Ile Val Lys Glu Ser Thr Pro Gly Tyr Asp Val Ile Pro
465 470 475 480

Arg Asn Ala Ser Glu Gln Ser Trp Lys Glu Trp Val Phe Asp Lys Asn
485 490 495

Tyr Arg Ser Asn Phe His Pro Val Gly Thr Ala Ala Met Met Pro Arg
500 505 510

Glu Ile Gly Gly Val Val Asp Glu Arg Leu Asn Val Tyr Gly Thr Thr
515 520 525

Asn Val Arg Val Val Asp Ala Ser Val Leu Pro Phe Gln Val Cys Gly
530 535 540

His Leu Val Ser Thr Leu Tyr Ala Val Ala Glu Arg Ala Ala Asp Leu
545 550 555 560

Ile Lys Ala Asp Ala Gly Arg Arg
565

<210> 21
<211> 36
<212> DNA
<213> Artificial Sequence

<220>
<223> The sequence of designed polynucleotide described in Example 1C

<400> 21
ggaattccat atgaaatatg attatatcgt tattgg 36

<210> 22
<211> 32
<212> DNA
<213> Artificial Sequence

<220>
<223> The sequence of designed polynucleotide described in Example 1C

<400> 22
cgggatccga agcgatgagt ataggtacct tc 32

<210> 23
<211> 25
<212> PRT
<213> Artificial Sequence

<220>
<223> The sequence of peptide fragment described in experimental example 1

<400> 23

```
Met Leu Gly Lys Leu Ser Phe Leu Ser Ala Leu Ser Leu Ala Val Ala
1               5                   10                  15

Ala Thr Leu Ser Asn Ser Thr Ser Ala
            20                  25
```